# EUROPEAN PATENT APPLICATION

(11) **EP 4 671 240 A1**
(43) Date of publication of application: **31.12.2025**
(21) Application number: 24759756.0
(22) Date of filing: 23.02.2024
(51) Int. Cl.: C07D 413/12, C07D 403/10, A61K 31/41, A61P 9/12

(54) **SALT CONTAINING AROMATIC RING DERIVATIVE ANTAGONIST, PREPARATION METHOD THEREFOR, AND APPLICATION THEREOF**

(30) Priority: 24.02.2023 CN 202310165061
(71) Applicant: Jiangsu Hansoh Pharmaceutical Group Co., Ltd., Lianyungang, Jiangsu 222047 (CN); Shanghai Hansoh Biomedical Co., Ltd., Shanghai 201203 (CN); Changzhou Hansoh Pharmaceutical Co., Ltd., Xinbei District Changzhou Jiangsu 213001 (CN)
(72) Inventor: LI, Yuanyuan, Shanghai 201203 (CN); GUO, Linsong, Shanghai 201203 (CN)
(74) Representative: Dehns
(86) International application number: PCT/CN2024/078256
(87) International publication number: WO 2024/175082

(57) **Abstract**

The present application relates to a salt of an aromatic ring-containing derivative antagonist, a crystal form thereof, a preparation method therefor, and an application thereof. Specifically, the present application relates to a compound salt of formula (I), a crystal form, a preparation method, and a pharmaceutical composition containing a therapeutically effective amount of the salt or crystal form, and the use thereof as a protease inhibitor in the preparation of a medicament for treating or preventing a disease with dual angiotensin-dependent and endothelin-dependent mechanisms.

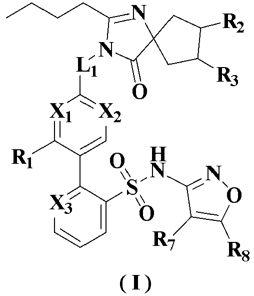

## Description

### Technical Field

The present application relates to the field of biomedicine, and in particular to a salt or a crystal form of a preparation for treating or preventing a disease with dual angiotensin-dependent and endothelin-dependent mechanisms, a preparation method therefor, and an application thereof.

### Background Art

Focal segmental glomerulosclerosis (FSGS) is one of the manifestations of nephrotic syndrome and the main cause of end-stage kidney disease. Its pathogenesis is complex and has not yet been fully understood. Current drug treatments, mainly glucocorticoids and immunosuppressants, have poor responses, cannot ideally control the occurrence and progression of FSGS, and have obvious side effects. There is currently no approved treatment for FSGS. The complete response rate of FSGS treatment is less than 30%. One-third of patients progress to chronic renal failure after five years and require long-term dialysis or kidney transplantation to maintain life, which brings heavy economic burden to families and society, and exploring new treatment options has become a focus.

In addition to FSGS, other kidney diseases or conditions characterized by glomerular damage include IgA nephropathy and idiopathic membranous nephropathy. IgA nephropathy, also called Berger's disease, is caused by the accumulation of immunoglobulin A (IgA) in the kidneys. The presence of IgA in the kidneys may lead to inflammation, damage to the glomeruli of the kidneys, and impaired kidney function, including proteinuria. In some cases, patients with IgA nephropathy progress to ESRD. IgA nephropathy is the most common form of glomerulonephritis in the world. In approximately 30% of patients, a decrease in glomerular filtration rate of approximately 50% over 10 years is observed. Patients with IgA nephropathy develop IgG autoantibodies against galactose-deficient IgA1 antibodies. This results in the deposition of these antibodies in the mesangium and activation of complement. Basic treatment for patients with IgA nephropathy involves eliminating risk factors, particularly hypertension, by blocking the renin-angiotensin-aldosterone system (RAAS). Immunosuppression has also been studied in various studies, but no clear advantage was observed. Common side effects of hormone therapy include increased blood sugar, osteoporosis, infection, *etc.* Therefore, there remains a need for compositions and methods for treating various kidney diseases or conditions, such as FSGS, IgA nephropathy, and IMN.

The endogenous vasoactive peptide angiotensin II (AngII) and endothelin-1 (ET-1) are two powerful vasoconstrictors and are thought to play a role in the control of vascular tone and pathological tissue remodelling associated with a variety of diseases, including diabetic nephropathy, heart failure, and chronic or persistently elevated blood pressure. The renin-angiotensin-aldosterone system (RAAS) regulates blood pressure, fluid and sodium balance. Excessive activation of RAAS can promote systemic and regional glomerular capillary hypertension, cause glomerular hemodynamic damage, and lead to kidney damage and kidney fibrosis via profibrotic and proinflammatory pathways. Drugs for RAAS system such as angiotensin receptor blockers (ARBs) have been used to treat diabetic nephropathy, heart failure, and chronic or persistently elevated blood pressure. In addition, accumulating data demonstrate the potential therapeutic benefits of ETA receptor antagonists (ERAs) in hypertension and diabetic nephropathy.

Studies have shown that the combination of ARB and ERA produces a synergistic effect, with AngII and ET-1 working together in blood pressure control and pathological tissue remodelling. Increased Ang II levels promote ET-1 synthesis and vasoconstriction. Blocking ET receptors with ETA can reduce AngII-induced vasoconstriction and reduce plasma aldosterone. ARB not only blocks the effect of AngII on its AT1 receptor, but also limits the production of ET-1. Therefore, blocking AngII and ET-1 activity simultaneously may provide better efficacy than blocking either substance alone. In addition, although ARB is the standard treatment for patients with diabetic nephropathy, dual antagonists (ARB and ERA) have been reported in phase II clinical development to improve proteinuria changes in patients with FSGS. Therefore, drugs with AT1/ETA dual-target antagonistic mechanism have the potential to treat kidney diseases and are of great significance for drug development.

An international patent (Application No.: PCT/CN2022/115068) discloses the structures of a series of aromatic ring-containing biological antagonists. In the subsequent research and development, in order to make the products easy to treat, filter, and dry, convenient to store, stable for a long time, highly bioavailable, etc., the present invention has conducted a comprehensive study on the salts and crystal forms of the above substances and is dedicated to obtaining the most suitable crystal form.

### Summary of the Invention

The full content involved in patent application PCT/CN2022/115068 is incorporated into the present invention by reference.

The objective of the present invention is to provide an acid salt of a compound as represented by general formula (I): 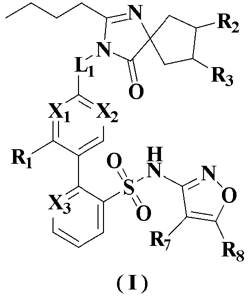 wherein
L₁ is -CH₂ or -CD₂-;
X₁ is N or CR¹;
X₂ is N or CR²;
X₃ is N or CR³;
R¹, R² and R³ are each independently selected from hydrogen, deuterium, halogen, amino, hydroxyl, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms, and the C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms is optionally further substituted with one or more substituents selected from deuterium, halogen, amino, hydroxyl, cyano, oxo, thio, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms;
R₁ is selected from hydrogen, C₁₋₃ alkyl or -(CH₂)ₙ₁O(CH₂)ₙ₂R_{A2}, and the C₁₋₃ alkyl is optionally further substituted with one or more substituents selected from deuterium, halogen, amino, hydroxyl, cyano, oxo, thio, C₁₋₃ alkyl, C₂₋₄ alkenyl, C₂₋₄ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy or C₃₋₆ cycloalkyl;
R_{A2} is selected from C₁₋₃ alkyl, C₁₋₃ deuteroalkyl, C₁₋₃ haloalkyl, C₃₋₆ cycloalkyl, 4- to 7-membered heterocyclyl, C₃₋₆ cycloalkyl C₁₋₃ alkyl or 4- to 7-membered heterocyclyl C₁₋₃ alkyl;
R₂ and R₃ are each independently selected from hydrogen, deuterium, halogen, amino, hydroxyl, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms, and the C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms is optionally further substituted with one or more substituents selected from deuterium, halogen, amino, hydroxyl, cyano, oxo, thio, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms;
alternatively, R₂ and R₃, together with the atom to which they are attached, form C₃₋₈ cycloalkyl or 3- to 8-membered heterocyclyl;
R₇ or R₈ is each independently selected from hydrogen, deuterium, halogen, amino, hydroxyl, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl or 5- to 10-membered heteroaryl;
when L₁ is -CH₂-, and R₂ and R₃ are both hydrogen, R₇ and R₈ are not both - CH₃;
when L₁ is -CH₂-, and R₂ and R₃ are both hydrogen, R₁ is not hydrogen;
n1 and n2 are each independently 0, 1, 2 or 3;
the acid in the acid salt is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, capric acid, caproic acid, caprylic acid, cinnamic acid, citric acid, cyclohexanesulfamic acid, camphorsulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfate, dibenzoyltartaric acid, ethane-1,2-disulfonic acid, ethylsulfonic acid, formic acid, fumaric acid, galactosonic acid, gentisic acid, glutaric acid, 2-ketoglutaric acid, glycolic acid, hippuric acid, isethionic acid, lactobionic acid, ascorbic acid, aspartic acid, lauric acid, camphoric acid, maleic acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, butanedioic acid, thiocyanic acid, pamoic acid, formic acid, undecylenic acid, trifluoroacetic acid, benzenesulfonic acid, p-toluenesulfonic acid or L-malic acid; preferably methanesulfonic acid, benzenesulfonic acid, isethionic acid, ethylsulfonic acid, hydrochloric acid, sulphuric acid, p-toluenesulfonic acid, phosphoric acid or hydrobromic acid.

In a preferred embodiment of the present invention,
R¹, R² and R³ are each independently selected from hydrogen, deuterium, halogen, amino, hydroxyl, methyl, ethyl or propyl;
R₁ is selected from hydrogen, -CH₃, -CH₂CH₃, 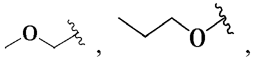 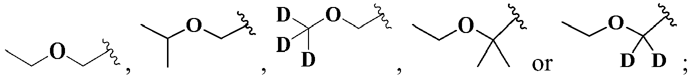
R₂ or R₃ is each independently selected from hydrogen, deuterium, fluorine, chlorine, bromine, methyl, ethyl or propyl;
alternatively, R₂ and R₃, together with the atom to which they are attached, form cyclopropyl, cyclobutyl, cyclopentyl or cyclohexyl;
R₇ is selected from deuterium, fluorine, chlorine, bromine, methyl, ethyl, propyl, deuterated methyl, dideuterated methyl or trideuterated methyl;
R₈ is selected from methyl, ethyl or propyl.

In a preferred embodiment of the present invention, the compound is
4'-((2-butyl-4-oxo-1,3-diazospiro[4.4]non-1-en-3-yl)methyl-d2)-N-(4,5-dimethylisoxazol-3-yl)- 2'-(ethoxymethyl)-[1,1'-biphenyl] -2-sulfonamide;
4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide;
4'-((2-butyl-4-oxo-1,3-diazaspiro[4.4]non-1-en-3-yl)methyl-d2)-N-(4-fluoro-5-methylisoxazol-3-yl)-2'-(ethoxymethyl)-[1,1'-biphenyl]-2-sulfonamide;
4'-((2-butyl-4-oxo-1,3-diazospiro[4.4]non-1-en-3-yl)methyl)-N-(4-chloro-5-methylisoxazol-3-yl)-2'-(ethoxydeuteromethyl)-[1,1'-biphenyl]-2-sulfonamide;
2-[4-[(2-butyl-4-oxo-1,3-diazaspiro[4.4]non-1-en-3-yl)methyl]-2-(ethoxymethyl)phenyl]-N-(4-chloro-5-methyl-isoxazol-3-yl)benzenesulfonamide; or
4'-((2'-butyl-5'-oxaspiro[bicyclo[3.1.0]hexane-3,4'-imidazole] -1'(5'H)-yl)methyl)-N-(4,5 -dimethylisoxazol-3-yl)-2'-(ethoxymethyl)-[1,1'-biphenyl] -2-sulfonamide;
the acid in the acid salt is selected from methanesulfonic acid, ethylsulfonic acid, hydrochloric acid, sulphuric acid, p-toluenesulfonic acid, benzenesulfonic acid, isethionic acid or hydrobromic acid.

In a further preferred embodiment of the present invention, the number of acid molecules is 0.2 to 3; preferably 0.2, 0.5, 1, 1.5, 2, 2.5 or 3; more preferably 0.5, 1, 2 or 3, further preferably 1.

In a further preferred embodiment of the present invention, the acid salt is a hydrate or an anhydrate; when the acid salt is a hydrate, the number of water molecules is 0.2 to 3; preferably 0.2, 0.5, 1, 1.5, 2, 2.5 or 3; more preferably 0.5, 1, 2 or 3.

In a further preferred embodiment of the present invention, the acid salt is in a crystal form;
preferably the crystal form of the acid salt of the compound 4'-((2-butyl-4-oxo-1,3-diazospiro[4.4]non-1-en-3-yl)methyl-d2)-N-(4,5-dimethylisoxazol-3-yl)- 2'-(ethoxymethyl)-[1,1'-biphenyl] -2-sulfonamide;
the crystal form of the acid salt of 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide;
the crystal form of the acid salt of 4'-((2-butyl-4-oxo-1,3-diazaspiro[4.4]non-1-en-3-yl)methyl-d2)-N-(4-fluoro-5-methylisoxazol-3-yl)-2'-(ethoxymethyl)-[1,1'-biphenyl]-2-sulfonamide;
the crystal form of the acid salt of 2-[4-[(2-butyl-4-oxo-1,3-diazaspiro[4.4]non-1-en-3-yl)methyl]-2-(ethoxymethyl)phenyl]-N-(4-chloro-5-methyl-isoxazol-3-yl)benzenesulfonamide;
the crystal form of the acid salt of 4'-((2'-butyl-5'-oxaspiro[bicyclo[3.1.0]hexane-3,4'-imidazole] -1'(5'H)-yl)methyl)-N-(4,5 - dimethylisoxazol-3-yl)-2'-(ethoxymethyl)-[1,1'-biphenyl] -2-sulfonamide;
more preferably the crystal form of methanesulfonic acid salt, the crystal form of ethylsulfonic acid salt, the crystal form of hydrochloride salt, the crystal form of sulphuric acid salt, the crystal form of p-toluenesulfonic acid salt, the crystal form of benzenesulfonic acid salt, the crystal form of isethionic acid salt or the crystal form of hydrobromic acid salt.

In a further preferred embodiment of the present invention, the crystal form of the acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide is:
crystal form A of the hydrochloride salt, characterized by an X-ray powder diffraction pattern having a diffraction peak at 2θ = 9.2±0.2°; or a diffraction peak at 2θ = 9.0±0.2°; or a diffraction peak at 2θ = 8.2±0.2°; or a diffraction peak at 2θ = 10.8±0.2°; or a diffraction peak at 2θ = 16.8±0.2°; or a diffraction peak at 2θ = 18.8±0.2°; or a diffraction peak at 2θ = 20.2±0.2°; or a diffraction peak at 2θ = 20.6±0.2°; or a diffraction peak at 2θ = 20.9±0.2°; or a diffraction peak at 2θ = 23.2±0.2°; or a diffraction peak at 2θ = 25.3±0.2°; or a diffraction peak at 2θ = 24.0±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
crystal form B of the hydrochloride salt, characterized by an X-ray powder diffraction pattern having a diffraction peak at 2θ = 10.4±0.2°; or a diffraction peak at 2θ = 7.3±0.2°; or a diffraction peak at 2θ = 14.1±0.2°; or a diffraction peak at 2θ = 14.6±0.2°; or a diffraction peak at 2θ = 17.0±0.2°; or a diffraction peak at 2θ = 19.3±0.2°; or a diffraction peak at 2θ = 21.9±0.2°; or a diffraction peak at 2θ = 24.8±0.2°; or a diffraction peak at 2θ = 11.0±0.2°; or a diffraction peak at 2θ = 13.1±0.2°; or a diffraction peak at 2θ = 20.3±0.2°; or a diffraction peak at 2θ = 22.1±0.2°; or a diffraction peak at 2θ = 24.1±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
crystal form C of the hydrochloride salt, characterized by an X-ray powder diffraction pattern having a diffraction peak at 2θ = 8.6±0.2°; or a diffraction peak at 2θ = 7.9±0.2°; or a diffraction peak at 2θ = 14.7±0.2°; or a diffraction peak at 2θ = 14.9±0.2°; or a diffraction peak at 2θ = 6.3±0.2°; or a diffraction peak at 2θ = 17.4±0.2°; or a diffraction peak at 2θ = 10.3±0.2°; or a diffraction peak at 2θ = 12.4±0.2°; or a diffraction peak at 2θ = 23.8±0.2°; or a diffraction peak at 2θ = 24.8±0.2°; or a diffraction peak at 2θ = 16.0±0.2°; or a diffraction peak at 2θ = 18.2±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
crystal form A of the ethylsulfonic acid salt, characterized by an X-ray powder diffraction pattern having a diffraction peak at 2θ = 8.7±0.2°; or a diffraction peak at 2θ = 7.4±0.2°; or a diffraction peak at 2θ = 17.0±0.2°; or a diffraction peak at 2θ = 17.5±0.2°; or a diffraction peak at 2θ = 19.4±0.2°; or a diffraction peak at 2θ = 22.7±0.2°; or a diffraction peak at 2θ = 26.4±0.2°; or a diffraction peak at 2θ = 28.8±0.2°; or a diffraction peak at 2θ = 20.1±0.2°; or a diffraction peak at 2θ = 35.1±0.2°; or a diffraction peak at 2θ = 35.4±0.2°; or a diffraction peak at 2θ = 15.5±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
crystal form B of the ethylsulfonic acid salt, characterized by an X-ray powder diffraction pattern having a diffraction peak at 2θ = 8.8±0.2°; or a diffraction peak at 2θ = 8.3±0.2°; or a diffraction peak at 2θ = 7.9±0.2°; or a diffraction peak at 2θ = 17.0±0.2°; or a diffraction peak at 2θ = 16.2±0.2°; or a diffraction peak at 2θ = 18.4±0.2°; or a diffraction peak at 2θ = 20.6±0.2°; or a diffraction peak at 2θ = 24.1±0.2°; or a diffraction peak at 2θ = 26.4±0.2°; or a diffraction peak at 2θ = 10.8±0.2°; or a diffraction peak at 2θ = 12.6±0.2°; or a diffraction peak at 2θ = 19.9±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
crystal form A of the p-toluenesulfonic acid salt, characterized by an X-ray powder diffraction pattern having a diffraction peak at 2θ = 5.8±0.2°; or a diffraction peak at 2θ = 6.2±0.2°; or a diffraction peak at 2θ = 18.6±0.2°; or a diffraction peak at 2θ = 23.2±0.2°; or a diffraction peak at 2θ = 29.1±0.2°; preferably comprising any 1, or 1-2, or 2-3 of the aforementioned diffraction peaks, more preferably comprising any 1, 2, 3, 4 or 5;
crystal form A of the methanesulfonic acid salt, characterized by an X-ray powder diffraction pattern having a diffraction peak at 2θ = 8.1±0.2°; or a diffraction peak at 2θ = 8.4±0.2°; or a diffraction peak at 2θ = 4.1±0.2°; or a diffraction peak at 2θ = 20.8±0.2°; or a diffraction peak at 20 = 22.3±0.2°; or a diffraction peak at 2θ = 18.9±0.2°; or a diffraction peak at 2θ = 24.5±0.2°; or a diffraction peak at 2θ = 20.0±0.2°; or a diffraction peak at 2θ = 24.3±0.2°; or a diffraction peak at 2θ = 11.1±0.2°; or a diffraction peak at 2θ = 12.6±0.2°; or a diffraction peak at 2θ = 16.5±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
crystal form A of the sulphuric acid salt, characterized by an X-ray powder diffraction pattern having a diffraction peak at 2θ = 8.3±0.2°; or a diffraction peak at 2θ = 20.9±0.2°; or a diffraction peak at 2θ = 18.5±0.2°; or a diffraction peak at 2θ = 24.4±0.2°; or a diffraction peak at 2θ = 10.0±0.2°; or a diffraction peak at 2θ = 11.0±0.2°; or a diffraction peak at 2θ = 12.6±0.2°; or a diffraction peak at 2θ = 16.3±0.2°; or a diffraction peak at 2θ = 19.9±0.2°; or a diffraction peak at 2θ = 20.4±0.2°; or a diffraction peak at 2θ = 21.9±0.2°; or a diffraction peak at 2θ = 26.7±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
crystal form A of the benzenesulfonic acid salt, characterized by an X-ray powder diffraction pattern having a diffraction peak at 2θ = 5.9±0.2°; or a diffraction peak at 2θ = 23.8±0.2°; or a diffraction peak at 2θ = 17.8±0.2°; or a diffraction peak at 2θ = 29.9±0.2°; preferably comprising any 1-3 or 2-4 of the aforementioned diffraction peaks, more preferably comprising any 1, 2, 3 or 4;
crystal form A of the isethionic acid salt, characterized by an X-ray powder diffraction pattern having a diffraction peak at 2θ = 9.9±0.2°; or a diffraction peak at 2θ = 19.9±0.2°; or a diffraction peak at 2θ = 8.3±0.2°; or a diffraction peak at 2θ = 20.5±0.2°; or a diffraction peak at 2θ = 18.2±0.2°; or a diffraction peak at 2θ = 23.9±0.2°; or a diffraction peak at 2θ = 10.6±0.2°; preferably comprising any 2-5, or 3-5, or 3-6, or 3-7 of the aforementioned diffraction peaks, more preferably comprising any 2, 3, 4 or 5;
crystal form A of the hydrobromic acid salt, characterized by an X-ray powder diffraction pattern having a diffraction peak at 2θ = 30.0±0.2°; or a diffraction peak at 2θ = 22.3±0.2°; or a diffraction peak at 2θ = 25.9±0.2°; or a diffraction peak at 2θ = 29.7±0.2°; or a diffraction peak at 2θ = 17.0±0.2°; or a diffraction peak at 2θ = 35.7±0.2°; or a diffraction peak at 2θ = 33.3±0.2°; or a diffraction peak at 2θ = 23.4±0.2°; or a diffraction peak at 2θ = 28.6±0.2°; or a diffraction peak at 2θ = 28.8±0.2°; or a diffraction peak at 2θ = 18.1±0.2°; or a diffraction peak at 2θ = 26.6±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8.

In a further preferred embodiment of the present invention,
the crystal form A of the hydrochloride salt is characterized by an X-ray powder diffraction pattern comprising at least one or more diffraction peaks at 2θ = 9.2±0.2°, 9.0±0.2° or 8.2±0.2°, preferably comprising two, more preferably comprising three; optionally further comprising at least one diffraction peak at 2θ = 10.8+0.2°, 16.8±0.2°, 18.8+0.2°, 20.2±0.2°, 20.6+0.2°, 20.9+0.2° or 23.2±0.2°, preferably comprising 2, 3, 4 or 5; for example, at 2θ =
9.2±0.2° or 9.0±0.2°;
9.2±0.2° or 8.2±0.2°;
9.0±0.2° or 8.2±0.2°;
9.2±0.2°, 9.0±0.2° or 8.2±0.2°;
9.2±0.2°, 8.2±0.2° or 10.8±0.2°;
9.0±0.2°, 8.2±0.2° or 10.8±0.2°;
9.2±0.2°, 9.0±0.2°, 8.2±0.2° or 10.8±0.2°;
9.2±0.2°, 8.2±0.2°, 10.8±0.2° or 16.8±0.2°;
9.0±0.2°, 8.2±0.2°, 10.8±0.2° or 16.8±0.2°;
9.2±0.2°, 9.0±0.2°, 8.2±0.2°, 10.8±0.2° or 16.8±0.2°;
9.2±0.2°, 8.2±0.2°, 10.8±0.2°, 16.8±0.2° or 18.8±0.2°;
9.0±0.2°, 8.2±0.2°, 10.8±0.2°, 16.8±0.2° or 18.8±0.2°;
9.2±0.2°, 9.0±0.2°, 8.2±0.2°, 10.8±0.2°, 16.8±0.2° or 18.8±0.2°;
9.2±0.2°, 8.2±0.2°, 10.8±0.2°, 16.8±0.2°, 18.8±0.2° or 20.2±0.2°;
9.0±0.2°, 8.2±0.2°, 10.8±0.2°, 16.8±0.2°, 18.8±0.2° or 20.2±0.2°;
9.2±0.2°, 9.0±0.2°, 8.2±0.2°, 10.8±0.2°, 16.8±0.2°, 18.8±0.2°, 20.2±0.2° or 20.6±0.2°;
9.2±0.2°, 8.2±0.2°, 10.8±0.2°, 16.8±0.2°, 18.8±0.2°, 20.2±0.2°, 20.6±0.2° or 20.9±0.2°;
9.0±0.2°, 8.2±0.2°, 10.8±0.2°, 16.8±0.2°, 18.8±0.2°, 20.2±0.2°, 20.6±0.2° or 20.9±0.2°;
9.2±0.2°, 9.0±0.2°, 8.2±0.2°, 10.8±0.2°, 16.8±0.2°, 18.8±0.2°, 20.6±0.2° or 20.9±0.2°;
9.2±0.2°, 8.2±0.2°, 10.8±0.2°, 16.8±0.2°, 18.8±0.2°, 20.2±0.2°, 20.9±0.2° or 23.2±0.2°;
9.0+0.2°, 8.2+0.2°, 10.8+0.2°, 16.8+0.2°, 18.8+0.2°, 20.2+0.2°, 20.9+0.2° or 23.2+0.2°;
the crystal form B of the hydrochloride salt is characterized by an X-ray powder diffraction pattern comprising at least one or more diffraction peaks at 2θ = 10.4+0.2°, 7.3+0.2° or 14.1+0.2°, preferably comprising two, more preferably comprising three; optionally further comprising at least one diffraction peak at 2θ = 14.6±0.2°, 17.0±0.2°, 19.3±0.2°, 21.9±0.2°, 24.8±0.2°, 11.0±0.2° or 13.1±0.2°, preferably comprising 2, 3, 4 or 5; for example, at 2θ =
10.4±0.2° or 7.3±0.2°;
10.4±0.2° or 14.1±0.2°;
7.3±0.2° or 14.1±0.2°;
10.4±0.2°, 7.3±0.2° or 14.1±0.2°;
10.4±0.2°, 14.1±0.2° or 14.6±0.2°;
7.3±0.2°, 14.1±0.2° or 14.6±0.2°;
10.4±0.2°, 7.3±0.2°, 14.1±0.2° or 14.6±0.2°;
10.4±0.2°, 14.1±0.2°, 14.6±0.2° or 17.0±0.2°;
7.3±0.2°, 14.1±0.2°, 14.6+0.2° or 17.0±0.2°;
10.4±0.2°, 7.3±0.2°, 14.1±0.2°, 14.6±0.2° or 17.0±0.2°;
10.4±0.2°, 14.1±0.2°, 14.6±0.2°, 17.0±0.2° or 19.3±0.2°;
7.3±0.2°, 14.1±0.2°, 14.6±0.2°, 17.0±0.2° or 19.3±0.2°;
10.4±0.2°, 7.3±0.2°, 14.1±0.2°, 14.6±0.2°, 17.0±0.2° or 19.3±0.2°;
10.4±0.2°, 14.1±0.2°, 14.6±0.2°, 17.0±0.2°, 19.3±0.2° or 21.9±0.2°;
7.3±0.2°, 14.1±0.2°, 14.6±0.2°, 17.0±0.2°, 19.3±0.2° or 21.9±0.2°;
10.4±0.2°, 7.3±0.2°, 14.1±0.2°, 14.6±0.2°, 17.0±0.2°, 19.3±0.2°, 21.9+0.2° or 24.8±0.2°;
10.4±0.2°, 14.1±0.2°, 14.6±0.2°, 17.0±0.2°, 19.3±0.2°, 21.9±0.2°, 24.8±0.2° or 11.0±0.2°;
7.3±0.2°, 14.1±0.2°, 14.6±0.2°, 17.0±0.2°, 19.3±0.2°, 21.9+0.2°, 24.8±0.2° or 11.0±0.2°;
10.4±0.2°, 7.3±0.2°, 14.1±0.2°, 14.6±0.2°, 17.0±0.2°, 19.3±0.2°, 24.8±0.2° or 11.0±0.2°;
10.4±0.2°, 14.1±0.2°, 14.6±0.2°, 17.0±0.2°, 19.3±0.2°, 21.9±0.2°, 11.0±0.2° or 13.1±0.2°;
7.3±0.2°, 14.1±0.2°, 14.6±0.2°, 17.0±0.2°, 19.3±0.2°, 21.9±0.2°, 11.0±0.2° or 13.1±0.2°;
the crystal form C of the hydrochloride salt is characterized by an X-ray powder diffraction pattern comprising at least one or more diffraction peaks at 2θ = 8.6±0.2°, 7.9±0.2° or 14.7±0.2°, preferably comprising two, more preferably comprising three; optionally further comprising at least one diffraction peak at 2θ = 14.9±0.2°, 6.3±0.2°, 17.4±0.2°, 10.3±0.2°, 12.4±0.2°, 23.8±0.2° or 24.8±0.2°, preferably comprising 2, 3, 4 or 5; for example, at 2θ =
8.6±0.2° or 7.9±0.2°;
8.6±0.2° or 14.7±0.2°;
7.9±0.2° or 14.7±0.2°;
8.6±0.2°, 7.9±0.2° or 14.7±0.2°;
8.6±0.2°, 14.7±0.2° or 14.9±0.2°;
7.9±0.2°, 14.7±0.2° or 14.9±0.2°;
8.6±0.2°, 7.9±0.2°, 14.7±0.2° or 14.9±0.2°;
8.6±0.2°, 14.7±0.2°, 14.9±0.2° or 6.3±0.2°;
7.9±0.2°, 14.7±0.2°, 14.9±0.2° or 6.3±0.2°;
8.6±0.2°, 7.9±0.2°, 14.7±0.2°, 14.9±0.2° or 6.3±0.2°;
8.6±0.2°, 14.7±0.2°, 14.9±0.2°, 6.3±0.2° or 17.4±0.2°;
7.9±0.2°, 14.7±0.2°, 14.9±0.2°, 6.3±0.2° or 17.4±0.2°;
8.6±0.2°, 7.9±0.2°, 14.7±0.2°, 14.9±0.2°, 6.3±0.2° or 17.4±0.2°;
8.6±0.2°, 14.7±0.2°, 14.9±0.2°, 6.3±0.2°, 17.4±0.2° or 10.3±0.2°;
7.9±0.2°, 14.7±0.2°, 14.9±0.2°, 6.3±0.2°, 17.4±0.2° or 10.3±0.2°;
8.6±0.2°, 7.9±0.2°, 14.7±0.2°, 14.9±0.2°, 6.3±0.2°, 17.4±0.2°, 10.3±0.2° or 12.4±0.2°;
8.6±0.2°, 14.7±0.2°, 14.9±0.2°, 6.3±0.2°, 17.4±0.2°, 10.3±0.2°, 12.4±0.2° or 23.8±0.2°;
7.9±0.2°, 14.7±0.2°, 14.9±0.2°, 6.3±0.2°, 17.4±0.2°, 10.3±0.2°, 12.4±0.2° or 23.8±0.2°;
the crystal form A of the ethylsulfonic acid salt is characterized by an X-ray powder diffraction pattern comprising at least one or more diffraction peaks at 2θ = 8.7±0.2°, 7.4±0.2° or 17.0±0.2°, preferably comprising two, more preferably comprising three; optionally further comprising at least one diffraction peak at 2θ = 17.5±0.2°, 19.4±0.2°, 22.7±0.2°, 26.4±0.2°, 28.8±0.2°, 20.1±0.2° or 35.1±0.2°, preferably comprising 2, 3, 4 or 5; for example, at 2θ =
8.7±0.2° or 7.4±0.2°;
8.7±0.2° or 17.0+0.2°;
7.4±0.2° or 17.0+0.2°;
8.7+0.2°, 7.4±0.2° or 17.0+0.2°;
8.7+0.2°, 17.0±0.2° or 17.5+0.2°;
7.4±0.2°, 17.0±0.2° or 17.5±0.2°;
8.7±0.2°, 7.4±0.2°, 17.0±0.2° or 17.5±0.2°;
8.7±0.2°, 17.0±0.2°, 17.5±0.2° or 19.4±0.2°;
7.4±0.2°, 17.0±0.2°, 17.5±0.2° or 19.4±0.2°;
8.7±0.2°, 7.4±0.2°, 17.0±0.2°, 17.5±0.2° or 19.4±0.2°;
8.7±0.2°, 17.0±0.2°, 17.5±0.2°, 19.4±0.2° or 22.7±0.2°;
7.4±0.2°, 17.0±0.2°, 17.5±0.2°, 19.4±0.2° or 22.7±0.2°;
8.7±0.2°, 7.4±0.2°, 17.0±0.2°, 17.5±0.2°, 19.4±0.2° or 22.7±0.2°;
8.7±0.2°, 17.0±0.2°, 17.5±0.2°, 19.4+0.2°, 22.7±0.2° or 26.4±0.2°;
7.4±0.2°, 17.0±0.2°, 17.5±0.2°, 19.4±0.2°, 22.7±0.2° or 26.4±0.2°;
8.7±0.2°, 7.4±0.2°, 17.0±0.2°, 17.5±0.2°, 19.4±0.2°, 22.7±0.2°, 26.4±0.2° or 28.8±0.2°;
8.7±0.2°, 17.0±0.2°, 17.5±0.2°, 19.4±0.2°, 22.7±0.2°, 26.4±0.2°, 28.8±0.2° or 20.1±0.2°;
7.4±0.2°, 17.0±0.2°, 17.5±0.2°, 19.4±0.2°, 22.7±0.2°, 26.4±0.2°, 28.8±0.2° or 20.1±0.2°;
8.7±0.2°, 7.4±0.2°, 17.0±0.2°, 17.5±0.2°, 19.4±0.2°, 22.7±0.2°, 28.8±0.2° or 20.1±0.2°;
8.7±0.2°, 17.0±0.2°, 17.5±0.2°, 19.4±0.2°, 22.7±0.2°, 26.4±0.2°, 20.1±0.2° or 35.1±0.2°;
7.4±0.2°, 17.0±0.2°, 17.5±0.2°, 19.4±0.2°, 22.7+0.2°, 26.4±0.2°, 20.1±0.2° or 35.1±0.2°;
the crystal form B of the ethylsulfonic acid salt is characterized by an X-ray powder diffraction pattern comprising at least one or more diffraction peaks at 2θ = 8.8±0.2°, 8.3±0.2° or 7.9±0.2°, preferably comprising two, more preferably comprising three; optionally further comprising at least one diffraction peak at 2θ = 17.0±0.2°, 16.2±0.2°, 18.4±0.2°, 20.6±0.2°, 24.1±0.2°, 26.4±0.2° or 10.8±0.2°, preferably comprising 2, 3, 4 or 5; for example, at 2θ =
8.8±0.2° or 8.3±0.2°;
8.8±0.2° or 7.9+0.2°;
8.3+0.2° or 7.9+0.2°;
8.8+0.2°, 8.3+0.2° or 7.9±0.2°
8.8+0.2°, 7.9±0.2° or 17.0+0.2°;
8.3+0.2°, 7.9±0.2° or 17.0+0.2°;
8.8±0.2°, 8.3±0.2°, 7.9±0.2° or 17.0±0.2°;
8.8±0.2°, 7.9±0.2°, 17.0±0.2° or 16.2±0.2°;
8.3±0.2°, 7.9±0.2°, 17.0±0.2° or 16.2±0.2°;
8.8±0.2°, 8.3±0.2°, 7.9±0.2°, 17.0±0.2° or 16.2±0.2°;
8.8±0.2°, 7.9±0.2°, 17.0±0.2°, 16.2±0.2° or 18.4±0.2°;
8.3±0.2°, 7.9±0.2°, 17.0±0.2°, 16.2±0.2° or 18.4±0.2°;
8.8±0.2°, 8.3±0.2°, 7.9±0.2°, 17.0±0.2°, 16.2±0.2° or 18.4±0.2°;
8.8±0.2°, 7.9±0.2°, 17.0±0.2°, 16.2+0.2°, 18.4±0.2° or 20.6±0.2°;
8.3±0.2°, 7.9±0.2°, 17.0±0.2°, 16.2+0.2°, 18.4±0.2° or 20.6±0.2°;
8.8±0.2°, 8.3±0.2°, 7.9±0.2°, 17.0±0.2°, 16.2±0.2°, 18.4±0.2°, 20.6±0.2° or 24.1±0.2°;
8.8±0.2°, 7.9±0.2°, 17.0±0.2°, 16.2±0.2°, 18.4±0.2°, 20.6±0.2°, 24.1±0.2° or 26.4±0.2°;
8.3±0.2°, 7.9±0.2°, 17.0±0.2°, 16.2±0.2°, 18.4±0.2°, 20.6±0.2°, 24.1±0.2° or 26.4±0.2°;
8.8±0.2°, 8.3±0.2°, 7.9±0.2°, 17.0±0.2°, 16.2±0.2°, 18.4±0.2°, 24.1±0.2° or 26.4±0.2°;
8.8±0.2°, 7.9±0.2°, 17.0±0.2°, 16.2±0.2°, 18.4±0.2°, 20.6±0.2°, 26.4±0.2° or 10.8±0.2°;
8.3±0.2°, 7.9±0.2°, 17.0±0.2°, 16.2±0.2°, 18.4±0.2°, 20.6±0.2°, 26.4±0.2° or 10.8±0.2°;
the crystal form A of the p-toluenesulfonic acid salt is characterized by an X-ray powder diffraction pattern comprising at least a diffraction peak at 2θ = 5.8±0.2°, optionally further comprising at least one diffraction peak at 2θ = 6.2±0.2°, 18.6±0.2°, 23.2±0.2° or 29.1±0.2°, preferably comprising 1, 2, 3 or 4; for example, at 2θ =
5.8±0.2°;
5.8±0.2° or 6.2±0.2°;
5.8±0.2° or 18.6+0.2°;
5.8±0.2° or 23.2+0.2°;
5.8±0.2° or 29.1±0.2°;
5.8±0.2°, 6.2+0.2° or 18.6+0.2°;
5.8±0.2°, 18.6±0.2° or 23.2+0.2°;
5.8±0.2°, 23.2±0.2° or 29.1±0.2°;
5.8±0.2°, 6.2±0.2°, 18.6±0.2° or 23.2±0.2°;
5.8±0.2°, 18.6±0.2°, 23.2±0.2° or 29.1±0.2°;
5.8±0.2°, 6.2±0.2°, 18.6±0.2°, 23.2±0.2° or 29.1±0.2°;
the crystal form A of the methanesulfonic acid salt is characterized by an X-ray powder diffraction pattern comprising at least one or more diffraction peaks at 2θ = 8.1±0.2°, 8.4±0.2° or 4.1±0.2°, preferably comprising two, more preferably comprising three; optionally further comprising at least one diffraction peak at 2θ = 20.8±0.2°, 22.3±0.2°, 18.9±0.2°, 24.5±0.2°, 20.0±0.2°, 24.3±0.2° or **11.1±0.2°,** preferably comprising 2, 3, 4 or 5; for example, at 2θ =
8.1±0.2° or 8.4±0.2°;
8.1±0.2° or 4.1±0.2°;
8.4±0.2° or 4.1±0.2°;
8.1±0.2°, 8.4±0.2° or 4.1±0.2°;
8.1±0.2°, 4.1±0.2° or 20.8±0.2°;
8.4±0.2°, 4.1±0.2° or 20.8±0.2°;
8.1±0.2°, 8.4±0.2°, 4.1±0.2° or 20.8±0.2°;
8.1±0.2°, 4.1±0.2°, 20.8±0.2° or 22.3±0.2°;
8.4±0.2°, 4.1±0.2°, 20.8±0.2° or 22.3±0.2°;
8.1±0.2°, 8.4±0.2°, 4.1±0.2°, 20.8±0.2° or 22.3±0.2°;
8.1±0.2°, 4.1±0.2°, 20.8±0.2°, 22.3±0.2° or 18.9±0.2°;
8.4±0.2°, 4.1±0.2°, 20.8±0.2°, 22.3±0.2° or 18.9±0.2°;
8.1±0.2°, 8.4±0.2°, 4.1±0.2°, 20.8±0.2°, 22.3±0.2° or 18.9±0.2°;
8.1±0.2°, 4.1±0.2°, 20.8±0.2°, 22.3±0.2°, 18.9±0.2° or 24.5±0.2°;
8.4±0.2°, 4.1±0.2°, 20.8±0.2°, 22.3±0.2°, 18.9±0.2° or 24.5±0.2°;
8.1±0.2°, 8.4±0.2°, 4.1±0.2°, 20.8±0.2°, 22.3±0.2°, 18.9±0.2°, 24.5±0.2° or 20.0±0.2°;
8.1±0.2°, 4.1±0.2°, 20.8±0.2°, 22.3±0.2°, 18.9±0.2°, 24.5±0.2°, 20.0±0.2° or 24.3±0.2°;
8.4+0.2°, 4.1+0.2°, 20.8±0.2°, 22.3±0.2°, 18.9+0.2°, 24.5±0.2°, 20.0+0.2° or 24.3±0.2°;
8.1±0.2°, 8.4+0.2°, 4.1±0.2°, 20.8+0.2°, 22.3+0.2°, 18.9±0.2°, 20.0+0.2° or 24.3±0.2°;
8.1+0.2°, 4.1+0.2°, 20.8±0.2°, 22.3±0.2°, 18.9+0.2°, 24.5±0.2°, 24.3+0.2° or 11.1±0.2°;
8.4±0.2°, 4.1±0.2°, 20.8±0.2°, 22.3±0.2°, 18.9±0.2°, 24.5±0.2°, 24.3±0.2° or 11.1±0.2°;
the crystal form A of the sulphuric acid salt is characterized by an X-ray powder diffraction pattern comprising at least one or more diffraction peaks at 2θ = 8.3±0.2°, 20.9±0.2° or 18.5±0.2°, preferably comprising two, more preferably comprising three; optionally further comprising at least one diffraction peak at 2θ = 24.4±0.2°, 10.0±0.2°, 11.0±0.2°, 12.6±0.2°, 16.3±0.2°, 19.9±0.2° or 20.4±0.2°, preferably comprising 2, 3, 4 or 5; for example, at 2θ =
8.3±0.2° or 20.9±0.2°;
8.3±0.2° or 18.5±0.2°;
20.9±0.2° or 18.5±0.2°;
8.3±0.2°, 20.9±0.2° or 18.5±0.2°;
8.3±0.2°, 18.5±0.2° or 24.4±0.2°;
20.9±0.2°, 18.5±0.2° or 24.4±0.2°;
8.3±0.2°, 20.9+0.2°, 18.5±0.2° or 24.4±0.2°;
8.3±0.2°, 18.5±0.2°, 24.4±0.2° or 10.0±0.2°;
20.9±0.2°, 18.5±0.2°, 24.4±0.2° or 10.0±0.2°;
8.3±0.2°, 20.9±0.2°, 18.5±0.2°, 24.4±0.2° or 10.0±0.2°;
8.3±0.2°, 18.5±0.2°, 24.4±0.2°, 10.0±0.2° or 11.0±0.2°;
20.9±0.2°, 18.5±0.2°, 24.4±0.2°, 10.0±0.2° or 11.0±0.2°;
8.3±0.2°, 20.9+0.2°, 18.5±0.2°, 24.4±0.2°, 10.0±0.2° or 11.0±0.2°;
8.3±0.2°, 18.5±0.2°, 24.4±0.2°, 10.0±0.2°, 11.0±0.2° or 12.6±0.2°;
20.9±0.2°, 18.5±0.2°, 24.4±0.2°, 10.0±0.2°, 11.0±0.2° or 12.6±0.2°;
8.3±0.2°, 20.9+0.2°, 18.5±0.2°, 24.4±0.2°, 10.0±0.2°, 11.0±0.2°, 12.6±0.2° or 16.3±0.2°;
8.3±0.2°, 18.5±0.2°, 24.4±0.2°, 10.0±0.2°, 11.0±0.2°, 12.6±0.2°, 16.3±0.2° or 19.9±0.2°;
20.9±0.2°, 18.5±0.2°, 24.4±0.2°, 10.0±0.2°, 11.0±0.2°, 12.6±0.2°, 16.3±0.2° or 19.9±0.2°;
8.3+0.2°, 20.9+0.2°, 18.5+0.2°, 24.4+0.2°, 10.0+0.2°, 11.0±0.2°, 16.3+0.2° or 19.9±0.2°;
8.3+0.2°, 18.5±0.2°, 24.4+0.2°, 10.0+0.2°, 11.0+0.2°, 12.6±0.2°, 19.9+0.2° or 20.4±0.2°;
20.9±0.2°, 18.5±0.2°, 24.4±0.2°, 10.0±0.2°, 11.0±0.2°, 12.6±0.2°, 19.9±0.2° or 20.4±0.2°;
the crystal form A of the benzenesulfonic acid salt is characterized by an X-ray powder diffraction pattern comprising at least one or more diffraction peaks at 2θ = 5.9±0.2°, 23.8±0.2°, 17.8±0.2° or 29.9±0.2°, preferably comprising two, more preferably comprising three, further preferably comprising four; for example, at 2θ
5.9±0.2°;
5.9±0.2° or 23.8±0.2°;
5.9±0.2° or 17.8±0.2°;
5.9±0.2° or 29.9±0.2°;
5.9±0.2°, 23.8±0.2° or 17.8±0.2°;
5.9±0.2°, 17.8±0.2° or 29.9±0.2°;
5.9±0.2°, 23.8±0.2°, 17.8±0.2° or 29.9±0.2°;
the crystal form A of the isethionic acid salt is characterized by an X-ray powder diffraction pattern comprising at least one or more diffraction peaks at 2θ = 9.9±0.2°, 19.9±0.2° or 8.3±0.2°, preferably comprising two, more preferably comprising three; optionally further comprising at least one diffraction peak at 2θ = 20.4±0.2°, 18.3±0.2°, 23.9±0.2° or 10.6±0.2°, preferably comprising 2, 3 or 4; for example, at 20 =
9.9±0.2° or 19.9±0.2°;
9.9±0.2° or 8.3±0.2°;
19.9±0.2° or 8.3±0.2°;
9.9±0.2°, 19.9±0.2° or 8.3±0.2°;
9.9±0.2°, 8.3±0.2° or 20.4±0.2°;
19.9±0.2°, 8.3±0.2° or 20.4±0.2°;
9.9±0.2°, 19.9±0.2°, 8.3±0.2° or 20.4±0.2°;
9.9±0.2°, 8.3±0.2°, 20.4±0.2° or 18.2±0.2°;
19.9±0.2°, 8.3±0.2°, 20.4±0.2° or 18.2±0.2°;
9.9+0.2°, 19.9+0.2°, 8.3+0.2°, 20.4+0.2° or 18.2+0.2°;
9.9+0.2°, 8.3+0.2°, 20.4+0.2°, 18.2+0.2° or 23.9+0.2°;
19.9+0.2°, 8.3+0.2°, 20.4+0.2°, 18.2+0.2° or 23.9+0.2°;
9.9+0.2°, 19.9+0.2°, 8.3+0.2°, 20.4+0.2°, 18.2+0.2° or 23.9+0.2°;
9.9+0.2°, 8.3+0.2°, 20.4+0.2°, 18.2+0.2°, 23.9+0.2° or 10.6+0.2°;
19.9±0.2°, 8.3±0.2°, 20.4±0.2°, 18.2±0.2°, 23.9±0.2° or 10.6±0.2°;
9.9±0.2°, 19.9±0.2°, 8.3±0.2°, 20.4±0.2°, 18.2±0.2°, 23.9±0.2° or 10.6±0.2°;
the crystal form A of the hydrobromic acid salt is characterized by an X-ray powder diffraction pattern comprising at least one or more diffraction peaks at 2θ = 30.0±0.2°, 22.3±0.2° or 25.9±0.2°, preferably comprising two, more preferably comprising three; optionally further comprising at least one diffraction peak at 2θ = 29.7±0.2°, 17.0±0.2°, 35.7±0.2°, 33.3±0.2°, 23.4±0.2°, 28.6±0.2° or 28.8±0.2°, preferably comprising 2, 3, 4 or 5; for example, at 2θ =
30.0±0.2° or 22.3±0.2°;
30.0±0.2° or 25.9±0.2°;
22.3±0.2° or 25.9±0.2°;
30.0±0.2°, 22.3±0.2° or 25.9±0.2°;
30.0±0.2°, 25.9±0.2° or 29.7±0.2°;
22.3±0.2°, 25.9±0.2° or 29.7±0.2°;
30.0±0.2°, 22.3±0.2°, 25.9±0.2° or 29.7±0.2°;
30.0±0.2°, 25.9±0.2°, 29.7±0.2° or 17.0±0.2°;
22.3±0.2°, 25.9±0.2°, 29.7±0.2° or 17.0±0.2°;
30.0±0.2°, 22.3±0.2°, 25.9±0.2°, 29.7±0.2° or 17.0±0.2°;
30.0±0.2°, 25.9±0.2°, 29.7±0.2°, 17.0±0.2° or 35.7±0.2°;
22.3±0.2°, 25.9±0.2°, 29.7±0.2°, 17.0±0.2° or 35.7±0.2°;
30.0±0.2°, 22.3±0.2°, 25.9±0.2°, 29.7±0.2°, 17.0±0.2° or 35.7±0.2°;
30.0±0.2°, 25.9±0.2°, 29.7±0.2°, 17.0±0.2°, 35.7±0.2° or 33.3+0.2°;
22.3±0.2°, 25.9±0.2°, 29.7±0.2°, 17.0±0.2°, 35.7±0.2° or 33.3±0.2°;
30.0±0.2°, 22.3±0.2°, 25.9±0.2°, 29.7±0.2°, 17.0±0.2°, 35.7±0.2°, 33.3±0.2° or 23.4±0.2°;
30.0±0.2°, 25.9±0.2°, 29.7±0.2°, 17.0±0.2°, 35.7±0.2°, 33.3±0.2°, 23.4±0.2° or 28.6±0.2°;
22.3±0.2°, 25.9±0.2°, 29.7±0.2°, 17.0±0.2°, 35.7±0.2°, 33.3±0.2°, 23.4±0.2° or 28.6±0.2°;
30.0+0.2°, 22.3+0.2°, 25.9±0.2°, 29.7+0.2°, 17.0±0.2°, 35.7±0.2°, 23.4±0.2° or 28.6±0.2°;
30.0+0.2°, 25.9+0.2°, 29.7±0.2°, 17.0+0.2°, 35.7+0.2°, 33.3+0.2°, 28.6+0.2° or 28.8±0.2°;
22.3+0.2°, 25.9+0.2°, 29.7+0.2°, 17.0+0.2°, 35.7+0.2°, 33.3+0.2°, 28.6+0.2° or 28.8±0.2°.

In a further preferred embodiment of the present invention,
the crystal form A of the hydrochloride salt is characterized by an X-ray powder diffraction pattern comprising one or more diffraction peaks at 20 = 9.2±0.2°, 9.0±0.2°, 8.2±0.2°, 10.8±0.2°, 16.8±0.2°, 18.8±0.2°, 20.2±0.2°, 20.6±0.2°, 20.9±0.2°, 23.2±0.2°, 25.3±0.2° or 24.0±0.2°, preferably comprising any 4, 5, 6, 8 or 10 of the aforementioned diffraction peaks; for example, at 2θ =
9.2±0.2°, 9.0±0.2°, 16.8±0.2° or 18.8±0.2°;
9.2±0.2°, 8.2±0.2°, 16.8±0.2° or 18.8±0.2°;
9.2±0.2°, 9.0±0.2°, 16.8±0.2°, 18.8±0.2°, 20.2±0.2° or 20.6±0.2°;
9.2±0.2°, 8.2±0.2°, 16.8±0.2°, 18.8±0.2°, 20.2±0.2° or 20.6±0.2°;
9.2±0.2°, 9.0±0.2°, 16.8+0.2°, 18.8±0.2°, 20.2±0.2°, 20.6±0.2°, 20.9±0.2° or 23.2±0.2°;
9.2±0.2°, 8.2±0.2°, 16.8±0.2°, 18.8±0.2°, 20.2±0.2°, 20.6±0.2°, 20.9±0.2° or 23.2±0.2°;
9.2±0.2°, 9.0±0.2°, 16.8±0.2°, 18.8±0.2°, 20.2±0.2°, 20.6±0.2°, 20.9±0.2°, 23.2±0.2°, 25.3±0.2° or 24.0±0.2°;
9.2±0.2°, 8.2±0.2°, 16.8±0.2°, 18.8±0.2°, 20.2±0.2°, 20.6±0.2°, 20.9±0.2°, 23.2±0.2°, 25.3±0.2° or 24.0±0.2°;
9.2±0.2°, 9.0±0.2°, 8.2±0.2°, 10.8±0.2°, 16.8±0.2°, 18.8±0.2°, 20.2±0.2°, 20.6±0.2°, 20.9±0.2° or 23.2±0.2°;
9.2±0.2°, 8.2±0.2°, 10.8±0.2°, 16.8±0.2°, 18.8±0.2°, 20.2±0.2°, 20.6±0.2°, 20.9±0.2°, 23.2±0.2° or 25.3±0.2°;
9.0±0.2°, 8.2±0.2°, 10.8±0.2°, 16.8±0.2°, 18.8±0.2°, 20.2±0.2°, 20.6±0.2°, 20.9±0.2°, 23.2±0.2° or 25.3±0.2°;
9.2±0.2°, 9.0±0.2°, 8.2±0.2°, 10.8±0.2°, 16.8±0.2°, 18.8±0.2°, 20.6±0.2°, 20.9±0.2°, 23.2±0.2° or 25.3±0.2°;
9.2±0.2°, 8.2±0.2°, 10.8±0.2°, 16.8±0.2°, 18.8±0.2°, 20.2±0.2°, 20.9±0.2°, 23.2+0.2°, 25.3+0.2° or 24.0+0.2°;
9.0+0.2°, 8.2+0.2°, 10.8±0.2°, 16.8±0.2°, 18.8±0.2°, 20.2±0.2°, 20.9+0.2°, 23.2+0.2°, 25.3+0.2° or 24.0+0.2°;
the crystal form B of the hydrochloride salt is characterized by an X-ray powder diffraction pattern comprising one or more diffraction peaks at 2θ = 10.4+0.2°, 7.3±0.2°, 14.1±0.2°, 14.6±0.2°, 17.0±0.2°, 19.3±0.2°, 21.9±0.2°, 24.8±0.2°, 11.0±0.2°, 13.1±0.2°, 20.3±0.2°, 22.1±0.2° or 24.1±0.2°, preferably comprising any 4, 5, 6, 8 or 10 of the aforementioned diffraction peaks; for example, at 2θ =
10.4±0.2°, 7.3±0.2°, 17.0±0.2° or 19.3±0.2°;
10.4±0.2°, 14.1±0.2°, 17.0±0.2° or 19.3±0.2°;
10.4±0.2°, 7.3±0.2°, 17.0±0.2°, 19.3±0.2°, 21.9±0.2° or 24.8±0.2°;
10.4±0.2°, 14.1±0.2°, 17.0±0.2°, 19.3±0.2°, 21.9±0.2° or 24.8±0.2°;
10.4±0.2°, 7.3±0.2°, 17.0±0.2°, 19.3±0.2°, 21.9±0.2°, 24.8±0.2°, 11.0±0.2° or 13.1±0.2°;
10.4±0.2°, 14.1±0.2°, 17.0±0.2°, 19.3±0.2°, 21.9±0.2°, 24.8±0.2°, 11.0±0.2° or 13.1±0.2°;
10.4±0.2°, 7.3±0.2°, 17.0±0.2°, 19.3±0.2°, 21.9±0.2°, 24.8±0.2°, 11.0±0.2°, 13.1±0.2°, 20.3±0.2° or 22.1±0.2°;
10.4±0.2°, 14.1±0.2°, 17.0±0.2°, 19.3±0.2°, 21.9±0.2°, 24.8±0.2°, 11.0±0.2°, 13.1±0.2°, 20.3±0.2° or 22.1±0.2°;
10.4±0.2°, 7.3±0.2°, 14.1±0.2°, 14.6±0.2°, 17.0±0.2°, 19.3±0.2°, 21.9±0.2°, 24.8±0.2°, 11.0±0.2° or 13.1±0.2°;
10.4±0.2°, 14.1±0.2°, 14.6±0.2°, 17.0±0.2°, 19.3±0.2°, 21.9±0.2°, 24.8±0.2°, 11.0±0.2°, 13.1±0.2° or 20.3±0.2°;
7.3±0.2°, 14.1±0.2°, 14.6±0.2°, 17.0±0.2°, 19.3±0.2°, 21.9±0.2°, 24.8±0.2°, 11.0±0.2°, 13.1±0.2° or 20.3±0.2°;
10.4±0.2°, 7.3±0.2°, 14.1±0.2°, 14.6±0.2°, 17.0±0.2°, 19.3±0.2°, 24.8±0.2°, 11.0±0.2°, 13.1±0.2° or 20.3±0.2°;
10.4±0.2°, 14.1±0.2°, 14.6±0.2°, 17.0±0.2°, 19.3±0.2°, 21.9±0.2°, 11.0±0.2°, 13.1±0.2°, 20.3±0.2° or 22.1±0.2°;
7.3±0.2°, 14.1±0.2°, 14.6±0.2°, 17.0±0.2°, 19.3±0.2°, 21.9±0.2°, 11.0±0.2°, 13.1±0.2°, 20.3±0.2° or 22.1±0.2°;
the crystal form C of the hydrochloride salt is characterized by an X-ray powder diffraction pattern comprising one or more diffraction peaks at 20 = 8.6±0.2°, 7.9+0.2°, 14.7+0.2°, 14.9±0.2°, 6.3±0.2°, 17.4±0.2°, 10.3±0.2°, 12.4±0.2°, 23.8+0.2°, 24.8+0.2°, 16.0±0.2° or 18.2±0.2°, preferably comprising any 4, 5, 6, 8 or 10 of the aforementioned diffraction peaks; for example, at 2θ =
8.6±0.2°, 7.9±0.2°, 14.7±0.2° or 6.3±.2°;
8.6±0.2°, 7.9±0.2°, 17.4±0.2° or 10.3±0.2°;
8.6±0.2°, 7.9±0.2°, 14.7±0.2°, 6.3±0.2°, 17.4±0.2° or 10.3±0.2°;
8.6±0.2°, 7.9±0.2°, 17.4±0.2°, 10.3±0.2°, 12.4±0.2° or 23.8±0.2°;
8.6±0.2°, 7.9±0.2°, 14.7±0.2°, 6.3±0.2°, 17.4±0.2°, 10.3±0.2°, 12.4±0.2° or 23.8±0.2°;
8.6±0.2°, 7.9±0.2°, 17.4±0.2°, 10.3±0.2°, 12.4±0.2°, 23.8±0.2°, 24.8±0.2° or 16.0±0.2°;
8.6±0.2°, 7.9±0.2°, 14.7±0.2°, 6.3±0.2°, 17.4±0.2°, 10.3±0.2°, 12.4±0.2°, 23.8±0.2°, 24.8±0.2° or 16.0±0.2°;
8.6±0.2°, 7.9±0.2°, 17.4±0.2°, 10.3±0.2°, 12.4±0.2°, 23.8±0.2°, 24.8±0.2°, 16.0±0.2° or 18.2±0.2°;
8.6±0.2°, 7.9±0.2°, 14.7±0.2°, 14.9±0.2°, 6.3±0.2°, 17.4±0.2°, 10.3±0.2°, 12.4±0.2°, 23.8±0.2° or 24.8±0.2°;
8.6±0.2°, 14.7±0.2°, 14.9±0.2°, 6.3±0.2°, 17.4±0.2°, 10.3±0.2°, 12.4±0.2°, 23.8±0.2°, 24.8±0.2° or 16.0±0.2°;
7.9±0.2°, 14.7±0.2°, 14.9+0.2°, 6.3±0.2°, 17.4±0.2°, 10.3±0.2°, 12.4±0.2°, 23.8±0.2°, 24.8±0.2° or 16.0±0.2°;
the crystal form A of the ethylsulfonic acid salt is characterized by an X-ray powder diffraction pattern comprising one or more diffraction peaks at 2θ = 8.7±0.2°, 7.4±0.2°, 17.0±0.2°, 17.5±0.2°, 19.4±0.2°, 22.7±0.2°, 26.4±0.2°, 28.8±0.2°, 20.1±0.2°, 35.1±0.2°, 35.4±0.2° or 15.5±0.2°, preferably comprising any 4, 5, 6, 8 or 10 of the aforementioned diffraction peaks; for example, at 2θ =
8.7±0.2°, 7.4±0.2°, 19.4±0.2° or 22.7±0.2°;
8.7±0.2°, 17.0±0.2°, 19.4±0.2° or 22.7±0.2°;
8.7±0.2°, 7.4±0.2°, 19.4±0.2°, 22.7±0.2°, 26.4±0.2° or 28.8±0.2°;
8.7±0.2°, 17.0±0.2°, 19.4±0.2°, 22.7±0.2°, 26.4±0.2° or 28.8±0.2°;
8.7±0.2°, 7.4±0.2°, 19.4±0.2°, 22.7±0.2°, 26.4±0.2°, 28.8±0.2°, 20.1±0.2° or 35.1±0.2°;
8.7+0.2°, 17.0+0.2°, 19.4+0.2°, 22.7+0.2°, 26.4+0.2°, 28.8+0.2°, 20.1+0.2° or 35.1+0.2°;
8.7+0.2°, 7.4±0.2°, 19.4+0.2°, 22.7±0.2°, 26.4±0.2°, 28.8±0.2°, 20.1+0.2°, 35.1±0.2°, 35.4±0.2° or 15.5±0.2°;
8.7+0.2°, 17.0+0.2°, 19.4+0.2°, 22.7+0.2°, 26.4±0.2°, 28.8±0.2°, 20.1±0.2°, 35.1±0.2°, 35.4±0.2° or 15.5±0.2°;
8.7±0.2°, 7.4±0.2°, 17.0±0.2°, 17.5±0.2°, 19.4±0.2°, 22.7±0.2°, 26.4±0.2°, 28.8±0.2°, 20.1±0.2° or 35.1±0.2°;
8.7±0.2°, 17.0±0.2°, 17.5±0.2°, 19.4±0.2°, 22.7±0.2°, 26.4±0.2°, 28.8±0.2° or 20.1±0.2°;
7.4±0.2°, 17.0±0.2°, 17.5±0.2°, 19.4±0.2°, 22.7±0.2°, 26.4±0.2°, 28.8±0.2°, 20.1±0.2°, 35.1±0.2° or 35.4±0.2°;
8.7±0.2°, 7.4±0.2°, 17.0±0.2°, 17.5±0.2°, 19.4±0.2°, 22.7±0.2°, 28.8±0.2°, 20.1±0.2°, 35.1±0.2° or 35.4±0.2°;
8.7±0.2°, 17.0±0.2°, 17.5±0.2°, 19.4±0.2°, 22.7±0.2°, 26.4±0.2°, 20.1±0.2°, 35.1±0.2°, 35.4±0.2° or 15.5±0.2°;
7.4±0.2°, 17.0±0.2°, 17.5±0.2°, 19.4±0.2°, 22.7±0.2°, 26.4±0.2°, 20.1±0.2°, 35.1±0.2°, 35.4±0.2° or 15.5±0.2°;
the crystal form B of the ethylsulfonic acid salt is characterized by an X-ray powder diffraction pattern comprising one or more diffraction peaks at 2θ = 8.8±0.2°, 8.3±0.2°, 7.9±0.2°, 17.0±0.2°, 16.2±0.2°, 18.4±0.2°, 20.6±0.2°, 24.1±0.2°, 26.4±0.2°, 10.8±0.2°, 12.6±0.2° or 19.9±0.2°, preferably comprising any 4, 5, 6, 8 or 10 of the aforementioned diffraction peaks; for example, at 2θ =
8.8±0.2°, 8.3±0.2°, 16.2±0.2° or 18.4±0.2°;
8.8±0.2°, 7.9±0.2°, 16.2±0.2° or 18.4±0.2°;
8.8±0.2°, 8.3±0.2°, 16.2±0.2°, 18.4±0.2°, 20.6±0.2° or 24.1±0.2°;
8.8±0.2°, 7.9±0.2°, 16.2±0.2°, 18.4±0.2°, 20.6±0.2° or 24.1±0.2°;
8.8±0.2°, 8.3±0.2°, 16.2±0.2°, 18.4±0.2°, 20.6±0.2°, 24.1±0.2°, 26.4±0.2° or 10.8±0.2°;
8.8±0.2°, 7.9±0.2°, 16.2±0.2°, 18.4±0.2°, 20.6±0.2°, 24.1±0.2°, 26.4±0.2° or 10.8±0.2°;
8.8±0.2°, 8.3±0.2°, 16.2±0.2°, 18.4±0.2°, 20.6±0.2°, 24.1±0.2°, 26.4±0.2°, 10.8±0.2°, 12.6±0.2° or 19.9±0.2°;
8.8+0.2°, 7.9±0.2°, 16.2±0.2°, 18.4±0.2°, 20.6±0.2°, 24.1+0.2°, 26.4+0.2°, 10.8+0.2°, 12.6±0.2° or 19.9+0.2°;
8.8+0.2°, 8.3±0.2°, 7.9±0.2°, 17.0+0.2°, 16.2±0.2°, 18.4±0.2°, 20.6+0.2°, 24.1±0.2°, 26.4±0.2° or 10.8±0.2°;
8.8+0.2°, 7.9+0.2°, 17.0±0.2°, 16.2±0.2°, 18.4+0.2°, 20.6+0.2°, 24.1±0.2° or 26.4±0.2°;
8.3±0.2°, 7.9±0.2°, 17.0±0.2°, 16.2±0.2°, 18.4±0.2°, 20.6±0.2°, 24.1±0.2°, 26.4±0.2°, 10.8±0.2° or 12.6±0.2°;
8.8±0.2°, 8.3±0.2°, 7.9±0.2°, 17.0±0.2°, 16.2±0.2°, 18.4±0.2°, 24.1±0.2°, 26.4±0.2°, 10.8±0.2° or 12.6±0.2°;
8.8±0.2°, 7.9±0.2°, 17.0±0.2°, 16.2±0.2°, 18.4±0.2°, 20.6±0.2°, 26.4±0.2°, 10.8±0.2°, 12.6±0.2° or 19.9±0.2°;
8.3±0.2°, 7.9±0.2°, 17.0±0.2°, 16.2±0.2°, 18.4±0.2°, 20.6±0.2°, 26.4±0.2°, 10.8±0.2°, 12.6±0.2° or 19.9±0.2°;
the crystal form A of the p-toluenesulfonic acid salt is characterized by an X-ray powder diffraction pattern comprising one or more diffraction peaks at 2θ = 5.8±0.2°, 6.2±0.2°, 18.6±0.2°, 23.2±0.2° or 29.1±0.2°, preferably comprising any 4, 5 or 6 of the aforementioned diffraction peaks; for example, at 2θ =
5.8±0.2°, 6.2±0.2°, 23.2±0.2° or 29.1±0.2°;
5.8±0.2°, 6.2±0.2°, 18.6±0.2° or 29.1±0.2°;
the crystal form A of the methanesulfonic acid salt is characterized by an X-ray powder diffraction pattern comprising one or more diffraction peaks at 2θ = 8.1±0.2°, 8.4±0.2°, 4.1±0.2°, 20.8±0.2°, 22.3±0.2°, 18.9±0.2°, 24.5±0.2°, 20.0±0.2°, 24.3±0.2°, 11.1±0.2°, 12.6±0.2° or 16.5±0.2°, preferably comprising any 4, 5, 6, 8 or 10 of the aforementioned diffraction peaks; for example, at 2θ =
8.1±0.2°, 8.4±0.2°, 22.3±0.2° or 18.9±0.2°;
8.1±0.2°, 4.1±0.2°, 22.3±0.2° or 18.9±0.2°;
8.1±0.2°, 8.4±0.2°, 22.3±0.2°, 18.9±0.2°, 24.5±0.2° or 20.0±0.2°;
8.1±0.2°, 4.1±0.2°, 22.3±0.2°, 18.9±0.2°, 24.5±0.2° or 20.0±0.2°;
8.1±0.2°, 8.4±0.2°, 22.3±0.2°, 18.9±0.2°, 24.5±0.2°, 20.0±0.2°, 24.3±0.2° or 11.1±0.2°;
8.1±0.2°, 4.1±0.2°, 22.3±0.2°, 18.9±0.2°, 24.5±0.2°, 20.0±0.2°, 24.3±0.2° or 11.1±0.2°;
8.1±0.2°, 8.4±0.2°, 22.3±0.2°, 18.9±0.2°, 24.5±0.2°, 20.0±0.2°, 24.3±0.2°, 11.1±0.2°, 12.6+0.2° or 16.5+0.2°;
8.1±0.2°, 4.1+0.2°, 22.3±0.2°, 18.9±0.2°, 24.5±0.2°, 20.0±0.2°, 24.3+0.2°, 11.1±0.2°, 12.6±0.2° or 16.5+0.2°;
8.1±0.2°, 8.4±0.2°, 4.1±0.2°, 20.8±0.2°, 22.3±0.2°, 18.9±0.2°, 24.5+0.2°, 20.0+0.2°, 24.3±0.2° or 11.1±0.2°;
8.1±0.2°, 4.1±0.2°, 20.8±0.2°, 22.3±0.2°, 18.9±0.2°, 24.5±0.2°, 20.0±0.2°, 24.3±0.2°, 11.1±0.2° or 12.6±0.2°;
8.4±0.2°, 4.1±0.2°, 20.8±0.2°, 22.3±0.2°, 18.94±0.2°, 24.5±0.2°, 20.0±0.2°, 24.3±0.2°, 11.1±0.2° or 12.6±0.2°;
8.1±0.2°, 8.4±0.2°, 4.1±0.2°, 20.8±0.2°, 22.3±0.2°, 18.9±0.2°, 20.0±0.2°, 24.3±0.2°, 11.1±0.2° or 12.6±0.2°;
8.1±0.2°, 4.1±0.2°, 20.8±0.2°, 22.3±0.2°, 18.9±0.2°, 24.5±0.2°, 24.3±0.2°, 11.1±0.2°, 12.6±0.2° or 16.5±0.2°;
8.4±0.2°, 4.1±0.2°, 20.8±0.2°, 22.3±0.2°, 18.9±0.2°, 24.5±0.2°, 24.3±0.2°, 11.1±0.2°, 12.6±0.2° or 16.5±0.2°;
the crystal form A of the sulphuric acid salt is characterized by an X-ray powder diffraction pattern comprising one or more diffraction peaks at 2θ = 8.3±0.2°, 20.9±0.2°, 18.5±0.2°, 24.4±0.2°, 10.0±0.2°, 11.0±0.2°, 12.6±0.2°, 16.3±0.2°, 19.9±0.2°, 20.4±0.2°, 21.9±0.2° or 26.7±0.2°, preferably comprising any 4, 5, 6, 8 or 10 of the aforementioned diffraction peaks; for example, at 2θ =
8.3±0.2°, 20.9±0.2°, 10.0±0.2° or 11.0±0.2°;
8.3±0.2°, 18.5±0.2°, 10.0±0.2° or 11.0±0.2°;
8.3±0.2°, 20.9±0.2°, 10.0±0.2°, 11.0±0.2°, 12.6±0.2° or 16.3±0.2°;
8.3±0.2°, 18.5±0.2°, 10.0±0.2°, 11.0±0.2°, 12.6±0.2° or 16.3±0.2°;
8.3±0.2°, 20.9±0.2°, 10.0±0.2°, 11.0±0.2°, 12.6±0.2°, 16.3±0.2°, 19.9±0.2° or 20.4±0.2°;
8.3±0.2°, 18.5±0.2°, 10.0±0.2°, 11.0±0.2°, 12.6±0.2°, 16.3±0.2°, 19.9±0.2° or 20.4±0.2°;
8.3±0.2°, 20.9±0.2°, 10.0±0.2°, 11.0±0.2°, 12.6±0.2°, 16.3±0.2°, 19.9±0.2°, 20.4±0.2°, 21.9±0.2° or 26.7±0.2°;
8.3±0.2°, 18.5±0.2°, 10.0±0.2°, 11.0±0.2°, 12.6±0.2°, 16.3±0.2°, 19.9±0.2°, 20.4±0.2°, 21.9±0.2° or 26.7±0.2°;
8.3±0.2°, 20.9±0.2°, 18.5±0.2°, 24.4±0.2°, 10.0±0.2°, 11.0±0.2°, 12.6±0.2°, 16.3+0.2°, 19.9+0.2° or 20.4+0.2°;
8.3+0.2°, 18.5+0.2°, 24.4+0.2°, 10.0+0.2°, 11.0±0.2°, 12.6±0.2°, 16.3±0.2°, 19.9+0.2°, 20.4+0.2° or 21.9+0.2°;
20.9+0.2°, 18.5+0.2°, 24.4+0.2°, 10.0+0.2°, 11.0±0.2°, 12.6±0.2°, 16.3+0.2°, 19.9+0.2°, 20.4+0.2° or 21.9+0.2°;
8.3±0.2°, 20.9±0.2°, 18.5±0.2°, 24.4±0.2°, 10.0±0.2°, 11.0±0.2°, 16.3±0.2°, 19.9±0.2°, 20.4±0.2° or 21.9±0.2°;
8.3±0.2°, 18.5±0.2°, 24.4±0.2°, 10.0±0.2°, 11.0±0.2°, 12.6±0.2°, 19.9±0.2°, 20.4±0.2°, 21.9±0.2° or 26.7±0.2°;
20.9±0.2°, 18.5±0.2°, 24.4±0.2°, 10.0±0.2°, 11.0±0.2°, 12.6±0.2°, 19.9±0.2°, 20.4±0.2°, 21.9±0.2° or 26.7±0.2°;
the crystal form A of the isethionic acid salt is characterized by an X-ray powder diffraction pattern comprising one or more diffraction peaks at 2θ = 9.9±0.2°, 19.9±0.2°, 8.3±0.2°, 20.5±0.2°, 18.2±0.2°, 23.9±0.2° or 10.6±0.2°, preferably comprising any 4, 5 or 6 of the aforementioned diffraction peaks; for example, at 2θ
9.9±0.2°, 19.9±0.2°, 20.4±0.2° or 18.2±0.2°;
9.9±0.2°, 8.3±0.2°, 20.5±0.2° or 18.2±0.2°;
9.9±0.2°, 19.9±0.2°, 20.5±0.2°, 18.2±0.2°, 23.9±0.2° or 10.6±0.2°;
9.9±0.2°, 8.3±0.2°, 20.5±0.2°, 18.2±0.2°, 23.9±0.2° or 10.6±0.2°;
the crystal form A of the hydrobromic acid salt is characterized by an X-ray powder diffraction pattern comprising one or more diffraction peaks at 2θ = 30.0±0.2°, 22.3±0.2°, 25.9±0.2°, 29.7+0.2°, 17.0±0.2°, 35.7+0.2°, 33.3+0.2°, 23.4±0.2°, 28.6±0.2°, 28.8+0.2°, 18.1±0.2° or 26.6±0.2°, preferably comprising any 4, 5, 6, 8 or 10 of the aforementioned diffraction peaks; for example, at 2θ =
30.0±0.2°, 22.3+0.2°, 17.0±0.2° or 35.7±0.2°;
30.0±0.2°, 25.9±0.2°, 17.0±0.2° or 35.7±0.2°;
30.0±0.2°, 22.3+0.2°, 17.0±0.2°, 35.7+0.2°, 33.3+0.2° or 23.4±0.2°;
30.0±0.2°, 25.9±0.2°, 17.0±0.2°, 35.7+0.2°, 33.3+0.2° or 23.4±0.2°;
30.0±0.2°, 22.3±0.2°, 17.0±0.2°, 35.7±0.2°, 33.3±0.2°, 23.4±0.2°, 28.6±0.2° or 28.8±0.2°;
30.0±0.2°, 25.9±0.2°, 17.0±0.2°, 35.7±0.2°, 33.3±0.2°, 23.4±0.2°, 28.6±0.2° or 28.8±0.2°;
30.0±0.2°, 22.3±0.2°, 17.0±0.2°, 35.7±0.2°, 33.3±0.2°, 23.4±0.2°, 28.6±0.2°, 28.8+0.2°, 18.1±0.2° or 26.6+0.2°;
30.0+0.2°, 25.9+0.2°, 17.0+0.2°, 35.7+0.2°, 33.3+0.2°, 23.4±0.2°, 28.6+0.2°, 28.8+0.2°, 18.1±0.2° or 26.6+0.2°;
30.0+0.2°, 22.3+0.2°, 25.9+0.2°, 29.7+0.2°, 17.0±0.2°, 35.7+0.2°, 33.3+0.2°, 23.4+0.2°, 28.6+0.2° or 28.8±0.2°;
30.0±0.2°, 25.9±0.2°, 29.7±0.2°, 17.0±0.2°, 35.7+0.2°, 33.3+0.2°, 23.4±0.2°, 28.6±0.2°, 28.8±0.2° or 18.1±0.2°;
22.3±0.2°, 25.9±0.2°, 29.7±0.2°, 17.0±0.2°, 35.7+0.2°, 33.3+0.2°, 23.4±0.2°, 28.6±0.2°, 28.8±0.2° or 18.1±0.2°;
30.0±0.2°, 22.3±0.2°, 25.9±0.2°, 29.7±0.2°, 17.0±0.2°, 35.7+0.2°, 23.4±0.2°, 28.6±0.2°, 28.8±0.2° or 18.1±0.2°;
30.0±0.2°, 25.9±0.2°, 29.7±0.2°, 17.0±0.2°, 35.7+0.2°, 33.3±0.2°, 28.6±0.2°, 28.8±0.2°, 18.1±0.2° or 26.6±0.2°;
22.3±0.2°, 25.9±0.2°, 29.7±0.2°, 17.0±0.2°, 35.7+0.2°, 33.3±0.2°, 28.6±0.2°, 28.8±0.2°, 18.1±0.2° or 26.6±0.2°.

In a further preferred embodiment of the present invention,
the crystal form A of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide has a melting point of 155°C-176°C;
the crystal form B of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide has a melting point of 169.4-174.9°C;
the crystal form A of the methanesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide has a melting point of 209°C-217°C;
the crystal form A of the sulphuric acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide has a melting point of 195°C-209°C;
the crystal form A of the hydrobromic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide has a melting point of 158°C-165°C.

In a further preferred embodiment of the present invention,
the crystal form A of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 1.

**Table 1**

| Serial No. | XRPD data of crystal form A of hydrochloride salt | | | |
|---|---|---|---|---|
| | 2θ (±0.2 °) | d value | Peak height | Proportion (I%) |
| 1 | 5.999 | 14.7204 | 58 | 2.3 |
| 2 | 8.233 | 10.731 | 101 | 4.1 |
| 3 | 8.965 | 9.8563 | 702 | 28.3 |
| 4 | 9.166 | 9.6398 | 2479 | 100 |
| 5 | 10.109 | 8.743 | 76 | 3.1 |
| 6 | 10.786 | 8.1959 | 141 | 5.7 |
| 7 | 11.892 | 7.4357 | 86 | 3.5 |
| 8 | 13.746 | 6.4366 | 71 | 2.9 |
| 9 | 14.516 | 6.097 | 82 | 3.3 |
| 10 | 15.799 | 5.6046 | 62 | 2.5 |
| 11 | 16.18 | 5.4737 | 54 | 2.2 |
| 12 | 16.409 | 5.3977 | 40 | 1.6 |
| 13 | 16.772 | 5.2816 | 193 | 7.8 |
| 14 | 17.041 | 5.1987 | 69 | 2.8 |
| 15 | 17.362 | 5.1035 | 65 | 2.6 |
| 16 | 18.169 | 4.8786 | 96 | 3.9 |
| 17 | 18.779 | 4.7214 | 135 | 5.4 |
| 18 | 20.16 | 4.4009 | 142 | 5.7 |
| 19 | 20.643 | 4.299 | 123 | 5 |
| 20 | 20.946 | 4.2376 | 130 | 5.2 |
| 21 | 21.536 | 4.1229 | 70 | 2.8 |
| 22 | 22.267 | 3.9891 | 99 | 4 |
| 23 | 22.732 | 3.9086 | 55 | 2.2 |
| 24 | 23.223 | 3.827 | 148 | 6 |
| 25 | 24.01 | 3.7034 | 122 | 4.9 |
| 26 | 25.291 | 3.5185 | 159 | 6.4 |
| 27 | 27.595 | 3.2298 | 113 | 4.6 |
| 28 | 28.131 | 3.1695 | 59 | 2.4 |
| 29 | 29.508 | 3.0246 | 49 | 2 |

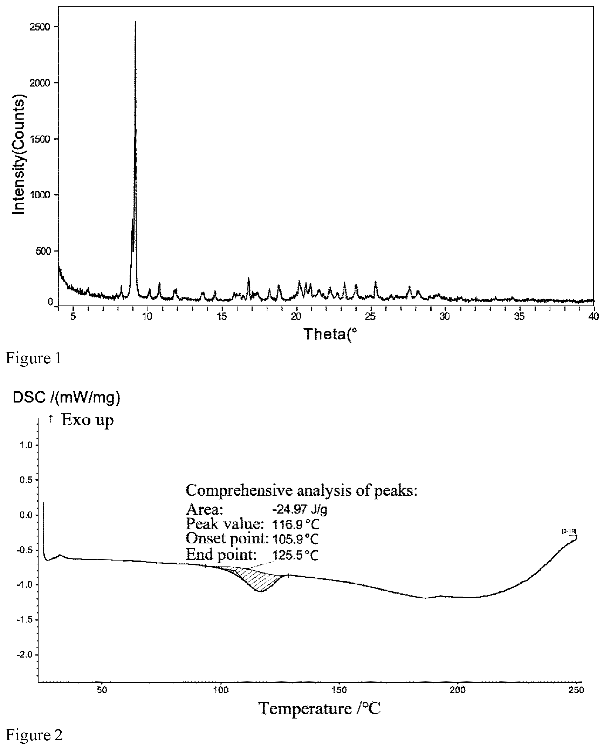

The crystal form A of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 1, and has a DSC pattern substantially as shown in FIG. 2.

The crystal form B of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 2.

**Table 2**

| Serial No. | XRPD data of crystal form B of hydrochloride salt | | | |
|---|---|---|---|---|
| | 2θ (±0.2 °) | d value | Peak height | Proportion (I%) |
| 1 | 7.281 | 12.1311 | 822 | 25 |
| 2 | 10.424 | 8.4796 | 3291 | 100 |
| 3 | 11.033 | 8.0125 | 389 | 11.8 |
| 4 | 11.94 | 7.4062 | 216 | 6.6 |
| 5 | 13.087 | 6.7592 | 296 | 9 |
| 6 | 13.472 | 6.5672 | 215 | 6.5 |
| 7 | 14.055 | 6.2959 | 734 | 22.3 |
| 8 | 14.56 | 6.0788 | 999 | 30.4 |
| 9 | 15.422 | 5.7409 | 159 | 4.8 |
| 10 | 15.823 | 5.5961 | 246 | 7.5 |
| 11 | 16.167 | 5.4779 | 276 | 8.4 |
| 12 | 17.018 | 5.206 | 1012 | 30.8 |
| 13 | 17.783 | 4.9835 | 432 | 13.1 |
| 14 | 18.654 | 4.7527 | 290 | 8.8 |
| 15 | 19.264 | 4.6036 | 1263 | 38.4 |
| 16 | 20.301 | 4.3707 | 397 | 12.1 |
| 17 | 20.944 | 4.238 | 191 | 5.8 |
| 18 | 21.901 | 4.055 | 797 | 24.2 |
| 19 | 22.128 | 4.0139 | 464 | 14.1 |
| 20 | 23.016 | 3.8609 | 318 | 9.7 |
| 21 | 23.723 | 3.7474 | 259 | 7.9 |
| 22 | 24.13 | 3.6851 | 452 | 13.7 |
| 23 | 24.516 | 3.628 | 156 | 4.7 |
| 24 | 24.759 | 3.5929 | 624 | 19 |
| 25 | 24.987 | 3.5607 | 537 | 16.3 |
| 26 | 25.348 | 3.5108 | 225 | 6.8 |
| 27 | 26.277 | 3.3888 | 296 | 9 |
| 28 | 26.772 | 3.3272 | 216 | 6.6 |
| 29 | 27.109 | 3.2866 | 231 | 7 |
| 30 | 27.295 | 3.2646 | 322 | 9.8 |
| 31 | 28.635 | 3.1148 | 275 | 8.4 |

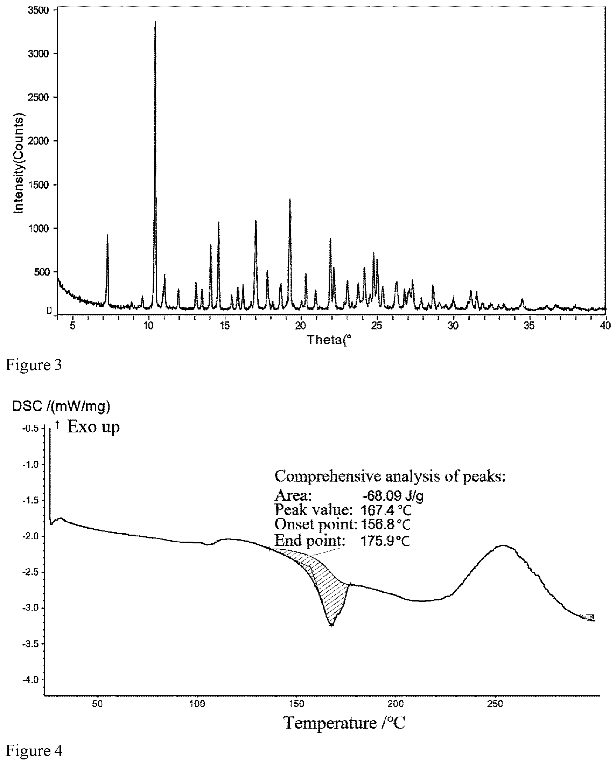

The crystal form B of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 3, and has a DSC pattern substantially as shown in FIG. 4.

The crystal form A of the ethylsulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 3.

**Table 3**

| Serial No. | XRPD data of crystal form A of ethylsulfonic acid salt | | | |
|---|---|---|---|---|
| | 2θ (±0.2 °) | d value | Peak height | Proportion (I%) |
| 1 | 7.439 | 11.8733 | 879 | 21.5 |
| 2 | 8.739 | 10.1101 | 4091 | 100 |
| 3 | 9.251 | 9.5513 | 54 | 1.3 |
| 4 | 12.46 | 7.0982 | 53 | 1.3 |
| 5 | 14.87 | 5.9525 | 60 | 1.5 |
| 6 | 15.113 | 5.8576 | 77 | 1.9 |
| 7 | 15.475 | 5.7212 | 94 | 2.3 |
| 8 | 15.784 | 5.61 | 71 | 1.7 |
| 9 | 17 | 5.2115 | 399 | 9.8 |
| 10 | 17.523 | 5.057 | 238 | 5.8 |
| 11 | 17.766 | 4.9882 | 70 | 1.7 |
| 12 | 18.066 | 4.9062 | 77 | 1.9 |
| 13 | 18.436 | 4.8084 | 100 | 2.4 |
| 14 | 19.41 | 4.5695 | 443 | 10.8 |
| 15 | 20.137 | 4.4059 | 161 | 3.9 |
| 16 | 20.867 | 4.2534 | 76 | 1.9 |
| 17 | 22.677 | 3.9178 | 296 | 7.2 |
| 18 | 24.051 | 3.697 | 151 | 3.7 |
| 19 | 26.362 | 3.378 | 562 | 13.7 |
| 20 | 26.827 | 3.3205 | 101 | 2.5 |
| 21 | 28.792 | 3.0982 | 276 | 6.7 |
| 22 | 31.695 | 2.8207 | 116 | 2.8 |
| 23 | 35.053 | 2.5578 | 206 | 5 |
| 24 | 35.37 | 2.5356 | 171 | 4.2 |

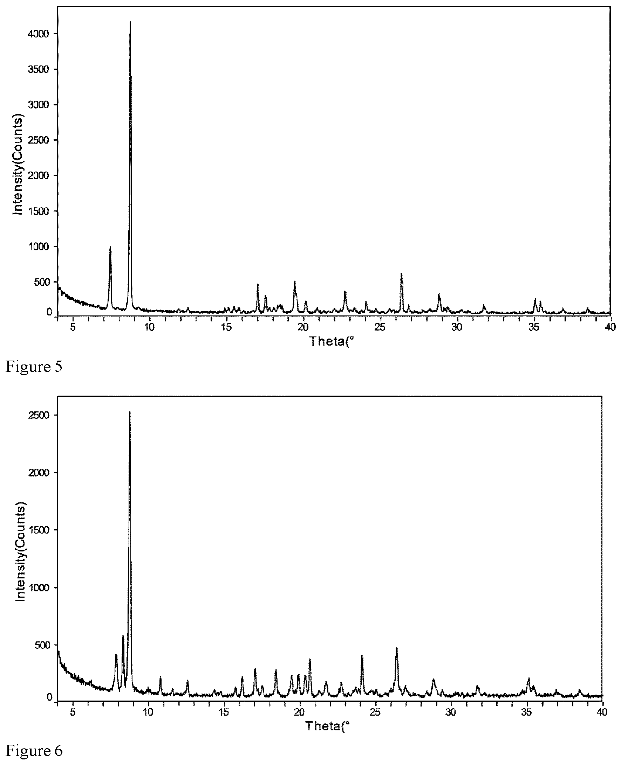

The crystal form A of the ethylsulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 5.

The crystal form B of the ethylsulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 4.

**Table 4**

| Serial No. | XRPD data of crystal form B of ethylsulfonic acid salt | | | |
|---|---|---|---|---|
| | 2θ (±0.2 °) | d value | Peak height | Proportion (I%) |
| 1 | 7.854 | 11.247 | 310 | 12.7 |
| 2 | 8.317 | 10.6229 | 487 | 20 |
| 3 | 8.762 | 10.0833 | 2435 | 100 |
| 4 | 10.789 | 8.1936 | 154 | 6.3 |
| 5 | 12.578 | 7.0319 | 126 | 5.2 |
| 6 | 15.739 | 5.626 | 77 | 3.2 |
| 7 | 16.183 | 5.4725 | 173 | 7.1 |
| 8 | 17.036 | 5.2005 | 243 | 10 |
| 9 | 17.484 | 5.0681 | 90 | 3.7 |
| 10 | 18.412 | 4.8148 | 237 | 9.7 |
| 11 | 19.446 | 4.5609 | 175 | 7.2 |
| 12 | 19.91 | 4.4557 | 190 | 7.8 |
| 13 | 20.358 | 4.3587 | 175 | 7.2 |
| 14 | 20.648 | 4.2982 | 318 | 13.1 |
| 15 | 21.701 | 4.0918 | 124 | 5.1 |
| 16 | 22.716 | 3.9113 | 119 | 4.9 |
| 17 | 24.094 | 3.6907 | 356 | 14.6 |
| 18 | 26.382 | 3.3755 | 422 | 17.3 |
| 19 | 26.968 | 3.3034 | 90 | 3.7 |
| 20 | 28.778 | 3.0997 | 153 | 6.3 |
| 21 | 31.697 | 2.8205 | 91 | 3.7 |
| 22 | 35.115 | 2.5535 | 157 | 6.4 |
| 23 | 35.402 | 2.5334 | 89 | 3.7 |

The crystal form B of the ethylsulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 6.

The crystal form A of the p-toluenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 5.

**Table 5**

| Serial No. | XRPD data of crystal form A of p-toluenesulfonic acid salt | | | |
|---|---|---|---|---|
| | 2θ (±0.2 °) | d value | Peak height | Proportion (I%) |
| 1 | 5.761 | 15.3274 | 7383 | 100 |
| 2 | 6.19 | 14.267 | 116 | 1.6 |
| 3 | 15.623 | 5.6674 | 46 | 0.6 |
| 4 | 17.355 | 5.1055 | 39 | 0.5 |
| 5 | 18.597 | 4.7673 | 109 | 1.5 |
| 6 | 21.604 | 4.11 | 43 | 0.6 |
| 7 | 21.976 | 4.0412 | 60 | 0.8 |
| 8 | 23.177 | 3.8346 | 92 | 1.2 |
| 9 | 25.352 | 3.5102 | 46 | 0.6 |
| 10 | 26.646 | 3.3427 | 36 | 0.5 |
| 11 | 28.495 | 3.1298 | 67 | 0.9 |
| 12 | 29.06 | 3.0702 | 99 | 1.3 |
| 13 | 29.833 | 2.9924 | 77 | 1 |
| 14 | 31.655 | 2.8242 | 34 | 0.5 |
| 15 | 33.92 | 2.6406 | 55 | 0.7 |
| 16 | 34.854 | 2.572 | 52 | 0.7 |

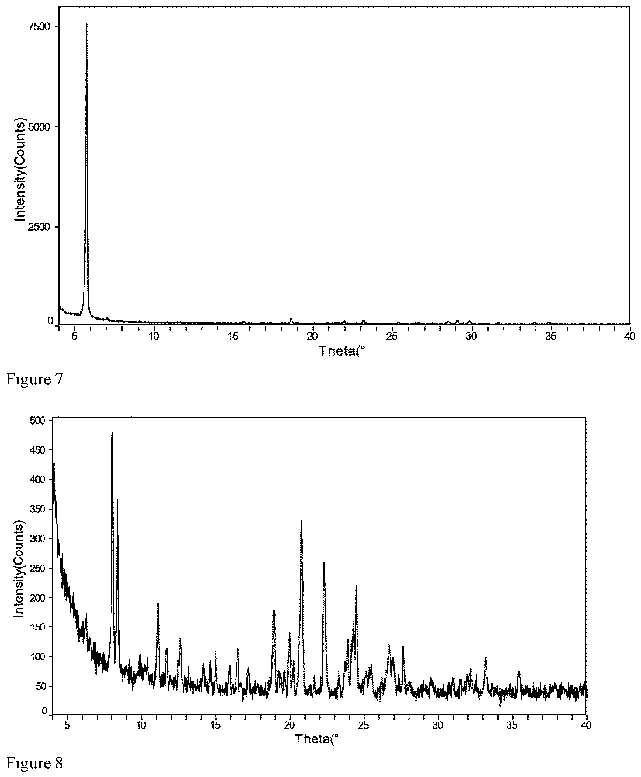

The crystal form A of the p-toluenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 7.

The crystal form A of the methanesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 6.

**Table 6**

| Serial No. | XRPD data of crystal form A of methanesulfonic acid salt | | | |
|---|---|---|---|---|
| | 2θ (±0.2 °) | d value | Peak height | Proportion (I%) |
| 1 | 4.14 | 21.3267 | 203 | 51.3 |
| 2 | 8.052 | 10.9706 | 396 | 100 |
| 3 | 8.385 | 10.5366 | 292 | 73.7 |
| 4 | 9.948 | 8.8838 | 45 | 11.4 |
| 5 | 10.386 | 8.5107 | 41 | 10.4 |
| 6 | 11.097 | 7.9668 | 135 | 34.1 |
| 7 | 11.695 | 7.5604 | 59 | 14.9 |
| 8 | 12.583 | 7.0291 | 74 | 18.7 |
| 9 | 14.178 | 6.2416 | 41 | 10.4 |
| 10 | 14.62 | 6.0537 | 47 | 11.9 |
| 11 | 15.972 | 5.5443 | 41 | 10.4 |
| 12 | 16.485 | 5.3731 | 74 | 18.7 |
| 13 | 17.178 | 5.1577 | 41 | 10.4 |
| 14 | 18.902 | 4.6909 | 140 | 35.4 |
| 15 | 19.957 | 4.4452 | 96 | 24.2 |
| 16 | 20.23 | 4.386 | 50 | 12.6 |
| 17 | 20.768 | 4.2736 | 291 | 73.5 |
| 18 | 22.287 | 3.9855 | 220 | 55.6 |
| 19 | 23.723 | 3.7474 | 47 | 11.9 |
| 20 | 23.892 | 3.7214 | 84 | 21.2 |
| 21 | 24.253 | 3.6667 | 114 | 28.8 |
| 22 | 24.474 | 3.6341 | 182 | 46 |
| 23 | 25.489 | 3.4918 | 43 | 10.9 |
| 24 | 26.649 | 3.3423 | 75 | 18.9 |
| 25 | 26.926 | 3.3085 | 54 | 13.6 |
| 26 | 27.605 | 3.2287 | 74 | 18.7 |
| 27 | 32.167 | 2.7805 | 40 | 10.1 |
| 28 | 33.175 | 2.6982 | 62 | 15.7 |
| 29 | 35.385 | 2.5346 | 42 | 10.6 |

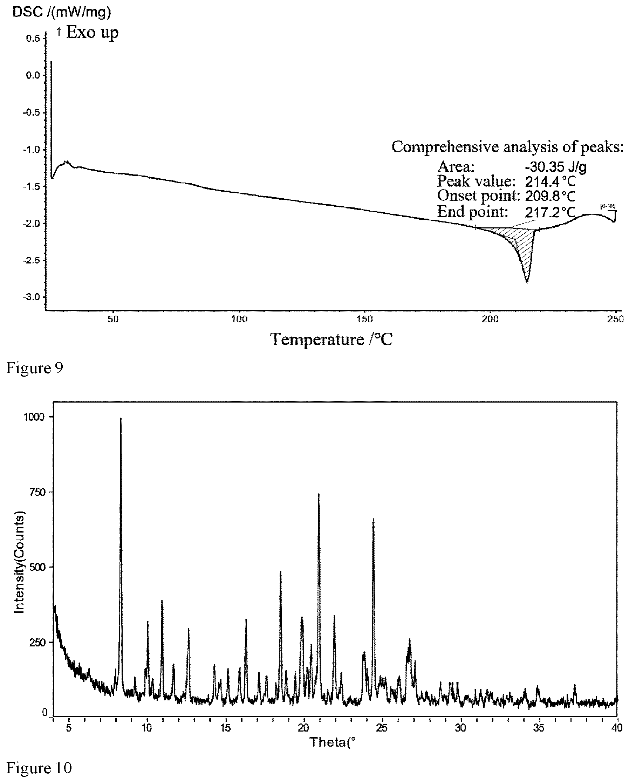

The crystal form A of the methanesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 8, and has a DSC pattern substantially as shown in FIG. 9.

The crystal form A of the sulphuric acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 7.

**Table 7**

| Serial No. | XRPD data of crystal form A of sulphuric acid salt | | | |
|---|---|---|---|---|
| | 2θ (±0.2 °) | d value | Peak height | Proportion (I%) |
| 1 | 7.968 | 11.0871 | 78 | 8.4 |
| 2 | 8.32 | 10.6187 | 925 | 100 |
| 3 | 9.215 | 9.589 | 63 | 6.8 |
| 4 | 10.04 | 8.8026 | 253 | 27.4 |
| 5 | 10.345 | 8.5441 | 59 | 6.4 |
| 6 | 10.952 | 8.0716 | 324 | 35 |
| 7 | 11.681 | 7.5695 | 118 | 12.8 |
| 8 | 12.638 | 6.9984 | 239 | 25.8 |
| 9 | 14.273 | 6.2003 | 125 | 13.5 |
| 10 | 14.663 | 6.0361 | 73 | 7.9 |
| 11 | 15.131 | 5.8504 | 111 | 12 |
| 12 | 15.875 | 5.5781 | 112 | 12.1 |
| 13 | 16.286 | 5.4383 | 272 | 29.4 |
| 14 | 17.113 | 5.1771 | 91 | 9.8 |
| 15 | 17.596 | 5.036 | 84 | 9.1 |
| 16 | 18.2 | 4.8703 | 56 | 6.1 |
| 17 | 18.494 | 4.7936 | 430 | 46.5 |
| 18 | 18.84 | 4.7062 | 98 | 10.6 |
| 19 | 19.428 | 4.5651 | 88 | 9.5 |
| 20 | 19.853 | 4.4684 | 264 | 28.5 |
| 21 | 20.216 | 4.389 | 113 | 12.2 |
| 22 | 20.441 | 4.3412 | 187 | 20.2 |
| 23 | 20.931 | 4.2407 | 696 | 75.2 |
| 24 | 21.922 | 4.0512 | 291 | 31.5 |
| 25 | 22.363 | 3.9723 | 105 | 11.4 |
| 26 | 23.846 | 3.7283 | 157 | 17 |
| 27 | 24.015 | 3.7026 | 101 | 10.9 |
| 28 | 24.417 | 3.6426 | 614 | 66.4 |
| 29 | 24.859 | 3.5787 | 72 | 7.8 |
| 30 | 25.186 | 3.5329 | 72 | 7.8 |
| 31 | 26.06 | 3.4164 | 75 | 8.1 |
| 32 | 26.625 | 3.3453 | 158 | 17.1 |
| 33 | 26.73 | 3.3324 | 205 | 22.2 |
| 34 | 27.069 | 3.2914 | 136 | 14.7 |

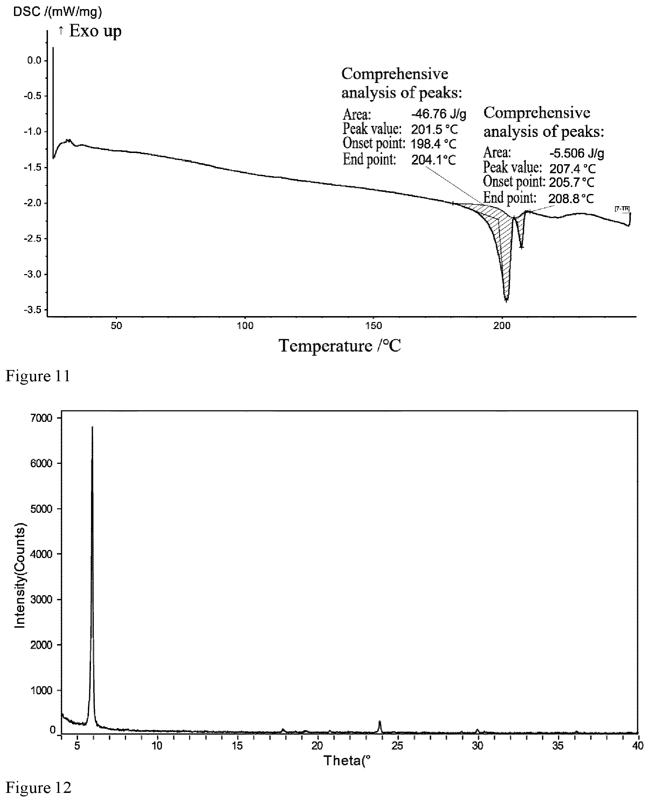

The crystal form A of the sulphuric acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 10, and has a DSC pattern substantially as shown in FIG. 11.

The crystal form A of the benzenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 8.

**Table 8**

| Serial No. | XRPD data of crystal form A of benzenesulfonic acid salt | | | |
|---|---|---|---|---|
| | 2θ (±0.2 °) | d value | Peak height | Proportion (I%) |
| 1 | 5.926 | 14.9025 | 6627 | 100 |
| 2 | 17.794 | 4.9806 | 74 | 1.1 |
| 3 | 19.204 | 4.618 | 39 | 0.6 |
| 4 | 20.73 | 4.2813 | 50 | 0.8 |
| 5 | 23.835 | 3.7302 | 255 | 3.8 |
| 6 | 28.969 | 3.0797 | 35 | 0.5 |
| 7 | 29.932 | 2.9828 | 78 | 1.2 |
| 8 | 36.09 | 2.4867 | 42 | 0.6 |

The crystal form A of the benzenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 12.

The crystal form A of the isethionic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 9.

**Table 9**

| Serial No. | XRPD data of crystal form A of isethionic acid salt | | | |
|---|---|---|---|---|
| | 2θ (±0.2 °) | d value | Peak height | Proportion (I%) |
| 1 | 6.11 | 14.4538 | 113 | 3.7 |
| 2 | 8.277 | 10.6731 | 483 | 15.6 |
| 3 | 9.934 | 8.8963 | 3088 | 100 |
| 4 | 10.636 | 8.3111 | 159 | 5.1 |
| 5 | 11.484 | 7.6989 | 57 | 1.8 |
| 6 | 12.55 | 7.0472 | 137 | 4.4 |
| 7 | 14.241 | 6.2143 | 93 | 3 |
| 8 | 14.505 | 6.1016 | 55 | 1.8 |
| 9 | 15.651 | 5.6575 | 55 | 1.8 |
| 10 | 16.049 | 5.5179 | 122 | 4 |
| 11 | 18.233 | 4.8616 | 272 | 8.8 |
| 12 | 19.891 | 4.46 | 933 | 30.2 |
| 13 | 20.462 | 4.3367 | 530 | 17.2 |
| 14 | 21.456 | 4.138 | 150 | 4.9 |
| 15 | 23.91 | 3.7186 | 383 | 12.4 |
| 16 | 25.044 | 3.5527 | 107 | 3.5 |
| 17 | 26.12 | 3.4088 | 119 | 3.9 |
| 18 | 29.884 | 2.9875 | 142 | 4.6 |

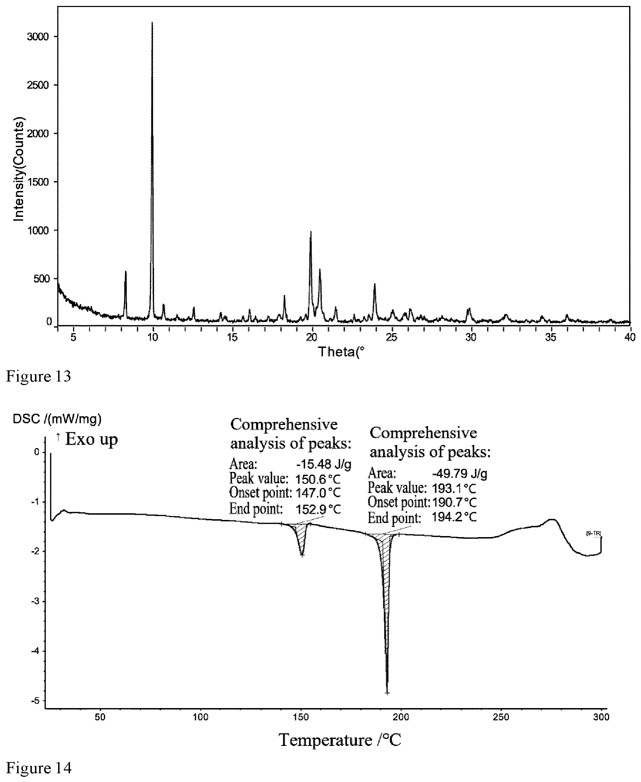

The crystal form A of the isethionic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 13, and has a DSC pattern substantially as shown in FIG. 14.

The crystal form A of the hydrobromic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibits X-ray characteristic diffraction peaks expressed in terms of 2θ angles and interplanar spacing d values using Cu-Kα radiation, as shown in Table 10.

**Table 10**

| Serial No. | XRPD data of crystal form A of hydrobromic acid salt | | | |
|---|---|---|---|---|
| | 2θ (±0.2 °) | d value | Peak height | Proportion (I%) |
| 1 | 17.017 | 5.2061 | 222 | 66.1 |
| 2 | 18.091 | 4.8995 | 84 | 25 |
| 3 | 22.311 | 3.9813 | 329 | 97.9 |
| 4 | 23.401 | 3.7984 | 118 | 35.1 |
| 5 | 25.874 | 3.4406 | 329 | 97.9 |
| 6 | 26.631 | 3.3445 | 99 | 29.5 |
| 7 | 28.597 | 3.1189 | 135 | 40.2 |
| 8 | 28.843 | 3.0929 | 132 | 39.3 |
| 9 | 29.65 | 3.0104 | 310 | 92.3 |
| 10 | 29.972 | 2.9788 | 336 | 100 |
| 11 | 30.333 | 2.9442 | 98 | 29.2 |
| 12 | 30.897 | 2.8918 | 51 | 15.2 |
| 13 | 32.146 | 2.7822 | 119 | 35.4 |
| 14 | 33.303 | 2.6881 | 195 | 58 |
| 15 | 34.393 | 2.6054 | 90 | 26.8 |
| 16 | 35.663 | 2.5155 | 261 | 77.7 |
| 17 | 37.284 | 2.4097 | 55 | 16.4 |
| 18 | 37.744 | 2.3814 | 48 | 14.3 |
| 19 | 38.579 | 2.3318 | 51 | 15.2 |

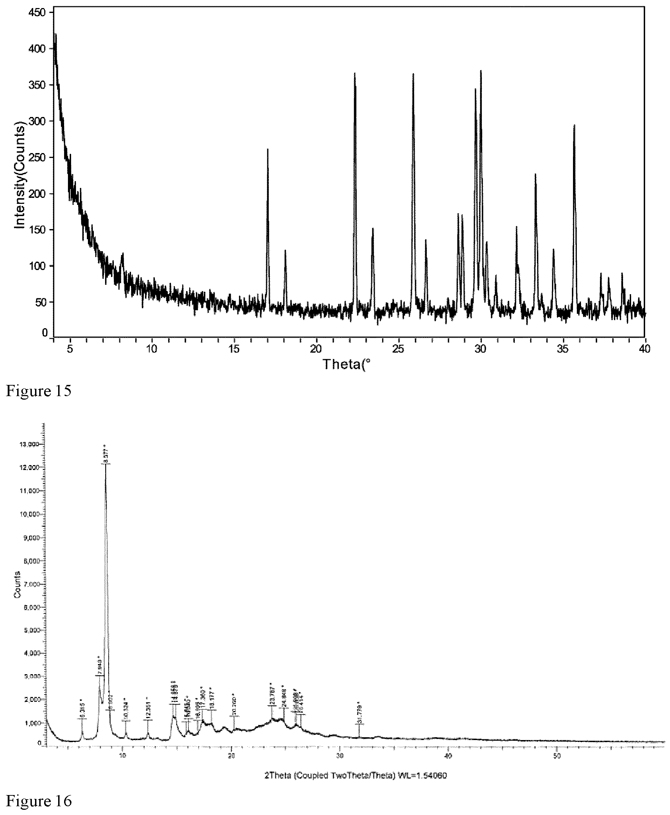

The crystal form A of the hydrobromic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 15.

The crystal form C of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide according to the present invention has an X-ray powder diffraction pattern substantially as shown in FIG. 16.

In a further preferred embodiment of the present invention,
the crystal form A of the hydrochloride salt exhibits an X-ray powder diffraction pattern wherein the 2θ error between the diffraction peaks with the top ten relative peak intensities and the diffraction peaks at the corresponding positions in FIG. 1 is ±0.2° to ±0.5°, preferably ±0.2° to ±0.3°, most preferably ±0.2°;
the crystal form B of the hydrochloride salt exhibits an X-ray powder diffraction pattern wherein the 2θ error between the diffraction peaks with the top ten relative peak intensities and the diffraction peaks at the corresponding positions in FIG. 3 is ±0.2° to ±0.5°, preferably ±0.2° to ±0.3°, most preferably ±0.2°;
the crystal form A of the ethylsulfonic acid salt exhibits an X-ray powder diffraction pattern wherein the 2θ error between the diffraction peaks with the top ten relative peak intensities and the diffraction peaks at the corresponding positions in FIG. 5 is ±0.2° to ±0.5°, preferably ±0.2° to ±0.3°, most preferably ±0.2°;
the crystal form B of the ethylsulfonic acid salt exhibits an X-ray powder diffraction pattern wherein the 2θ error between the diffraction peaks with the top ten relative peak intensities and the diffraction peaks at the corresponding positions in FIG. 6 is ±0.2° to ±0.5°, preferably ±0.2° to ±0.3°, most preferably ±0.2°;
the crystal form A of the p-toluenesulfonic acid salt exhibits an X-ray powder diffraction pattern wherein the 2θ error between the diffraction peaks with the top six relative peak intensities and the diffraction peaks at the corresponding positions in FIG. 7 is ±0.2° to ±0.5°, preferably ±0.2° to ±0.3°, most preferably ±0.2°;
the crystal form A of the methanesulfonic acid salt exhibits an X-ray powder diffraction pattern wherein the 2θ error between the diffraction peaks with the top four relative peak intensities and the diffraction peaks at the corresponding positions in FIG. 8 is ±0.2° to ±0.5°, preferably ±0.2° to ±0.3°, most preferably ±0.2°;
the crystal form A of the sulphuric acid salt exhibits an X-ray powder diffraction pattern wherein the 2θ error between the diffraction peaks with the top ten relative peak intensities and the diffraction peaks at the corresponding positions in FIG. 10 is ±0.2° to ±0.5°, preferably ±0.2° to ±0.3°, most preferably ±0.2°;
the crystal form A of the benzenesulfonic acid salt exhibits an X-ray powder diffraction pattern wherein the 2θ error between the diffraction peaks with the top ten relative peak intensities and the diffraction peaks at the corresponding positions in FIG. 12 is ±0.2° to ±0.5°, preferably ±0.2° to ±0.3°, most preferably ±0.2°;
the crystal form A of the isethionic acid salt exhibits an X-ray powder diffraction pattern wherein the 2θ error between the diffraction peaks with the top seven relative peak intensities and the diffraction peaks at the corresponding positions in FIG. 13 is ±0.2° to ±0.5°, preferably ±0.2° to ±0.3°, most preferably ±0.2°;
the crystal form A of the hydrobromic acid salt exhibits an X-ray powder diffraction pattern wherein the 2θ error between the diffraction peaks with the top five relative peak intensities and the diffraction peaks at the corresponding positions in FIG. 15 is ±0.2° to ±0.5°, preferably ±0.2° to ±0.3°, most preferably ±0.2°;
the crystal form C of the hydrochloride salt exhibits an X-ray powder diffraction pattern wherein the 2θ error between the diffraction peaks with the top five relative peak intensities and the diffraction peaks at the corresponding positions in FIG. 16 is ±0.2° to ±0.5°, preferably ±0.2° to ±0.3°, most preferably ±0.2°.

In a further preferred embodiment of the present invention, the crystal form of the acid salt is a hydrate or an anhydrate, and when the crystal form of the acid salt is a hydrate, the number of water molecules is 0.2 to 3, preferably 0.2, 0.5, 1, 1.5, 2, 2.5 or 3, more preferably 0.5, 1, 2 or 3; further, the water in the hydrate exists as channel water, or crystal water, or a combination thereof.

The present invention provides a method for preparing an acid salt of a compound, comprising the following steps:
1) weighing an appropriate amount of a free base and dissolving the free base in a benign solvent;
2) weighing an appropriate amount of a counter-ion acid and dissolving the counter-ion acid in an organic solvent, wherein the counter-ion acid is preferably used in an amount of 1.0 to 1.5 equivalents;
3) combining the aforementioned two solutions, and stirring the combined solution to allow precipitation, or dropwise adding a poor solvent followed by the stirring to allow precipitation; and
4) rapidly centrifuging the resultant or leaving the resultant to stand for blow drying to obtain a target product,
wherein
the benign solvent is selected from one or more of acetone, tetrahydrofuran, ethyl formate, ethyl acetate, 2-methyl-tetrahydrofuran, 2-butanone, n-butanol, 1,4-dioxane, isobutanol, N,N-dimethylformamide, N,N-dimethylacetamide, n-propanol or tert-butanol; preferably one or more of 2-methyl-tetrahydrofuran, ethyl acetate, 2-butanone, acetone or ethyl formate;
the organic solvent is selected from one or more of methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyl-tetrahydrofuran, 2-butanone, 3-pentanone, heptane, methyl tert-butyl ether, isopropyl ether, 1,4-dioxane, tert-butanol or N,N-dimethylformamide; preferably one or more of methanol, ethanol or acetonitrile; the aforementioned benign solvent and organic solution must be miscible when used;
the poor solvent is selected from one or more of heptane, water, methyl tert-butyl ether, cyclohexane, toluene, isopropyl ether or ethyl acetate; preferably one or more of water, methyl tert-butyl ether or isopropyl ether;
the counter-ion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4- acetamidobenzoic acid, 4-aminobenzoic acid, capric acid, caproic acid, caprylic acid, cinnamic acid, citric acid, cyclohexanesulfamic acid, camphorsulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfate, dibenzoyltartaric acid, ethane-1,2-disulfonic acid, ethylsulfonic acid, isethionic acid, formic acid, fumaric acid, galactosonic acid, gentisic acid, glutaric acid, 2-ketoglutaric acid, glycolic acid, hippuric acid, isethionic acid, lactobionic acid, ascorbic acid, aspartic acid, lauric acid, camphoric acid, maleic acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, butanedioic acid, thiocyanic acid, pamoic acid, formic acid, undecylenic acid, trifluoroacetic acid, p-toluenesulfonic acid or L-malic acid; preferably methanesulfonic acid, ethylsulfonic acid, hydrochloric acid, sulphuric acid, p-toluenesulfonic acid, benzenesulfonic acid, isethionic acid or hydrobromic acid.

The present invention provides a method for preparing an acid salt of a compound, comprising the following steps:
1) weighing an appropriate amount of a free base and suspending the free base with a poor solvent;
2) weighing an appropriate amount of a counter-ion acid and dissolving the counter-ion acid in an organic solvent, wherein the counter-ion acid is preferably used in an amount of 1.0 to 1.5 equivalents;
3) adding the aforementioned solution to the aforementioned suspension and stirring the mixed solution for 2 hours;
4) rapidly centrifuging the resultant or leaving the resultant to stand for blow drying to obtain a salt of a compound as represented by general formula (I),
wherein
the poor solvent is selected from one or more of ethanol, ethyl acetate, ethyl formate, isopropanol, isopropyl acetate, methyl tert-butyl ether, dichloromethane, methanol, acetonitrile, chlorobenzene, benzene, toluene, n-butanol, isobutanol or 3-pentanone; preferably one or more of ethanol, ethyl acetate, isopropanol or isopropyl acetate;
the organic solvent is selected from one or more of methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyl-tetrahydrofuran, 2-butanone, 3-pentanone, heptane, methyl tert-butyl ether, isopropyl ether, 1,4-dioxane, tert-butanol or N,N-dimethylformamide; preferably one or more of methanol, ethanol or acetonitrile; the aforementioned benign solvent and organic solution must be miscible when used;
the counter-ion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4- acetamidobenzoic acid, 4-aminobenzoic acid, capric acid, caproic acid, caprylic acid, cinnamic acid, citric acid, cyclohexanesulfamic acid, camphorsulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfate, dibenzoyltartaric acid, ethane-1,2-disulfonic acid, ethylsulfonic acid, isethionic acid, formic acid, fumaric acid, galactosonic acid, gentisic acid, glutaric acid, 2-ketoglutaric acid, glycolic acid, hippuric acid, isethionic acid, lactobionic acid, ascorbic acid, aspartic acid, lauric acid, camphoric acid, maleic acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, butanedioic acid, thiocyanic acid, pamoic acid, formic acid, undecylenic acid, trifluoroacetic acid, p-toluenesulfonic acid or L-malic acid; preferably methanesulfonic acid, ethylsulfonic acid, hydrochloric acid, sulphuric acid, p-toluenesulfonic acid, benzenesulfonic acid, isethionic acid or hydrobromic acid.

The present invention further relates to a pharmaceutical composition, comprising a therapeutically effective dose of the acid salt of the compound as represented by general formula (I) or a crystal form, and one or more pharmaceutically acceptable carriers, diluents or excipients.

The pharmaceutical composition according to the present invention is suitable for administration routes such as oral administration and injection, and the oral administration route is preferred. Dosage forms include tablets, capsules, dispersions and suspensions, with tablets being preferred.

In certain embodiments of the present invention, in the pharmaceutical composition, the compound as represented by general formula (I) has a weight percentage of 0.1% to 95%, preferably 0.5% to 85%, more preferably 1% to 60%, further preferably 10% to 50%, even further preferably 15% to 40%, yet further preferably 20% to 30%, most preferably 20% to 25%.

In certain embodiments of the present invention, in the pharmaceutical composition, the compound as represented by general formula (I) has a weight percentage of 0.1% to 99%, 0.2% to 98.5%, 0.3% to 98%, 0.4% to 97.5%, 0.5% to 97%, 0.6% to 96.5%, 0.7% to 96%, 0.8% to 95.5%, 0.9% to 95%, 1% to 94.5%, 1.1% to 94%, 1.2% to 93.5%, 1.3% to 93%, 1.4% to 92.5%, 1.5% to 92%, 1.6% to 91.5%, 1.7% to 91%, 1.8% to 90.5%, 1.9% to 90%, 2% to 89.5%, 2.1% to 89%, 2.2% to 88.5%, 2.3% to 88%, 2.4% to 87.5%, 2.5% to 87%, 2.6% to 86.5%, 2.7% to 86%, 2.8% to 85.5%, 2.9% to 85%, 3% to 84.5%, 3.1% to 84%, 3.2% to 83.5%, 3.3% to 83%, 3.4% to 82.5%, 3.5% to 82%, 3.6% to 81.5%, 3.7% to 81%, 3.8% to 80.5%, 3.9% to 80%, 4% to 79.5%, 4.1% to 79%, 4.2% to 78.5%, 4.3% to 78%, 4.4% to 77.5%, 4.5% to 77%, 4.6% to 76.5%, 4.7% to 76%, 4.8% to 75.5%, 4.9% to 75%, 5% to 74.5%, 5.1% to 74%, 5.2% to 73.5%, 5.3% to 73%, 5.4% to 72.5%, 5.5% to 72%, 5.6% to 71.5%, 5.7% to 71%, 5.8% to 70.5%, 5.9% to 70%, 6% to 69.5%, 6.1% to 69%, 6.2% to 68.5%, 6.3% to 68%, 6.4% to 67.5%, 6.5% to 67%, 6.6% to 66.5%, 6.7% to 66%, 6.8% to 65.5%, 6.9% to 65%, 7% to 64.5%, 7.1% to 64%, 7.2% to 63.5%, 7.3% to 63%, 7.4% to 62.5%, 7.5% to 62%, 7.6% to 61.5%, 7.7% to 61%, 7.8% to 60.5%, 7.9% to 60%, 8% to 59.5%, 8.1% to 59%, 8.2% to 58.5%, 8.3% to 58%, 8.4% to 57.5%, 8.5% to 57%, 8.6% to 56.5%, 8.7% to 56%, 8.8% to 55.5%, 8.9% to 55%, 9% to 54.5%, 9.1% to 54%, 9.2% to 53.5%, 9.3% to 53%, 9.4% to 52.5%, 9.5% to 52%, 9.6% to 51.5%, 9.7% to 51%, 9.8% to 50.5%, 9.9% to 50%, 10% to 49.5%, 10.1% to 49%, 10.2% to 48.5%, 10.3% to 48%, 10.4% to 47.5%, 10.5% to 47%, 10.6% to 46.5%, 10.7% to 46%, 10.8% to 45.5%, 10.9% to 45%, 11% to 44.5%, 11.1% to 44%, 11.2% to 43.5%, 11.3% to 43%, 11.4% to 42.5%, 11.5% to 42%, 11.6% to 41.5%, 11.7% to 41%, 11.8% to 40.5%, 11.9% to 40%, 12% to 39.5%, 12.1% to 39%, 12.2% to 38.5%, 12.3% to 38%, 12.4% to 37.5%, 12.5% to 37%, 12.6% to 36.5%, 12.7% to 36%, 12.8% to 35.5%, 12.9% to 35%, 13% to 34.5%, 13.1% to 34%, 13.2% to 33.5%, 13.3% to 33%, 13.4% to 32.5%, 13.5% to 32%, 13.6% to 31.5%, 13.7% to 31%, 13.8% to 30.5%, 13.9% to 30%, 14% to 29.5%, 14.1% to 29%, 14.2% to 28.5%, 14.3% to 28%, 14.4% to 27.5%, 14.5% to 27%, 14.6% to 26.5%, 14.7% to 26%, 14.8% to 25.5%, 14.9% to 25%, 15% to 24.5%, 15.1% to 24%, 15.2% to 23.5%, 15.3% to 23%, 15.4% to 22.5%, 15.5% to 22%, 15.6% to 21.5%, 15.7% to 21%, 15.8% to 20.5%, 15.9% to 20%, 16% to 19.5%, 16.1% to 19%, 16.2% to 18.5%, 16.3% to 18%, 16.4% to 17.5% or 16.5% to 17%, on a free base basis.

In certain embodiments of the present invention, in the pharmaceutical composition, the compound as represented by general formula (I) has a weight percentage of 15% to 30%, 15.1% to 29.9%, 15.2% to 29.8%, 15.3% to 29.7%, 15.4% to 29.6%, 15.5% to 29.5%, 15.6% to 29.4%, 15.7% to 29.3%, 15.8% to 29.2%, 15.9% to 29.1%, 16% to 29%, 16.1% to 28.9%, 16.2% to 28.8%, 16.3% to 28.7%, 16.4% to 28.6%, 16.5% to 28.5%, 16.6% to 28.4%, 16.7% to 28.3%, 16.8% to 28.2%, 16.9% to 28.1%, 17% to 28%, 17.1% to 27.9%, 17.2% to 27.8%, 17.3% to 27.7%, 17.4% to 27.6%, 17.5% to 27.5%, 17.6% to 27.4%, 17.7% to 27.3%, 17.8% to 27.2%, 17.9% to 27.1%, 18% to 27%, 18.1% to 26.9%, 18.2% to 26.8%, 18.3% to 26.7%, 18.4% to 26.6%, 18.5% to 26.5%, 18.6% to 26.4%, 18.7% to 26.3%, 18.8% to 26.2%, 18.9% to 26.1%, 19% to 26%, 19.1% to 25.9%, 19.2% to 25.8%, 19.3% to 25.7%, 19.4% to 25.6%, 19.5% to 25.5%, 19.6% to 25.4%, 19.7% to 25.3%, 19.8% to 25.2%, 19.9% to 25.1%, 20% to 25%, 20.1% to 24.9%, 20.2% to 24.8%, 20.3% to 24.7%, 20.4% to 24.6%, 20.5% to 24.5%, 20.6% to 24.4%, 20.7% to 24.3%, 20.8% to 24.2%, 20.9% to 24.1%, 21% to 24%, 21.1% to 23.9%, 21.2% to 23.8%, 21.3% to 23.7%, 21.4% to 23.6%, 21.5% to 23.5%, 21.6% to 23.4%, 21.7% to 23.3%, 21.8% to 23.2%, 21.9% to 23.1%, 22% to 23%, 22.1% to 22.9%, 22.2% to 22.8%, 22.3% to 22.7% or 22.4% to 22.6%, on a free base basis.

On the other hand, the objective of the present invention is to provide an acid salt or a crystal form of a compound as represented by general formula (I), or a pharmaceutical composition thereof in the preparation of treatment and/or prevention angiotensin II (AT)-dependent.

On the other hand, the objective of the present invention is to provide the use of an acid salt or a crystal form of a compound as represented by general formula (I), or a pharmaceutical composition thereof in the preparation of a drug for treating and/or preventing an endothelin (ET)-dependent disease.

On the other hand, the objective of the present invention is to provide the use of an acid salt or a crystal form of a compound as represented by general formula (I), or a pharmaceutical composition thereof in the preparation of a drug for treating and/or preventing a disease with dual angiotensin-dependent and endothelin (DARA)-dependent mechanisms.

On the other hand, the objective of the present invention is to provide the use of an acid salt or a crystal form of a compound as represented by general formula (I), or a pharmaceutical composition thereof in the preparation of a drug for treating and/or preventing pain, sexual dysfunction, hypoxia and an ischemic disease, dementia, a neurological disease, a liver disease, cancer, hypertension, diabetes, a kidney disease and related diseases.

The present invention further relates to a method for treating and/or preventing pain, sexual dysfunction, hypoxia and an ischemic disease, dementia, a neurological disease, a liver disease, cancer, hypertension, diabetes, a kidney disease and related diseases.

On the other hand, the objective of the present invention is to provide the use of an acid salt or a crystal form of a compound as represented by general formula (I), or a pharmaceutical composition thereof for treatment and/or prevention of pain, sexual dysfunction, hypoxia and an ischemic disease, dementia, a neurological disease, a liver disease, cancer, hypertension, diabetes, a kidney disease and related diseases.

In the above technical solutions, the kidney-related diseases are selected from diseases or conditions related to kidney, glomerular or glomerular mesangial cell function, more preferably focal segmental glomerulosclerosis or IgA nephropathy.

Unless otherwise indicated, reference herein to "the compound as represented by formula (I)" or "the compound of the present invention" also encompasses any stereoisomer of the compound.

Unless otherwise indicated, reference herein to "the compound as represented by formula (I)" or "the compound of the present invention" also encompasses an isotopically labelled compound obtained by replacing any atom in the compound with an isotopic atom thereof. The present invention includes all pharmaceutically acceptable isotopically labelled compounds of the compound as represented by formula (I), wherein one or more atoms are replaced by atoms having the same atomic number but different atomic masses or mass numbers as those typically found in nature.

Examples of suitable isotopes for inclusion in the compound of the present invention include isotopes of hydrogen, such as 2H (D) and 3H (T), isotopes of carbon, such as 11C, 13C and 14C, isotopes of chlorine, such as 36Cl, isotopes of fluorine, such as 18F, isotopes of iodine, such as 123I and 125I, isotopes of nitrogen, such as 13N and 15N, isotopes of oxygen, such as 150, 170 and 180, and isotopes of sulphur, such as 35S.

The isotopically labelled compound as represented by formula (I) can generally be prepared by conventional techniques known to those skilled in the art or by using an appropriate isotopically labelled reagent in place of the non-labelled reagent previously used in an analogous manner to those described in the Examples and Preparations appended herein.

### Brief Description of the Drawings

The accompanying drawings are used for providing a further understanding of the present invention and constitute a part of this specification, and are used, together with the following specific embodiments, for explaining the present invention but do not limit the present invention.
FIG. 1 is an XRPD pattern of crystal form A of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 2 is a DSC pattern of crystal form A of the hydrochloride salt of the compound (4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 3 is an XRPD pattern of crystal form B of the hydrochloride salt of the compound (4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 4 is a DSC pattern of crystal form B of the hydrochloride salt of the compound (4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 5 is an XRPD pattern of crystal form A of the ethylsulfonic acid salt of the compound (4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 6 is an XRPD pattern of crystal form B of the ethylsulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 7 is an XRPD pattern of crystal form A of the p-toluenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 8 is an XRPD pattern of crystal form A of the methanesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 9 is a DSC pattern of crystal form A of the methanesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 10 is an XRPD pattern of crystal form A of the sulphuric acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 11 is a DSC pattern of crystal form A of the sulphuric acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 12 is an XRPD pattern of crystal form A of the benzenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 13 is an XRPD pattern of crystal form A of the isethionic acid salt of 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 14 is a DSC pattern of crystal form A of the isethionic acid salt of 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 15 is an XRPD pattern of crystal form A of the hydrobromic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.
FIG. 16 is an XRPD pattern of crystal form C of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

### Detailed Description of the Invention

Unless stated to the contrary, the terms used in the description and claims have the following meanings.

The term "alkyl" refers to a saturated aliphatic hydrocarbon group, which is a linear or branched chain group containing 1 to 20 carbon atoms, preferably alkyl containing 1 to 12 carbon atoms, more preferably alkyl containing 1 to 8 carbon atoms, further preferably alkyl containing 1 to 6 carbon atoms, most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 4-heptyl, 1-propylbutyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, their respective branched chain isomers, etc. Lower alkyl groups containing 1 to 6 carbon atoms are more preferred, and non-limiting examples thereof include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, 4-heptyl, 1-propylbutyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted. When the alkyl is substituted, the substituent can be substituted at any available connection point, and the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group. In the present invention, alkyl is preferably methyl, ethyl, isopropyl, tert-butyl, haloalkyl, deuteroalkyl, alkoxy-substituted alkyl, and hydroxyl-substituted alkyl.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spiro, fused and bridged cycloalkyl, preferably cyclopropyl, cyclobutyl, cyclohexyl, cyclopentyl, and cycloheptyl.

The cycloalkyl ring can be fused to an aryl, heteroaryl or heterocycloalkyl ring, wherein the ring connected to the parent structure is cycloalkyl, and non-limiting examples include indanyl, tetrahydronaphthyl, benzocycloheptanyl, etc. Cycloalkyl can be optionally substituted or unsubstituted. When the cycloalkyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "heterocyclyl" refers to a saturated or partially unsaturated monocyclic or polycyclic hydrocarbon substituent, which comprises 3 to 20 ring atoms, wherein one or more ring atoms are heteroatoms selected from nitrogen, oxygen, C(O) or S(O)ₘ (wherein m is an integer of 0 to 2), but excluding ring moieties of -O-O-, -O-S- or -S-S-, and the remaining ring atoms are carbon. Preferably, the heterocyclyl comprises 3 to 12 ring atoms, among which 1-4 are heteroatoms; more preferably, the heterocyclyl comprises 3 to 8 ring atoms; most preferably, the heterocyclyl comprises 3 to 8 ring atoms; further preferably, the heterocyclyl is a 3- to -8-membered heterocyclyl group comprising 1 to 3 nitrogen atoms, which is optionally substituted with 1 to 2 oxygen atoms, sulphur atoms, or oxo, including nitrogen-containing monocyclic heterocyclyl, nitrogen-containing spiroheterocyclyl, or nitrogen-containing fused heterocyclyl.

Non-limiting examples of monocyclic heterocyclyl include oxetanyl, azetidinyl, thietanyl, pyrrolidinyl, imidazolidinyl, tetrahydrofuryl, tetrahydrothienyl, tetrahydropyranyl, dihydroimidazolyl, dihydrofuryl, dihydropyrazolyl, dihydropyrrolyl, piperidinyl, piperazinyl, morpholinyl, thiomorpholinyl, homopiperazinyl, azepanyl, 1,4-diazacycloheptyl, pyranyl, tetrahydrothiopyranyl dioxide group, *etc.;* preferably oxetanyl, azetidinyl, thietanyl, tetrahydrofuryl, tetrahydropyranyl, tetrahydrothienyl, tetrahydrothiopyranyl, tetrahydrothiopyranyl dioxide group, pyrrolidinyl, morpholinyl, piperidinyl, piperazinyl, hexahydropyrazinyl, hexahydropyrimidinyl, azepanyl, 1,4-diazacycloheptyl and piperazinyl; more preferably piperidinyl, piperazinyl, pyrrolidinyl, morpholinyl, azetidinyl, dihydrotetrazolyl, pyrimidin-4(3H)-one, 1,2,4-oxadiazol-5(2H)-one or 5,6-dihydro-4H-cyclopenta[d]isoxazole. Polycyclic heterocyclyl includes spiro, fused and bridged heterocyclyl, wherein the spiro, fused and bridged heterocyclyl are optionally connected to other groups via a single bond, or further fused to other cycloalkyl, heterocyclyl, aryl and heteroaryl via any two or more atoms on the ring.

Heterocyclyl can be optionally substituted or unsubstituted. When the heterocyclyl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or a carboxylate group.

The term "aryl" refers to a 6- to 14-membered all-carbon monocyclic or fused polycyclic (i.e., rings sharing an adjacent pair of carbon atoms) group having a conjugated π electron system, preferably 6- to 12-membered aryl, such as phenyl and naphthyl, more preferably phenyl.

Aryl can be substituted or unsubstituted. When the aryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "heteroaryl" refers to a heteroaromatic system containing 1 to 4 heteroatoms and 5 to 14 ring atoms, wherein the heteroatoms are selected from oxygen, sulphur, and nitrogen. The heteroaryl is preferably 5- to 12-membered, more preferably 5-membered or 6-membered, such as imidazolyl, furyl, thienyl, thiazolyl, pyrazolyl, oxazolyl, pyrrolyl, triazolyl, tetrazolyl , pyridyl, pyrimidinyl, thiadiazolyl, pyrazinyl, *etc.,* preferably pyridyl, pyrazinyl, oxadiazolyl, triazolyl, tetrazolyl, thienyl, imidazolyl, pyrazolyl, oxazolyl, thiazolyl, pyrimidinyl or thiazolyl; more preferably pyridyl, oxadiazolyl, pyrazolyl, pyrazinyl, isoxazolyl, triazolyl, tetrazolyl, pyrrolyl, thiazolyl and oxazolyl.

Heteroaryl can be optionally substituted or unsubstituted. When the heteroaryl is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

The term "alkoxy" refers to -O-(alkyl) and -O-(unsubstituted cycloalkyl), wherein the alkyl is as defined above. Non-limiting examples of alkoxy include: methoxy, ethoxy, propoxy, butoxy, cyclopropoxy, cyclobutoxy, cyclopentyloxy or cyclohexyloxy; Alkoxy can be optionally substituted or unsubstituted. When the alkoxy is substituted, the substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl, or a carboxylate group.

"Haloalkyl" refers to alkyl substituted with one or more halogen, wherein the alkyl is defined as above.

"Haloalkoxy" refers to alkoxy substituted with one or more halogen, wherein the alkoxy is defined as above.

"Hydroxyalkyl" refers to alkyl substituted with one or more hydroxyl, where the alkyl is defined as above.

"Hydroxyl" refers to an -OH group.

"Halogen" refers to fluorine, chlorine, bromine, or iodine.

"Amino" refers to -NH₂.

"Cyano" refers to -CN.

"DMF" refers to N,N-dimethylformamide.

"TFA" refers to trifluoroacetic acid.

"MeCN" refers to acetonitrile.

"DCM" refers to dichloromethane.

Different expressions such as "X is selected from A, B, or C", "X is selected from A, B and C", "X is A, B or C", and "X is A, B and C" all express the same meaning, that is, X can be any one or more of A, B, and C.

The hydrogen atoms according to the present invention can be replaced with the isotope deuterium thereof, and any hydrogen atom in the example compounds involved in the present invention can also be replaced with a deuterium atom.

"Optional" or "optionally" means that the event or circumstance subsequently described may but not necessarily occur, and the description includes the occasion where the event or circumstance occurs or does not occur. For example, "heterocyclyl optionally substituted with alkyl" means that the alkyl may but not necessarily be present, and the description includes the case where the heterocyclyl is substituted with alkyl and the case where the heterocyclyl is not substituted with alkyl.

"Substituted" means that one or more hydrogen atoms, preferably at most 5, more preferably 1-3 hydrogen atoms in the group are each independently substituted with a corresponding number of substituents. It goes without saying, the substituents may be only in possible chemical positions thereof, and those skilled in the art can determine the possible or impossible substitutions (by experiment or theory) without paying too much effort. For example, an amino or hydroxyl group having a free hydrogen may be unstable when combined with the carbon atoms having an unsaturated (e.g., olefinic) bond.

"Pharmaceutical composition" denotes a mixture containing one or more compounds described herein or physiologically/pharmaceutically acceptable salts or prodrugs thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

"Pharmaceutically acceptable salts" refer to salts of the compounds of the present invention, which are safe and effective when used in mammals, and have appropriate biological activity.

X-ray powder diffraction pattern (XRPD) refers to the experimentally observed diffraction pattern or the parameters derived therefrom. The X-ray powder diffraction pattern is characterized by the peak position (abscissa) and the peak intensity (ordinate). Those skilled in the art will appreciate that the experimental error depends on the conditions of the instrument, the preparation of the sample and the purity of the sample. In particular, it is well known to those skilled in the art that the X-ray diffraction pattern often changes with the conditions of the instrument, and those skilled in the art should understand that the appropriate error tolerance of XRPD can be: 2θ±0.5°, 2θ±0.4°, 2θ±0.3° or 2θ±0.2°. In particular, it should be noted that the relative intensity of the X-ray diffraction pattern may also change with experimental conditions, and thus the order of peak intensity cannot be used as the only or decisive factor. In addition, due to the influence of experimental factors such as sample height, peak angles may shift as a whole, and a certain shift is usually allowed. Therefore, those skilled in the art will appreciate that any crystal form having the characteristic peak identical or similar to those of the pattern of the present invention falls within the scope of the present invention.

"DSC" refers to differential scanning calorimetry (DSC) experiment.

### Detailed Description of Embodiments

The present invention will be further described below in conjunction with examples, but these examples are not meant to limit the scope of the present invention.

### Example

The structure of the compound of the present invention is determined by nuclear magnetic resonance (NMR) or/and liquid chromatography-mass spectrometry (LC-MS). NMR chemical shift (δ) is given in parts per million (ppm). NMR is determined with Bruker AVANCE-400 nuclear magnetic resonance instrument; the solvents for determination are deuterated dimethyl sulfoxide (DMSO-*d₆*), deuterated methanol (CD₃OD) and deuterated chloroform (CDCl₃); and the internal standard is tetramethylsilane (TMS).

Agilent 1200 Infinity Series mass spectrometer is used for measurement by liquid chromatography-mass chromatography (LC-MS). For determination by HPLC, Agilent 1200DAD high-pressure liquid chromatograph (Sunfire C18 150 × 4.6 mm chromatographic column) and Waters 2695-2996 high-pressure liquid chromatograph (Gimini C₁₈ 150 × 4.6 mm chromatographic column) are used.

Yantai Huanghai HSGF254 or Qingdao GF254 silica gel plate is used as a thin layer chromatography silica plate, and TLC is of the specification of 0.15 to 0.20 mm, and the specification when separating and purifying a product by thin layer chromatography is 0.4 to 0.5 mm. For column chromatography, Yantai Huanghai silica gel of 200-300 mesh is generally used as a carrier.

The starting materials in the examples of the present invention are known and can be purchased on the market, or can be synthesized using or according to methods known in the art.

Unless otherwise specified, all reactions in the present invention are carried out under continuous magnetic stirring in a dry nitrogen or argon atmosphere, the solvent is a dry solvent, and the reaction temperature unit is degrees Celsius.

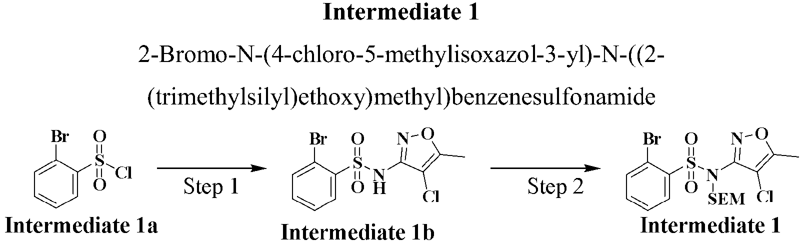

### Step 1

### Preparation of 2-bromo-N-(4-chloro-5-methylisoxazol-3-yl)benzenesulfonamide

4-Chloro-5-methylisoxazole-3-amine (5.0 g, 37.8 mmol) was dissolved in tetrahydrofuran (50 mL), the reaction solution was cooled to -78°C, and then potassium tert-butoxide (8.43 g, 75.3 mmol) was added to the reaction solution, and the reaction solution was stirred and reacted at -78°C for 0.5 h. Intermediate **1a** (10.0 g, 39.4 mmol) was added to the reaction solution, and the reaction solution was stirred at room temperature for 1 h. Water and dichloromethane (3 x 20 mL) were added for extraction. The organic layers were combined, dried over anhydrous sodium sulfate, filtered, and concentrated to afford the target product intermediate **1b** (12.5 g, yield: 88.5%).

MS m/z (ESI): 351.2 [M+1]⁺.

### Step 2

### Preparation of 2-bromo-N-(4-chloro-5-methylisoxazol-3-yl)-N-((2-(trimethylsilyl)ethoxy)methyl)benzenesulfonamide

Intermediate **1b** (12.5 g, 35.7 mmol) and potassium carbonate (9.8 g, 71.4 mmol) were dissolved in N,N-dimethylformamide (20 mL), and then 2-(trimethylsilyl)ethyloxymethyl chloride (8.9 g, 53.6 mmol) was added to the reaction solution, and the reaction was stirred at room temperature for 16 h. Water and dichloromethane (3 x 20 mL) were added for extraction. The organic layers were combined, dried over anhydrous sodium sulfate, filtered and concentrated, and the residue was purified by column chromatography (petroleum ether/ethyl acetate system) to afford the target product intermediate **1** (17.2 g, yield: 98.5%).

### Example 1

### 4'-((2-Butyl-4-oxo-1,3-diazospiro[4.4]non-1-en-3-yl)methyl-d2)-N-(4,5-dimethylisoxazol-3-yl)- 2'-(ethoxymethyl)-[1,1'-biphenyl] -2-sulfonamide

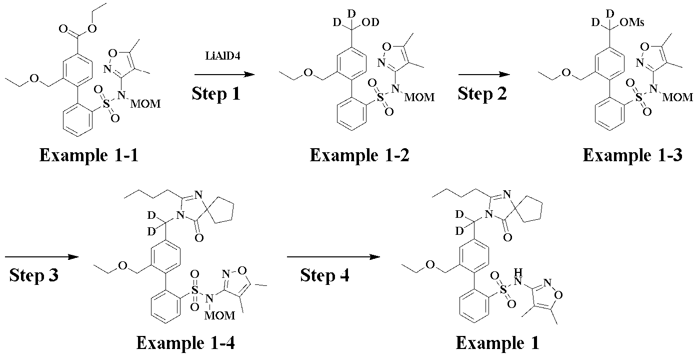

### Step 1

### Preparation of N-(4,5-dimethylisoxazol-3-yl)-2'-(ethoxymethyl)-4'-((hydroxy-d)-methyl-d2)-N-(methoxymethyl)-[1,1'-biphenyl]- 2-sulfonamide

Example **1-1** (80 mg, 0.17 mmol) (referring to WO 2010114801A1 for the preparation method) and deuterated lithium aluminium hydride (11 mg, 0.26 mmol) were dissolved in tetrahydrofuran (5 mL), and the reaction solution was cooled to 0°C, stirred and reacted for 2 h. Saturated sodium chloride aqueous solution (10 mL) was added to the reaction solution, and the mixture was extracted with ethyl acetate (2 x 10 mL). The organic phases were combined, dried over anhydrous sodium sulfate and concentrated, and the residue was purified by silica gel column chromatography (petroleum ether/ethyl acetate system) to afford Example **1-2** (50 mg, 70%).

MS m/z (ESI): 464.2 [M+1] ⁺.

### Step 2

### Preparation of (2'-(N-(4,5-dimethylisoxazol-3-yl)-N-(methoxymethyl)sulfamoyl)-2-(ethoxymethyl)-[1,1'-biphenyl]-4-yl)methyl-d2 methanesulfonic acid

Under ice bath conditions, methylsulfonyl chloride (14.8 mg, 0.13 mmol) and diisopropylethylamine (41.8 mg, 0.32 mmol) were added to a solution of Example **1-2** (50 mg, 0.11 mmol) in dichloromethane (4 mL), and the reaction solution was warmed to room temperature and stirred for 1 h. The reaction solution was concentrated to afford crude Example **1-3** (60 mg, 98%), which was directly used in the next reaction.

MS m/z (ESI): 541.2 [M+1] ⁺.

### Step 3

### Preparation of 4'-((2-butyl-4-oxo-1,3-diazaspiro[4.4]non-1-en-3-yl)methyl-d2)-N-(4,5-dimethylisoxazol-3-yl)- 2'-(ethoxymethyl)-N-(methoxymethyl)-[1,1'-biphenyl] -2-sulfonamide

Example **1-3** (60 mg, 0.11 mmol) was dissolved in DMF (4 mL), potassium carbonate (30.7 mg, 0.24 mmol) and 2-butyl-1,3-diazaspiro-[4,4]non-1-en-4-one (25.8 mg, 0.13 mmol) were added under ice bath conditions, and the reaction solution was stirred at room temperature for 2 h. The reaction solution was concentrated, and the crude product was purified by HPLC to afford Example **1-4** (42 mg, 72%).

MS m/z (ESI): 639.3 [M+1] ⁺.

### Step 4

### Preparation of 4'-((2-butyl-4-oxo-1,3-diazaspiro[4.4]non-1-en-3-yl)methyl-d2)-N-(4,5-dimethylisoxazol-3-yl)- 2'-(ethoxymethyl)-[1,1'-biphenyl] -2-sulfonamide

Example **1-4** (42 mg, 0.07 mmol) was dissolved in ethanol (2 mL), and 6N hydrochloric acid was added. The mixture was heated to reflux for 1 h, adjusted to pH 8 with sodium carbonate, and then adjusted to pH 5, and extracted with ethyl acetate (2 x 10 mL). The organic phases were combined, dried over anhydrous sodium sulfate and concentrated, and the residue was purified by reverse HPLC to afford Example **1** (10 mg, 26%).

MS m/z (ESI): 595.3 [M+1] ⁺.

### Example 2

### 4'-((2-Butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide

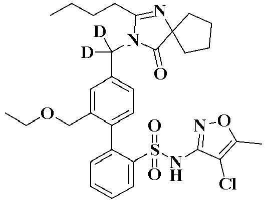

### Method I:

Synthesis method of Example **2** referred to the synthesis method of Example **1,** and 4-chloro-5-methylisoxazoleamine was used in place of 4,5-dimethylisoxazoleamine to afford Example **2** (51 mg, yield: 50.3%).

### Method II:

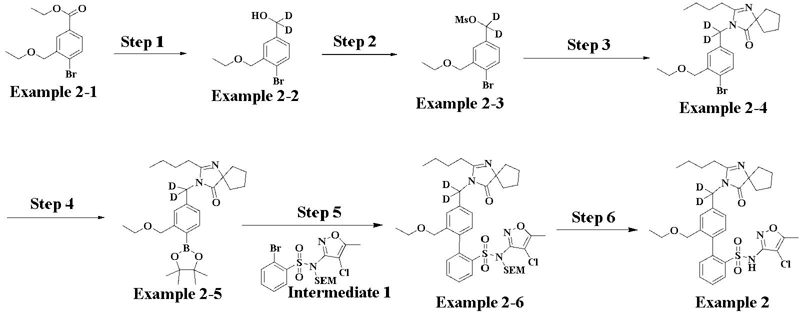

### Step 1

### Preparation of (4-bromo-3-(ethoxymethyl)phenyl)-d2methanol

Example **2-1** (1.0 g, 3.48 mmol) (referring to WO 2010114801A1 for the preparation method) and deuterated lithium aluminium hydride (219.3 mg, 5.22 mmol) were dissolved in tetrahydrofuran (20 mL), and the reaction solution was cooled to 0°C, stirred and reacted for 2 h. Saturated sodium chloride aqueous solution (50 mL) was added to the reaction solution, and the mixture was extracted with ethyl acetate (2 x 100 mL). The organic phases were combined, dried over anhydrous sodium sulfate and concentrated, and the residue was purified by silica gel column chromatography (petroleum ether/ethyl acetate system) to afford Example **2-2** (850 mg, 98%).

MS m/z (ESI): 248.1 [M+1] ⁺.

### Step 2

### Preparation of 4-bromo-3-(ethoxymethyl)benzyl-d2methanesulfonate

Under ice bath conditions, methylsulfonyl chloride (433.4 mg, 3.78 mmol) and diisopropylethylamine (1.33 g, 10.32 mmol) were added to a solution of Example **2-2** (50 mg, 3.44 mmol) in dichloromethane (20 mL), and the reaction solution was warmed to room temperature and stirred for 1 h. The reaction solution was concentrated to afford crude Example **2-3** (1.1 g, 98%), which was directly used in the next reaction.

MS m/z (ESI): 326.2 [M+1] ⁺.

### Step 3

### Preparation of 3-(4-bromo-3-(ethoxymethyl)phenyl)methyl-d2)-2-butyl-1,3-diazaaspirin[4.4]non-1-en-4-one

Example **2-3** (1.1 g, 3.38 mmol) was dissolved in DMF (15 mL), potassium carbonate (1.03 g, 7.44 mmol) and 2-butyl-1,3-diazaspiro-[4,4]non-1-en-4-one (858.4 mg, 3.72 mmol) were added under ice bath conditions, and the reaction solution was stirred at room temperature for 2 h. The reaction solution was concentrated, and the crude product was purified by HPLC to afford Example **2-4** (1.2 g, 86%).

MS m/z (ESI): 424.4 [M+1] ⁺.

### Step 4

### Preparation of 2-butyl-3-((3-(ethoxymethyl)-4-(4,4,5,5-tetramethyl-1,3,2-dioxaborolan-2-yl)phenyl)methyl-d2)-1,3-diazaspiro[4.4]non-1-en-4-one

Compound **2-4** (1.2 g, 2.86 mmol) was dissolved in 15 mL of 1,4-dioxane, and bis(pinacolato)diboron (0.87 g, 3.4 mmol), 1,1 '-bis(diphenylphosphino)ferrocene palladium(II) dichloride (102.9 mg, 0.14 mmol) and potassium acetate (0.84 g, 8.56 mmol) were added. The mixture was heated to 80°C under nitrogen protection, stirred and reacted for 3 h. The reaction solution was cooled to room temperature, saturated sodium chloride solution (50 mL) was added, and the mixture was extracted with ethyl acetate (100 mL × 3). The organic phases were combined, dried over anhydrous sodium sulfate, filtered and concentrated. The residue was purified by silica gel chromatography with an eluent system (ethyl acetate/petroleum ether = 10% to 50%) to afford Example **2-5** (1.0 g, 80.0%).

MS m/z (ESI): 471.5 [M+1] ⁺.

### Step 5

### Preparation of 4'-(2-butyl-4-oxo-1,3-diazaspiro[4.4]non-1-en-3-ylmethyl-d2)-n-(4-chloro-5-methylisoxazol-3-yl)-2'-(ethoxymethyl)-n-(2-trimethylsilylethoxy)methyl)-[1,1'-biphenyl]-2-sulfonamide

Example **2-5** (0.5 g, 1.07 mmol) was dissolved in 20 mL of 1,4-dioxane and water (2 ml), and intermediate **1** (0.4 g, 1.07 mmol), 1,1'-bis(diphenylphosphino)ferrocene palladium(II) dichloride (0.039 g, 0.053 mmol) and potassium carbonate (0.3 g, 3.2 mmol) were added. The mixture was heated to 90°C under nitrogen protection, stirred and reacted for 16 h. The reaction solution was cooled to room temperature, saturated sodium chloride solution (50 mL) was added, and the mixture was extracted with ethyl acetate (100 mL × 3). The organic phases were combined, dried over anhydrous sodium sulfate, filtered and concentrated. The residue was purified by silica gel chromatography with an eluent system (ethyl acetate/petroleum ether = 10% to 50%) to afford Example **2-6** (0.45 g, yield: 66.0%).

MS m/z (ESI): 746.4[M+1] ⁺.

### Step 6

### Preparation of 4'-(2-butyl-4-oxo-1,3-diazaspiro[4.4]non-1-en-3-ylmethyl-d2)-n-(4-chloro-5-methylisoxazol-3-yl)-2'-(ethoxymethyl)-[1,1'-biphenyl]-2-sulfonamide

Example **2-6** (0.45 g, 0.7 mmol) was dissolved in 10 mL of 4M HCl/dioxane. The mixture was heated to 70°C, stirred and reacted for 2 h. The reaction solution was cooled to room temperature, saturated sodium chloride solution (50 mL) was added, and the mixture was extracted with ethyl acetate (100 mL × 3). The organic phases were combined, dried over anhydrous sodium sulfate, filtered and concentrated. The residue was purified by silica gel chromatography with an eluent system (i.e., p-HPLC (FA)) to afford Example 2 (0.2 g, 50.0%).

MS m/z (ESI): 615.2 [M+1] ⁺.

¹H NMR (400 MHz, DMSO-d6) δ 8.05 - 7.97 (m, 1H), 7.54 (s, 2H), 7.22 - 7.12 (m, 2H), 6.99 (s, 2H), 4.08 (d, J = 13.1 Hz, 1H), 3.99 (d, J = 13.1 Hz, 1H), 3.21 (ddd, J = 9.4, 7.0, 3.6 Hz, 2H), 2.35 (t, J = 7.5 Hz, 2H), 2.25 (s, 3H), 1.85 (d, J = 8.5 Hz, 6H), 1.69 (d, J = 8.8 Hz, 2H), 1.50 (q, J = 7.7 Hz, 2H), 1.28 (d, J = 7.6 Hz, 2H), 1.01 (t, J = 6.9 Hz, 3H), 0.82 (t, J = 7.3 Hz, 3H).

### Example 3

### 4'-((2-Butyl-4-oxo-1,3-diazaspiro[4.4]non-1-en-3-yl)methyl-d2)-N-(4-fluoro-5-methylisoxazol-3-yl)-2'-(ethoxymethyl)-[1,1'-biphenyl]-2-sulfonamide

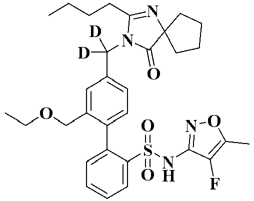

Synthesis method of Example **3** referred to the synthesis method of Example **1,** and 4-fluoro-5-methylisoxazoleamine was used in place of 4,5-dimethylisoxazoleamine to afford Example **3** (11 mg, a solid, yield: 40.3%).

MS m/z (ESI): 599.3 [M+1] ⁺.

¹H NMR (400 MHz, DMSO-d6) δ 8.05 - 7.97 (m, 1H), 7.54 (s, 2H), 7.22 - 7.12 (m, 2H), 6.99 (s, 2H), 4.08 (d, J = 13.1 Hz, 1H), 3.99 (d, J = 13.1 Hz, 1H), 3.21 (ddd, J = 9.4, 7.0, 3.6 Hz, 2H), 2.35 (t, J = 7.5 Hz, 2H), 2.25 (s, 3H), 1.85 (d, J = 8.5 Hz, 6H), 1.69 (d, J = 8.8 Hz, 2H), 1.50 (q, J = 7.7 Hz, 2H), 1.28 (d, J = 7.6 Hz, 2H), 1.01 (t, J = 6.9 Hz, 3H), 0.82 (t, J = 7.3 Hz, 3H).

### Example 4

### 4'-((2-Butyl-4-oxo-1,3-diazospiro[4.4]non-1-en-3-yl)methyl)-N-(4-chloro-5-methylisoxazol-3-yl)-2'-(ethoxydeuteromethyl)-[1,1'-biphenyl]-2-sulfonamide

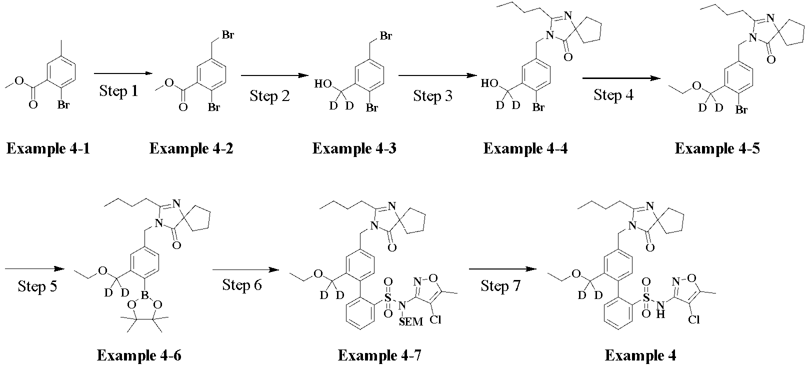

### Step 1

### Preparation of methyl 2-bromo-5-(bromomethyl)benzoate

N-Bromosuccinimide (854.68 mg, 4.80 mmol) and Example **4-1** (1.0 g, 4.37 mmol) were dissolved in carbon tetrachloride (5 mL), and then benzoyl peroxide (105.74 mg, 436.55 µmol) was added to the reaction solution, and the reaction solution was stirred and reacted at 80°C for 16 h. Saturated sodium chloride solution (10 mL) was added to the reaction solution, and the mixture was extracted with dichloromethane (10 mL x 3). The organic phases were combined, dried, and concentrated to afford a crude product. The crude product was purified by column chromatography (petroleum ether/ethyl acetate system) to afford the target molecule Example **4-2** (1.1 g, 3.57 mmol, yield: 81.82%).

### Step 2

Preparation of (2-bromo-5-(bromomethyl)phenyl)deuteromethan-ol Deuterated lithium aluminium hydride (104.72 mg, 2.44 mmol) was dissolved in tetrahydrofuran (3 mL), and then Example **4-2** (500 mg, 1.62 mmol) was added, and the reaction solution was stirred and reacted at room temperature for 1 h. Water (0.1 mL), 15% sodium hydroxide solution (0.1 mL) and water (0.3 mL) were added to the reaction solution in sequence, and the mixture was stirred for 0.5 h and then filtered. The filter cake was washed with dichloromethane (10 mL x 3), and the filtrate was dried and concentrated to afford the target molecule Example **4-3** (310 mg, 1.10 mmol, yield: 67.72%).

### Step 3

### Preparation of 3-(4-bromo-3-(hydroxymethyl2)benzyl)-2-butyl-1,3-diazaspiro[4.4]non-1-en-4-one

2-Butyl-1,3-diazaspiro[4.4]non-1-en-4-one (62.01 mg, 319.18 µmol) and Example **4-3** were dissolved in acetonitrile (2 mL), and then potassium carbonate (29.36 mg, 212.79 µmol) was added, and the reaction solution was stirred and reacted at 80°C for 3 h. After the reaction was completed, saturated sodium chloride solution (10 mL) was added, and the mixture was extracted with dichloromethane (10 mL x 3). The organic phases were combined, dried, and concentrated to afford a crude product. The crude product was purified by column chromatography (petroleum ether/ethyl acetate system) to afford the target molecule Example **4-4** (18 mg, 45.53 µmol, yield: 21.40%).

MS m/z (ESI): 395.2[M+1] ⁺.

### Step 4

### Preparation of 3-(4-bromo-3-(ethoxymethylD2)benzyl)-2-butyl-1,3-diazaspiro[4.4]non-1-en-4-one

Example **4-4** (500 mg, 1.26 mmol) was dissolved in tetrahydrofuran (2 mL), and then sodium hydride (151.76 mg, 3.79 mmol, 60% purity) was added, and the reaction solution was stirred and reacted at room temperature for 0.5 h. Ethyl iodide (986.30 mg, 6.32 mmol) was added, and the reaction solution was stirred and reacted at room temperature for 1.5 h. After the reaction was completed, saturated sodium chloride solution (10 mL) was added, and the mixture was extracted with dichloromethane (10 mL x 3). The organic phases were combined, dried, and concentrated to afford a crude product. The crude product was purified by column chromatography (petroleum ether/ethyl acetate system) to afford the target molecule Example **4-5** (210 mg, 496.01 µmol, yield: 39.22%).

MS m/z (ESI): 423.2[M+1] ⁺.

### Step 5

### Preparation of 2-butyl-3-(3-(ethoxymethyl-d2)-4-(4,4,5,5-tetramethyl-1,3,2-dioxaborolan-2-yl)benzyl)-1,3-diazaspiro[4.4 ]non-1-en-4-one

Example **4-5** (100 mg, 236.19 µmol), bis(pinacolato)diboron (71.97 mg, 283.43 µmol), [1,1'-bis(diphenylphosphino)ferrocene]palladium(II) dichloride dichloromethane complex (19.27 mg, 23.62 µmol) and potassium acetate (45.35 mg, 472.39 µmol) were dissolved in dioxane (5 mL), and the reaction solution was stirred and reacted at 90°C for 16 h. Saturated sodium chloride aqueous solution (10 mL) was added, and the mixture was extracted with dichloromethane (10 mL x 3). The organic phases were combined, dried and concentrated to afford the target molecule crude Example **4-6** (105 mg, 223.19 µmol, yield: 94.50%). The crude product was directly used in the next step without purification.

MS m/z (ESI): 471.2[M+1] ⁺.

### Step 6

### Preparation of 4'-((2-butyl-4-oxo-1,3-diazaspiro[4.4]non-1-en-3-yl)methyl)-N-(4-chloro-5-methylisoxazol-3-yl)- 2'-(ethoxymethyl-d2)-N-(((2-(trimethylsilyl)ethoxy)methyl)-[1,1'-biphenyl] -2-sulfonamide

Intermediate 1 (93 mg, 235.05 µmol), Example **4-6** (110.58 mg, 235.05 µmol), [1,1'-bis(diphenylphosphino)ferrocene]palladium(II) dichloride dichloromethane complex (21.53 mg, 23.51 µmol) and caesium carbonate (229.88 mg, 705.16 µmol) were dissolved in dioxane and water (2.5 mL, 4 : 1), and the reaction solution was stirred and reacted at 100°C for 1 h. Saturated sodium chloride aqueous solution (10 mL) was added, and the mixture was extracted with dichloromethane (10 mL x 3). The organic phases were combined, dried, and concentrated to afford a crude product. The crude product was purified by column chromatography (petroleum ether/ethyl acetate system) to afford the target molecule Example 4-7 (106 mg, 160.79 µmol, yield: 68.41%).

MS m/z (ESI): 745.3[M+1] ⁺.

### Step 7

### 4'-((2-butyl-4-oxo-1,3-diazospiro[4.4]non-1-en-3-yl)methyl)-N-(4-chloro-5-methylisoxazol-3-yl)- 2'-(ethoxydeuteromethyl)-[1,1'-biphenyl] -2-sulfonamide

Synthesis method of Example **4** referred to the synthesis method of Example **2,** and Example **4-7** was used as raw material to afford the title compound Example **4** (32 mg, 33.5%).

MS m/z (ESI): 615.2[M+1] ⁺.

¹H NMR (400 MHz, DMSO) δ 8.01 (dd, *J =* 16.0, 14.4 Hz, 1H), 7.60 (t, *J* = 29.2 Hz, 2H), 7.15 (d, *J =* 11.1 Hz, 2H), 7.00 (s, 2H), 4.83 - 4.66 (m, 2H), 3.28 - 3.13 (m, 2H), 2.36 (t, *J =* 7.5 Hz, 2H), 2.28 (s, 3H), 1.86 (d, *J =* 6.4 Hz, 6H), 1.71 (d, *J* = 8.0 Hz, 2H), 1.52 (dt, *J* = 15.2, 7.5 Hz, 2H), 1.34 - 1.25 (m, 2H), 1.01 (t, *J* = 7.0 Hz, 3H), 0.88 - 0.75 (m, 3H).

### Example 5

### 4'-((2-Butyl-4-oxo-1,3-diazospiro[4.4]non-1-en-3-yl)methyl)-N-(4-chloro-5-methylisoxazol-3-yl)- 2'-(((methoxy-d3)methyl)-[1,1'-biphenyl] -2-sulfonamide

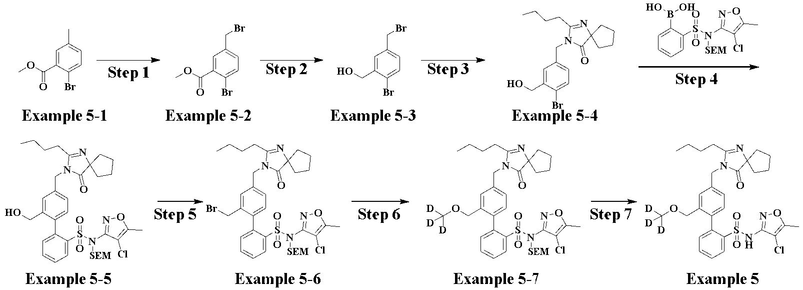

### Step 1

### Preparation of methyl 2-bromo-5-(bromomethyl)benzoate

Bromosuccinimide (8.16 g, 45.84 mmol) was added to a solution of Example **5-1** (10 g, 43.65 mmol) in MeCN (80 mL) under nitrogen protection, and the mixture was stirred at 25°C overnight, *i.e.,* 12 h. The reaction solution was concentrated, diluted with 100 mL of ethyl acetate, and washed three times with 50 mL of water. The organic phases were combined, dried and concentrated, and the residue was purified by silica gel column chromatography with eluent system B to afford Example **5- 2** (10 g, yield: 74.3%).

¹H NMR (400 MHz, Chloroform-d) δ 7.82 (d, J = 2.3 Hz, 1H), 7.64 (d, J = 8.3 Hz, 1H), 7.36 (dd, J = 8.3, 2.4 Hz, 1H), 4.44 (s, 2H), 3.94 (s, 3H).

### Step 2

### Preparation of (2-bromo-5-(bromomethyl)phenyl)methanol

Diisobutylaluminium hydride (1 M, 62.11 mL) was added to a solution of Example **5-2** (10 g, 31.06 mmol) in DCM under nitrogen protection at 0°C, and the mixture was stirred at 20°C for 1 h. The reaction solution was quenched with ice water (200 mL) and extracted with dichloromethane (100 mL*3), and the organic phases were combined, dried and concentrated to afford Example **5-3** (6.0 g, a solid), which was directly used in the next step.

### Step 3

### Preparation of 3-(4-bromo-3-(hydroxymethyl)benzyl)-2-butyl-1,3-diazaspiro[4.4]non-1-en-4-one

Potassium carbonate (1.23 g, 8.93 mmol) was added to a solution of Example **5-3** (1.25 g, 4.46 mmol) and 2-butyl-1,3-diazaspiro[4.4]non-1-en-4-one hydrochloride (1.03 g, 4.46 mmol) in MeCN (15 mL), and the mixture was stirred at 80°C for 12 h. The reaction solution was quenched with water (10 mL) and extracted with dichloromethane (10 mL*3). The organic phases were combined, dried and concentrated, and the residue was purified by silica gel column chromatography with eluent system B to afford Example **5-4** (1.0 g, a white solid, yield: 56.9%).

MS m/z (ESI): 493.0 [M+1] ⁺.

### Step 4

### Preparation of 4'-((2-butyl-4-oxo-1,3-diazaspiro[4.4]non-1-en-3-yl)methyl)-N-(4-chloro-5-methylisoxazol-3-yl)-2'-(hydroxymethyl)-N-(methoxymethyl)-[1,1'-biphenyl]-2-sulfonamide

Example **5-4** (400 mg, 1.05 mmol), (2-(N-(4-chloro-5-methylisoxazol-3-yl)-N-(methoxymethyl)sulfamoyl)phenyl)boronic acid (380 mg, 1.06 mmol), [1,1'-bis(diphenylphosphino)ferrocene]palladium(II) dichloride dichloromethane complex (85 mg, 105.1 µmol), K₂CO₃ (285 mg, 2.10 mmol), 1'4-Dioxane (5 mL) and H₂O (1 mL) were added to a reactor. The mixture was stirred at 100°C for 12 h under nitrogen protection. After the reaction solution was cooled, the reaction solution was quenched with 8 mL of water and extracted with ethyl acetate (10 mL*3). The organic phases were combined, dried and concentrated, and the residue was purified by silica gel column chromatography with eluent system B to afford Example **5-5** (500 mg, a solid, yield: 75.3%).

MS m/z (ESI): 715.3[M+1] ⁺.

### Step 5

### Preparation of 2'-(bromomethyl)-4'-(2-butyl-4-oxo-1,3-diazaspiro[4.4]non-1-en-3-yl)methyl)-N-(4-chloro-5-methylisoxazol-3-yl)-N-(methoxymethyl)-[1,1'-biphenyl]-2-sulfonamide

Carbon tetrabromide (525 mg, 1.58 mmol) and triphenylphosphine (310 mg, 1.18 mmol) were added to a solution of Example **5-5** (500 mg, 0.79 mmol) in DCM (10 mL) under nitrogen protection at 0°C, and the mixture was stirred at 20°C for 1 h. The reaction solution was quenched with water (10 mL) and extracted with dichloromethane (10 mL*3). The organic phases were combined, dried and concentrated, and the residue was purified by silica gel column chromatography with eluent system B to afford Example **5-6** (520 mg, a white solid, yield: 95.3%).

MS m/z (ESI): 778.2 [M+1] ⁺.

### Step 6

### Preparation of 4'-((2-butyl-4-oxo-1,3-diazaspiro[4.4]non-1-en-3-yl)methyl)-N-(4-chloro-5-methylisoxazol-3-yl)- 2'-(((trifluoromethoxy)methyl)-N-((2-(trimethylsilyl)ethoxy)methyl)-[1,1'-biphenyl] -2-sulfonamide

Deuterated methanol (11.6 mg, 0.33 mmol) and silver fluoride (48 mg, 0.33 mmol) were dissolved in acetonitrile (5 mL). The reaction solution was cooled to - 30°C, stirred and reacted for 2 h. Example **5-6** (125 mg, 0.16 mmol) dissolved in 5 mL of acetonitrile was added to the reaction solution, and the reaction solution was stirred and reacted at room temperature for 24 h. Saturated sodium chloride aqueous solution (10 mL) was added to the reaction solution, and the mixture was extracted with ethyl acetate (3 x 10 mL). The organic phases were combined, dried and concentrated, and the residue was purified by column chromatography (petroleum ether/ethyl acetate system) to afford Example **5-7** (85 mg, 69.1%).

MS m/z (ESI): 783.2 [M+1] ⁺.

### Step 7

### Preparation of 4'-((2-butyl-4-oxo-1,3-diazaspiro[4.4]non-1-en-3-yl)methyl)-N-(4-chloro-5-methylisoxazol-3-yl)- 2'-((trifluoromethoxy)methyl)-[1,1'-biphenyl] -2-sulfonamide

Synthesis method of Example **5** referred to the synthesis method of Example **2,** and Example **5-7** was used as raw material to afford the title compound Example **5** (26 mg, yield: 56.3%).

MS m/z (ESI): 602.2 [M+1] ⁺.

¹H NMR (400 MHz, DMSO) δ 8.09 - 7.93 (m, 1H), 7.53 (s, 2H), 7.14 (d, J = 21.2 Hz, 2H), 6.98 (dd, J = 16.8, 7.7 Hz, 2H), 4.73 (s, 2H), 4.00 (dd, J = 31.4, 13.0 Hz, 2H), 2.36 (t, J = 7.5 Hz, 2H), 2.25 (s, 3H), 1.85 (d, J = 7.1 Hz, 6H), 1.69 (d, J = 7.5 Hz, 2H), 1.52 (dt, J = 15.2, 7.6 Hz, 2H), 1.29 (dt, J = 22.4, 7.5 Hz, 2H), 0.82 (t, J = 7.3 Hz, 3H).

### Example 6

### 4'-(2-Butyl-4-oxo-1,3-diazaspiro[4.4]non-1-en-3-yl)methyl)-2'-(ethoxymethyl)-N-(5-methyl-4-(methyl-d₃) isoxazol-3-yl)-[1,1'-biphenyl]-2-sulfonamide

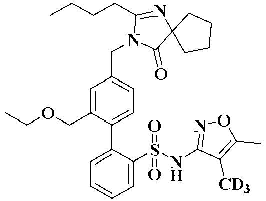

Synthesis method of Example **6** referred to the synthesis method of Example **1,** and 5-methyl-4-(deuteromethyl)isoxazole-3-amine was used in place of 4,5-dimethylisoxazolamine to afford Example **6** (6.8 mg, yield: 14.0%).

MS m/z (ESI): 596.3 [M+1] ⁺.

### Example 7

### 2-(4-((2-Butyl-4-oxo-1,3-diazaspiro[4.4]non-1-en-3-yl)methyl-d2)-2-(ethoxymethyl)phenyl)-N-(4-fluoro-5-methylisoxazol-3-yl)pyridine-3-sulfonamide

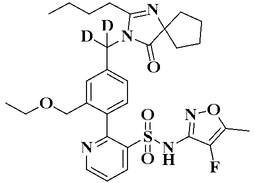

Synthesis method of Example 7 referred to the synthesis method of Example 1, and 4-fluoro-5-methylisoxazoleamine was used in place of 4,5-dimethylisoxazoleamine to afford Example 7 (19 mg, yield: 50.3%).

MS m/z (ESI): 600.3 [M+1]

¹H NMR (400 MHz, DMSO) δ 8.79 (t, *J =* 9.8 Hz, 1H), 8.41 (d, *J =* 8.1 Hz, 1H), 7.64 (dd, *J =* 8.0, 4.8 Hz, 1H), 7.22 (s, 1H), 7.05 (dt, *J =* 42.2, 21.0 Hz, 3H), 4.09 (s, 2H), 3.22 (dd, *J =* 13.9, 7.0 Hz, 2H), 2.36 (t, *J =* 7.5 Hz, 2H), 2.28 (s, 3H), 1.87 (s, 6H), 1.71 (s, 2H), 1.52 (dt, *J* = 15.2, 7.5 Hz, 2H), 1.30 (dt, *J =* 14.7, 7.4 Hz, 2H), 0.99 (t, *J* = 7.0 Hz, 3H), 0.83 (t, *J* = 7.3 Hz, 3H).

### Example 8

### 2-[4-[(2-Butyl-4-oxo-1,3-diazaspiro[4.4]non-1-en-3-yl)methyl]-2-(ethoxymethyl)phenyl]-N-(4-chloro-5-methyl-isoxazol-3-yl)benzenesulfonamide

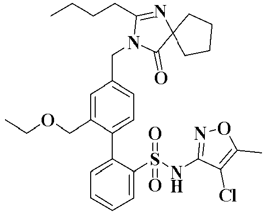

### Method I:

Synthesis method of Example **8** referred to the synthesis method of Example **1,** and 4-chloro-5-methylisoxazoleamine was used in place of 4,5-dimethylisoxazoleamine to afford Example **8** (56.6 mg, a white solid, yield: 45.8%).

### Method II:

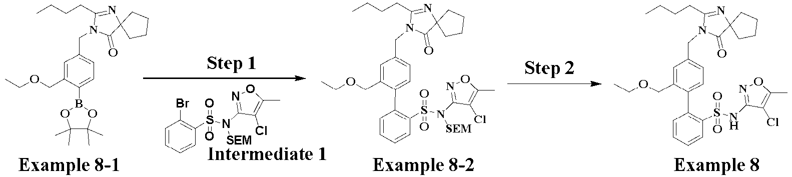

### Step 1

### Preparation of 4'-(2-butyl-4-oxo-1,3-diazaspiro[4.4]non-1-en-3-ylmethyl)-n-(4-chloro-5-methylisoxazol-3-yl)-2'-(ethoxymethyl)-n-(2-trimethylsilylethoxy)methyl)-[1,1'-biphenyl]-2-sulfonamide

Example **8-1** (0.3 g, 0.64 mmol) (referring to WO 2010114801 A1 for the preparation method) was dissolved in 10 mL of 1,4-dioxane and water (1 ml), and intermediate **1** (0.24 g, 0.64 mmol), 1,1'-bis(diphenylphosphino)ferrocene palladium(II) dichloride (0.023 g, 0.032 mmol) and potassium carbonate (0.18 g, 1.9 mmol) were added. The mixture was heated to 90°C under nitrogen protection, stirred and reacted for 16 h. The reaction solution was cooled to room temperature, saturated sodium chloride solution (50 mL) was added, and the mixture was extracted with ethyl acetate (100 mL × 3). The organic phases were combined, dried over anhydrous sodium sulfate, filtered and concentrated. The residue was purified by silica gel chromatography with an eluent system (ethyl acetate/petroleum ether = 10% to 50%) to afford Example **8-2** (0.27 g, a solid, yield: 66.0%).

MS m/z (ESI): 744.4[M+1] ⁺.

### Step 2

### Preparation of 4'-(2-butyl-4-oxo-1,3-diazaspiro[4.4]non-1-en-3-ylmethyl)-n-(4-chloro-5-methylisoxazol-3-yl)-2'-(ethoxymethyl)-[1,1'-biphenyl]-2-sulfonamide

Example **8-2** (0.27 g, 0.7 mmol) was dissolved in 10 mL of 4M HCl/dioxane. The mixture was heated to 70°C, stirred and reacted for 2 h. The reaction solution was cooled to room temperature, saturated sodium chloride solution (50 mL) was added, and the mixture was extracted with ethyl acetate (100 mL × 3). The organic phases were combined, dried over anhydrous sodium sulfate, filtered and concentrated. The residue was purified by silica gel chromatography with an eluent system (i.e., p-HPLC (FA)) to afford Example **8** (0.12 g, 54.5%).

MS m/z (ESI): 612.8 [M+1] ⁺.

¹H NMR (400 MHz, DMSO-*d*₆) δ 7.97 - 7.95 (m, 1H), 7.37 - 7.31 (m, 2H), 7.09 (s, 1H), 6.97 (d, *J* = 7.6 Hz, 1H), 6.93 - 6.88 (m, 2H), 4.70 (s, 2H), 4.08 (d, *J* = 13.2 Hz, 1H), 3.93 (d, *J =* 13.2 Hz, 1H), 3.24 - 3.15 (m, 2H), 2.36 (t, *J =* 7.6 Hz, 2H), 2.12 (s, 3H), 1.88 - 1.81 (m, 6H), 1.72 - 1.65 (m, 2H), 1.57 - 1.49 (m, 2H), 1.33 - 1.27 (m, 2H), 1.01 (t, *J* = 6.8 Hz, 3H), 0.83 (t, *J* = 7.2 Hz, 3H).

### Example 9

### 4'-((2'-Butyl-5'-oxaspiro[bicyclo[3.1.0]hexane-3,4'-imidazole] -1'(5'H)-yl)methyl)-N-(4,5 -dimethylisoxazol-3-yl)-2'-(ethoxymethyl)-[1,1'-biphenyl] -2-sulfonamide

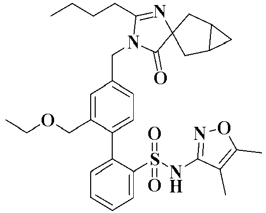

Synthesis method of Example **9** referred to the synthesis method of Example **1,** and 2'-butylspiro[bicyclo[3.1.0]hexane-3,4'-imidazole]-5'(1'H)-one was used in place of 2-butyl-1,3-diazaspiro-[4,4]non-1-en-4-one and lithium aluminium hydride in place of deuterated lithium aluminium hydride, to synthesize Example **9** (11 mg, yield: 36%).

MS m/z (ESI): 605.2 [M+1]⁺.

¹H NMR (400 MHz, DMSO) δ 8.06 (dd, *J =* 7.4, 1.9 Hz, 1H), 7.66 - 7.57 (m, 2H), 7.21 - 7.14 (m, 2H), 7.03 - 6.92 (m, 2H), 4.68 (s, 2H), 4.00 (s, 2H), 3.21 (d, *J* = 6.8 Hz, 2H), 2.32 (d, *J* = 7.5 Hz, 2H), 2.20 (s, 4H), 1.86 (d, *J* = 13.5 Hz, 2H), 1.66 (s, 3H), 1.50 (dq, *J =* 9.2, 5.5 Hz, 5H), 1.33 - 1.26 (m, 2H), 1.08 - 0.98 (m, 4H), 0.82 (t, *J* = 7.3 Hz, 3H), 0.56 (q, *J* = 4.0 Hz, 1H).

### Biological assay and evaluation

The present invention will be further described and explained below in conjunction with test examples, but these examples are not meant to limit the scope of the present invention.

### 1. Cell function experiment

**Test example 1. Determination of the effect of the compounds of the present invention on calcium current in cells stably expressing AT1 receptors**
1. Experimental objective:
To test the antagonistic effect of compounds on HEK293-AT1 cell activity
2. Experimental instruments and reagents:
2.1 Instruments:
384-well assay plate (Corning: 3764);
384-well Echo compound plate (Labcyte: LP-0200);
384-well compound plate (PE: 6008590);
Bravo Tip (Agilent: 10734-202);
FLIPR Tip (Molecular Device: 9000-0764);
Plate reader FLIPR Tetra (Molecular Device);
Pipetting workstation Bravo (Agilent);
ECHO 550 (LABCYTE);
Liquid injector Multidrop Combi (ThermoFisher).

2.2 Reagents:
DMEM, high glucose (Gibco: 12100);
Foetal bovine serum (Biosera: FB-1058/500);
P/S (Biosera: XC-A4122);
5X Matrigel (Corning: 354230);
HBSS (Sigma: H1387);
HEPES (Invitrogen: 15630080);
Fluo-8 AM (AAT Bioquest: 21080);
Probenecid (Sigma: P8761);
Pluronic F-127 (Sigma: P2443-250G);
Angiotensin III TFA (MCE: HY-113035A);
Irbesartan (MCE: HY-B0202);
1000X Fluo-8 AM (2 mM): Fluo-8 AM was dissolved in DMSO, and the mixture was shaken for 1-2 min, sub-packaged and stored at -20°C;
Complete culture medium: DMEM + 10% FBS + 1X P/S;
Cell seeding medium: DMEM + 10% FBS + 1X PS;
Experimental buffer 1: 1X HBSS + 20 mM HEPES +1 mM Probenecid + 0.025% Pluronic F-127;
Experimental buffer 2: 1X HBSS + 20 mM HEPES + 0.075% Pluronic F-127; 1X Matrigel: 5X Matrigel was diluted with DMEM;
Cell strain: HDB HEK293-AT1.

3. Experimental method:
1) HEK293-AT1 cell strain was cultured in complete culture medium at 37°C, 5% CO₂ to 70%-90% confluency.
2) The 384-well cell plate was coated with 1X Matrigel with 5 µL/well at room temperature for 10 to 30 min.
3) The cells were digested and resuspended in cell seeding medium, 8,000 cells/well/20 µL were seeded into a 384-well cell culture plate, and the cells were cultured at 37°C, 5% CO₂ for 24 h.
4) The cell culture plate was taken out of the CO₂ incubator and equilibrated at room temperature for 10 min.
5) 1000X Fluo-8 AM was taken and diluted to 1X Fluo-8 AM with experimental buffer 1 that had been equilibrated to room temperature, with a concentration of 2 µM.
6) The culture medium was removed from the cell culture plate, 20 µL of 1X Fluo-8 AM was added to each well, and the mixture was centrifuged at 300 rpm for 60 seconds at room temperature and incubated at room temperature in the dark for 1 h.
7) Positive control compound and test compound working solution (3X) were formulated:
   (1) instrument Bravo was used to dilute the compound to 11 concentration points on the 384-well Echo compound plate (LABCYTE: LP-0200);
   (2) instrument ECHO was used to transfer 90 nL of compound per well (compound storage concentration, such as the highest concentration point 10 mM) to the 384-well compound plate (PE: 6008590);
   (3) Multidrop Combi was used to add 30 uL of experimental buffer 2 to the 384-well compound plate (PE: 6008590), and the positive control compound and test compound were diluted to 30 µM (3X) and placed at room temperature until use.
   8) FLIPR Tetra was used to add 10 µL of diluted 3X compound to the experimental well of the corresponding 384-well cell plate, the compound and cells were incubated for 10 min at room temperature, and then 10 µL of diluted 4X agonist was added while reading and collecting data.

4. Experimental data processing method:
FLIPR Tetra was used to read and collect the fluorescence signal value (RFU). The maximum RFU value was used to calculate the percent activation data based on the readings of the Low control (DMSO control) and High control (100 nM positive compound) experimental groups {% activation rate = (RFUsample - RFUlow control) / (RFUhigh control - RFUlow control)×100}. The test compound was diluted 3X in the reaction system to generate 11 concentration points ranging from 10 uM to 0.17 nM. XLFit was used to fit the percentage activation rate and 11-point concentration data to a parametric nonlinear logic formula to calculate the IC₅₀ value of the compound.
5. Experimental results:

**Table 11 IC₅₀ values of compounds on calcium current in cells stably expressing AT1 receptors**

| No. | IC₅₀ (nM) |
|---|---|
| Example 2 | 6.2 |
| Example 4 | 7.2 |
| Example 8 | 3.2 |
| Example 9 | 9.6 |

6. Experimental conclusion:
It can be seen from the data in the table that the example compounds shown in the present invention show good antagonistic effects in the experiment of the effect on calcium current in cells stably expressing AT1 receptors.

**Test example 2. Determination of the effect of the compounds of the present invention on calcium current in cells stably expressing ETA receptors**
1. Experimental objective:
To test the antagonistic effect of compounds on HEK293-ETA cell activity
2. Experimental instruments and reagents:
2.1 Instruments:
   384-well assay plate (Corning: 3764);
   384-well Echo compound plate (Labcyte: LP-0200);
   384-well compound plate (PE: 6008590);
   Bravo Tip (Agilent: 10734-202);
   FLIPR Tip (Molecular Device: 9000-0764)
   Plate reader FLIPR Tetra (Molecular Device);
   Pipetting workstations Bravo (Agilent) and ECHO 550 (LABCYTE);
   Liquid injector Multidrop Combi (ThermoFisher).
2.2 Reagents:
   DMEM, high glucose (Gibco: 12100);
   Foetal bovine serum (Biosera: FB-1058/500);
   P/S (Biosera: XC-A4122);
   5X Matrigel (Corning: 354230);
   HBSS (Sigma: H1387);
   HEPES (Invitrogen: 15630080);
   Fluo-8 AM (AAT Bioquest: 21080);
   Probenecid (Sigma: P8761);
   Pluronic F-127 (Sigma: P2443-250G);
   Endothelin 1 (MCE: HY-P0202);
   Zibotentan (MCE: HY-10088);
   1000X Fluo-8 AM (2 mM): Fluo-8 AM was dissolved in DMSO, and the mixture was shaken for 1-2 min, sub-packaged and stored at -20°C;
   Complete culture medium: DMEM + 10% FBS + 1X P/S;
   Cell seeding medium: DMEM + 10% FBS + 1X PS;
   Experimental buffer 1: 1X HBSS + 20 mM HEPES +1 mM Probenecid + 0.025% Pluronic F-127;
   Experimental buffer 2: 1X HBSS + 20 mM HEPES + 0.075% Pluronic F-127; 1X Matrigel: 5X Matrigel was diluted with DMEM;
   Cell strain: HDB HEK293-ETA.

3. Experimental method:
1) HEK293-ETA cell strain was cultured in complete culture medium at 37°C, 5% CO₂ to 70%-90% confluency.
2) The 384-well cell plate was coated with 1X Matrigel with 5 µL/well at room temperature for 10 to 30 min.
3) The cells were digested and resuspended in cell seeding medium, 8,000 cells/well/20 µL were seeded into a 384-well cell culture plate, and the cells were cultured at 37°C, 5% CO₂ for 24 h.
4) The cell culture plate was taken out of the CO₂ incubator and equilibrated at room temperature for 10 min.
5) 1000X Fluo-8 AM was taken and diluted to 1X Fluo-8 AM with experimental buffer 1 that had been equilibrated to room temperature, with a concentration of 2 µM.
6) The culture medium was removed from the cell culture plate, 20 µL of 1X Fluo-8 AM was added to each well, and the mixture was centrifuged at 300 rpm for 60 seconds at room temperature and incubated at room temperature in the dark for 1 h.
7) Positive control compound and test compound working solution (3X) were formulated:
   (1) instrument Bravo was used to dilute the compound to 11 concentration points on the 384-well Echo compound plate (LABCYTE: LP-0200);
   (2) instrument ECHO was used to transfer 90 nL of compound per well (compound storage concentration, such as the highest concentration point 10 mM) to the 384-well compound plate (PE: 6008590);
   (3) Multidrop Combi was used to add 30 µL of experimental buffer 2 to the 384-well compound plate (PE: 6008590), and the positive control compound and test compound were diluted to 30 µM (3X) and placed at room temperature until use.
8) FLIPR Tetra was used to add 10 µL of diluted 3X compound to the experimental well of the corresponding 384-well cell plate, the compound and cells were incubated for 10 min at room temperature, and then 10 µL of diluted 4X agonist was added while reading and collecting data.

4. Experimental data processing method:
FLIPR Tetra was used to read and collect the fluorescence signal value (RFU). The maximum RFU value was used to calculate the percent activation data based on the readings of the Low control (DMSO control) and High control (100 nM positive compound) experimental groups {% activation rate = (RFUsample - RFUlow control) / (RFUhigh control - RFUlow control)×100}. The test compound was diluted 3X in the reaction system to generate 11 concentration points ranging from 10 µM to 0.17 nM. XLFit was used to fit the percentage activation rate and 11-point concentration data to a parametric nonlinear logic formula to calculate the IC₅₀ value of the compound.
5. Experimental results:

**Table 12 IC₅₀ values of compounds on calcium current in cells stably expressing ETA receptors**

| No. | IC₅₀ (nM) |
|---|---|
| Example 2 | 15.1 |
| Example 4 | 12.9 |
| Example 8 | 14.9 |
| Example 9 | 8.2 |

6. Experimental conclusion:
It can be seen from the data in the table that the example compounds shown in the present invention show good antagonistic effects in the experiment of the effect on calcium current in cells stably expressing ETA receptors.
**2. Rat pharmacokinetic evaluation assay**
1. Study objective:
SD rats were used as test animals to study the pharmacokinetic behaviour of the compounds of the present invention in rat (plasma) after oral administration at a dose of 5 mg/kg.
2. Experimental scheme:
2.1 Experimental drugs:
   The example compounds of the present invention, made in house.
2.2 Experimental animals:

3 SD rats/group, male, from Shanghai Jiesijie Experimental Animal Co., Ltd., animal production license number (SCXK (Shanghai) 2013-0006 N0.311620400001794).
2.3 Preparation formulation:
0.5% CMC-Na (1% Tween80) was dissolved by ultrasonication, and formulated into a clear solution or homogeneous suspension.
2.4 Administration:
p.o. respectively after the rats were fasted overnight;
the p.o. dose was 5 mg/kg and the administration volume was 10 mL/kg.

2.5 Sample collection:
After oral administration of rats, 0.2 mL of blood was collected from the jugular vein at 0.25 h, 0.5 h, 1.0 h, 2.0 h, and 4.0 h, placed in an EDTA-2K test tube, and centrifuged at 6000 rpm for 6 min at 4°C to separate plasma which was stored at - 80°C; the animals were fed 4 h after administration.
2.6 Sample treatment:
1) Acetonitrile (160 µL) was added to plasma sample (40 µL) for precipitation, and the mixture was centrifuged at 3500 × g for 5-20 min.
2) The treated supernatant solution was taken and subjected to LC/MS/MS to analyse the concentrations of the test compound. LC/MS/MS analysis instrument: AB Sciex API 4000 Qtrap.

2.7 Liquid phase analysis:
• Liquid phase conditions: Shimadzu LC-20AD pump
• Chromatographic column: Agilent ZORBAX XDB-C18 (50×2.1 mm, 3.5 µm), mobile phase: liquid A: 0.1% formic acid in water, liquid B: acetonitrile
• Flow rate: 0.4 mL/min
• Elution time: 0-4.0 min, the eluent was as follows:

| Time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis

The main pharmacokinetic parameters were calculated using WinNonlin 6.1. The results of pharmacokinetic experiments in rats are as shown in Table 13 below:

**Table 13: Pharmacokinetic parameters of the compounds of the present invention in rats after oral administration**

| No. | AUC_{0-∞} (ng/mL*h) | Cₘₐₓ (ng/mL) | Tₘₐₓ (h) | MRT _{0-∞} (h) |
|---|---|---|---|---|
| Example 2 | 10223 | 1693 | 0.5 | 8.4 |
| Example 8 | 16024 | 2140 | 0.5 | 8.6 |
| Example 9 | 9763 | 2627 | 0.5 | 3.8 |

### 3.4 Experimental conclusion:

The data in the table show that in the rat pharmacokinetic evaluation experiment, the example compounds of the present invention show high exposure after oral administration.

### 3. In vitro ADMET evaluation assay

### Test example 1. Liver microsomal metabolism stability assay

### 1. Experimental objective:

The objective of this experiment was to detect the stability of the example compounds in liver microsomes of rats, dogs and humans.

### 2. Experimental steps:

### 2.1 Formulation of compound working solution

Formulation of compound working solution: compound stock solution was added to phosphate buffer to a final concentration of 20 µM.

### 2.2 Formulation of liver microsome working solution

Dilution was performed with 100 mM phosphate buffer to a final concentration of 0.625 mg/mL.

### 2.3 Preparation of NADPH and UDPGA

NADPH (reduced nicotinamide adenine dinucleotide phosphate) and UDPGA (uridine diphosphate glucuronic acid) were weighed, and 100 mM phosphate buffer was added to a final concentration of 20 mM.

### 2.4 Preparation of perforation agent

1 mg of Alamethicin was weighed, and 200 µL of DMSO was added to formulate a 5 mg/mL solution. Then dilution was performed with phosphate buffer to a final concentration of 50 µg/mL.

### 2.5 Formulation of reaction stop solution

Stop solution: cold acetonitrile containing 100 ng/mL labetalol hydrochloride and 400 ng/mL tolbutamide as internal standards.

### 2.6 Incubation process

400 µL of formulated liver microsomes, 25 µL of compound working solution and 25 µL of Alamethicin were added in sequence to a 96-well plate, and the mixture was pre-incubated at 37°C for 10 min. Then 50 µL of formulated NADPH/UDPGA was added to start the reaction and the mixture was incubated at 37°C. The total volume of the reaction system was 500 µL. The final contents of each component are as follows:

| Component | Content |
|---|---|
| Liver microsomes | 0.5 mg/mL |
| Compound | 1 µM |
| NADPH | 2 mM |
| UDPGA | 2 mM |
| Alamethicin | 2.5 µg/mL |

### 2.7 Sample analysis

### 2.7.1 Chromatographic conditions:

Instrument: Shimadzu LC-30 AD;
Chromatographic column: XBridge^{®} C18 (50*4.6 mm, 5 µm particle size);
Mobile phase: A: 0.1% formic acid solution, B: methanol
Elution gradient: 0.2-1.6 min 5% A to 95% A, 3.0-3.1 min 95% A to 5% A
Run time: 4.0 min.

### 2.7.2 Mass spectrometry conditions:

Instrument: API5500 liquid chromatography-mass spectrometer, AB Sciex;
Ion source: electrospray ionization source (ESI);
Drying gas: N₂, temperature 500°C;
Electrospray voltage: 5000 V;
Detection mode: positive ion detection;
Scan mode: Multiple Reaction Monitoring (MRM) mode.

### 3. Experimental results:

Table 14 Results of liver microsomal metabolism stability of example compounds

| Example No. | Rat | | Dog | | Human | |
|---|---|---|---|---|---|---|
| | *t*_{1/2} (min) | Remaining rate (%, 60 min) | *t*_{1/2} (min) | Remaining rate (%, 60 min) | *t*_{1/2} (min) | Remaining rate (%, 60 min) |
| 2 | 26.3 | 29.4 | 29.9 | 24.6 | 38.5 | 33.0 |
| 4 | 24.0 | 17.1 | 29.5 | 23.9 | 26.2 | 30.7 |
| 8 | 29.9 | 23.3 | 28.5 | 23.6 | 36.5 | 32.0 |

### 4. Experimental conclusion:

The above data show that the example compounds of the present invention have good metabolism stability in liver microsomes of rats, dogs and humans.

### Test example 2. CYP enzyme single-point inhibition assay

### 1. Experimental objective

Human liver microsome incubation system was used, and the single-point method was used to quickly predict the inhibition on CYP450 enzyme subtypes (1A2, 2C19, 2D6, 3A4-M, and 3A4-T) by compounds.

### 2. Experimental steps

### 2.1 Solution formulation

For 2.5 mM NADPH, 4.165 mg of NADPH (reduced nicotinamide adenine dinucleotide phosphate) was weighed and 100 mM phosphate buffer was added to a final volume of 2 mL. For 0.25 mg/mL microsomes, 50 µL of 20 mg/mL microsomes was mixed with 4 mL of 100 mM phosphate buffer evenly.

Formulation of test compound reaction solution:

The test example compounds were weighed, diluted to 10 mM with DMSO, and then diluted to 100 µM with 100 mM phosphate buffer.

### 2.2 Experimental process:

1. Liver microsomes (40 µL), substrate (10 µL), and test compound (10 µL) were added to a 96-well plate, and the mixture was pre-incubated for 3 min.
2. NADPH (40 µL) was added.
3. At 20 min, acetonitrile stop solution containing internal standard (300 µL) was added.
4. The mixture was centrifuged and the sample was loaded.

### 3. Experimental results:

**Table 15 Results of single-point inhibition on CYP enzyme by example compounds**

| **Compound** | **IC₅₀ (µM)** | | | | |
|---|---|---|---|---|---|
| | **1A2** | **2C19** | **2D6** | **3A4-M** | **3A4-T** |
| 2 | >100 | >100 | >100 | 14.7 | >100 |
| 8 | >100 | >100 | >100 | 24.9 | >100 |

| | | | | | |
|---|---|---|---|---|---|
| Note: Strong inhibition: IC₅₀ < 1 µM; Medium inhibition: 1 µM < IC₅₀ < 10 µM; Weak inhibition: IC₅₀ > 10 µM | | | | | |

### 4. Experimental conclusion:

The above data show that the example compounds of the present invention do not have strong inhibition, but all have weak inhibition on each CYP enzyme subtype, and the risk of DDI is low.

### 4. Pharmacodynamic evaluation of compounds on blood pressure in spontaneously hypertensive rats

### 1. Experimental objective:

This experiment used a spontaneously hypertensive rat (SHR) model to evaluate the pharmacodynamic effect of the test compounds on the blood pressure and heart rate of the model.

### 2. Main experimental instrument and material

Kent Scientific CODA non-invasive blood pressure system.

### 3. Experimental animals:

Spontaneously hypertensive rats (SHR) were provided by Beijing Vital River Laboratory Animal Technology Co., Ltd., male, 150-200 g, 13-15 weeks old, 50 rats.

### 4. Experimental method:

4.1 Adaptation period: After animals arrived at the experimental facility, the animals were adapted to the animal facility for 5-7 days. 4.2 After the adaptation period, the animals were adapted to the tail oversleeve restrain for 3 days, and the tail oversleeve restrain was performed twice a day. After the last restrain adaptation, the basal blood pressure was measured, and the animals were randomly divided into groups according to the basal blood pressure. There was no significant difference between the average systolic blood pressure of animals in each group, and the number of animals in each group met the requirements of statistical testing and pharmacodynamic guidelines. 4.3 On the second day after grouping, the animals were administered according to experimental design and grouping (P.O, 5 mL/kg), and the systolic blood pressure (SBP), diastolic blood pressure (DBP), mean arterial pressure (MAP) and heart rate (HR) of the animals were measured before administration and at 1, 2, 4, 6, 8 and 24 h after administration using tail oversleeve method.

### 5. Data processing and analysis

Statistical differences between groups were analysed by Two-way ANOVA test. Among them, P < 0.05 indicated a significant difference, and P < 0.01 indicated an extremely significant difference.

### 6. Experimental results

The results show that in the spontaneously hypertensive model, compared with the vehicle group, the example compounds 44, 45 and 49 of the present invention significantly reduced blood pressure (systolic blood pressure, diastolic blood pressure and mean arterial pressure) 1-8 h after administration in the 30 mpk administration group (P < 0.05); in addition, no significant effect on heart rate was found in each administration group, and the safety was good.

### Study on salts of compounds and crystal forms thereof

It is well-known to those skilled in the art that the aforementioned example compounds are proven to have good antagonistic effects on cells stably expressing ETA receptors, and the pharmaceutically acceptable salts thereof tend to have the same pharmacological and pharmacodynamic activities. On this basis, the inventors further studied the physicochemical properties of the salt forms and crystal forms of the corresponding compounds. However, the preparation and characterization of the following specific salt forms or crystal forms do not represent a limitation on the scope of protection of the present invention. Those skilled in the art can, on the basis of the present invention, obtain more salt forms and crystals of the compound of the present invention through conventional salt formation or crystallization means, and these salt forms and crystals are all solutions protected by the present invention. The specific method is as follows.

### 1. Experimental instruments

### 1.1 Some parameters of physicochemical testing instruments

| | | |
|---|---|---|
| XRPD | Instrument model | BRUKER D8 ADVANCE |
| | Diffraction line | CuK (40 kV, 25 mA) |
| | Scan rate | 0.02°/S (20 value) |
| | Scan range | 4° - 40°(2θ value) |
| DSC | Instrument model | NETZSCH DSC 214 polyma |
| | Purge gas | Nitrogen |
| | Purge rate | 40 mL/min |
| | Heating rate | 10°C/min |
| | Temperature range | 25°C - 300°C |
| | Pan type | Aluminium pan |
| TGA | Instrument model | NETZSCH TG 209 Tarsus |
| | Purge gas | Nitrogen |
| | Purge rate | 40 mL/min |
| | Heating rate | 10°C/min |
| | Temperature range | 35°C-350°C |
| | Pan type | Al₂O₃ |

### 1.2 Instrument and liquid phase analysis conditions

### 1.2.1 Instruments and equipment

| Name of instrument | Model |
|---|---|
| Analytical balance | METTLER TOLEDO XA105 |
| Water purifier | Milli-Q Plus, Millipore |
| High performance liquid chromatograph | Agilent1260 |
| Pump | Agilent G1311B |
| Sample injector | G1329B |
| Column oven | G1316A |
| Detector | G1315D |

### 1.2.2 Chromatographic conditions

| | | | |
|---|---|---|---|
| Mobile phase | A: 0.05% trifluoroacetic acid in water; B: 0.05% trifluoroacetic acid in acetonitrile | | |
| Chromatographic column | Bonus RP (4.6 mm*75 mm, 3.5 um) | | |
| Chromatographic column temperature | 35°C | | |
| Sample pan temperature | 37°C | | |
| Flow rate | 1.0 mL/min | | |
| Wavelength | 254 nm | | |
| Injection volume | 20 µL | | |

| | Time (min) | A% | B% |
|---|---|---|---|
| Gradient | 0.00 | 80.0 | 20.0 |
| | 6.00 | 40.0 | 60.0 |
| | 10.00 | 10.0 | 90.0 |
| | 10.10 | 80.0 | 20.0 |
| | 15.00 | 80.0 | 20.0 |

### 2. Study on salt forms of compounds

### 2.1 Salt form screening of example compound 2

### 2.1.1 Experimental objective:

To screen the salt form of the compound.

### 2.1.2 Experimental steps:

1) Operation procedure
(1) Salt form screening by evaporation method

0.1748 g of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed and 3500 µL of ethanol was added. The mixture was dissolved clear by ultrasonication, and filtered to obtain the stock solution. 200 µL of the stock solution was taken, and a counter-ion acid solution at a molar ratio of 1 : 1.2 was added. The mixture was mixed evenly, and after the phenomena were observed and recorded, the mixture was slowly evaporated to dryness to obtain the resulting solid.

| **Counter-ion acid** | **Volume of acid added** | **Phenomenon (evaporated to dryness at room temperature)** |
|---|---|---|
| Hydrochloric acid | 19.50 µL | Salt formation |
| p-Toluenesulfonic acid | 19.50 µL | Salt formation |
| Methanesulfonic acid | 19.50 µL | Salt formation |
| Benzenesulfonic acid | 19.50 µL | Salt formation |
| Isethionic acid | 19.50 µL | Salt formation |
| Phosphoric acid | 19.50 µL | Salt formation |
| Tartaric acid | 19.50 µL*2 | Salt formation |
| Citric acid | 19.50 µL | Salt formation |

### (2) Salt formation by solvent slurry method

0.1748 g of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed and 3500 µL of ethanol was added. The mixture was dissolved clear by ultrasonication, and filtered to obtain the stock solution. 200 µL of the stock solution was taken, and a counter-ion acid solution at a molar ratio of 1 : 1.2 was added. The mixture was mixed evenly, and after the phenomena were observed and recorded, the mixture was slowly evaporated to dryness, anti-solvents ethyl acetate and MTBE were added, and the resulting mixture was continued to stir at room temperature.

| **Counter-ion acid** | **Volume of acid added** | **Phenomenon (evaporated to dryness at room temperature)** |
|---|---|---|
| Hydrobromic acid | 19.50 µL | Salt formation |
| Sulphuric acid | 19.50 µL | Salt formation |
| Oxalic acid | 19.50 µL | Salt formation |
| Maleic acid | 19.50 µL | Salt formation |
| Ethylsulfonic acid | 19.50 µL | Salt formation |
| Fumaric acid | 19.50 µL*4 | Salt formation |

### 2.1.3 Experimental results:

Through salt form screening experiments, the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base can form salts with hydrochloric acid, p-toluenesulfonic acid, methanesulfonic acid, benzenesulfonic acid, isethionic acid, phosphoric acid, tartaric acid, citric acid, hydrobromic acid, sulphuric acid, oxalic acid, maleic acid, ethylsulfonic acid or fumaric acid.

As described above, those skilled in the art can obtain more pharmaceutically acceptable salts by using conventional methods based on the present invention.

### 3. Study on crystal forms of compounds and salts thereof

### 3.1 Study on crystal forms of compounds

### 3.1.1 Experimental objective:

To screen the crystal form of example compound 2.

### 3.1.2 Experimental steps:

1) Operation procedure
(1) Crystal form preparation by evaporation method

0.1748 g of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed and 3500 µL of ethanol was added. The mixture was dissolved clear by ultrasonication, and filtered to obtain the stock solution. 200 µL of the stock solution was taken, and a counter-ion acid solution at a molar ratio of 1 : 1.2 was added. The mixture was mixed evenly, and after the phenomena were observed and recorded, the mixture was slowly evaporated to dryness to obtain the resulting solid. The solid was vacuum dried and characterized by XRD and DSC.

| **Counter-ion acid** | **Volume of acid added** | **XRD characterization** |
|---|---|---|
| Hydrochloric acid | 19.50 µL | Crystal form |
| p-Toluenesulfonic acid | 19.50 µL | Crystal form |
| Methanesulfonic acid | 19.50 µL | Crystal form |
| Benzenesulfonic acid | 19.50 µL | Crystal form |
| Isethionic acid | 19.50 µL | Crystal form |

### (2) Crystal form of salt formed by solvent slurry method

0.1748 g of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed and 3500 µL of ethanol was added. The mixture was dissolved clear by ultrasonication, and filtered to obtain the stock solution. 200 µL of the stock solution was taken, and a counter-ion acid solution at a molar ratio of 1 : 1.2 was added. The mixture was mixed evenly, and after the phenomena were observed and recorded, the mixture was slowly evaporated to dryness, anti-solvents ethyl acetate and MTBE were added, and the resulting mixture was continued to stir at room temperature. After the solid was precipitated, it was vacuum dried and characterized by XRD and DSC.

| **Counter-ion acid** | **Volume of acid added** | **XRD characterization** |
|---|---|---|
| Hydrobromic acid | 19.50 µL | Crystal form |
| Sulphuric acid | 19.50 µL | Crystal form |
| Ethylsulfonic acid | 19.50 µL | Crystal form |

### 3.1.3 Experimental results

Through the experiment on crystal form study of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide salt, the hydrobromic acid salt, sulphuric acid salt, p-toluenesulfonic acid salt, methanesulfonic acid salt, ethylsulfonic acid salt and benzenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide all formed crystal forms.

### 3.2 Preparation of crystal forms of compounds

### 3.2.1 Experimental objective:

To prepare crystal forms of compounds and salts thereof

### 3.2.2 Experimental steps:

### 1) Operation procedure

### I. Preparation of crystal form A of hydrochloride salt

20.48 mg of crystal form B of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide was weighed and dissolved in 0.2 mL of ethanol at 40°C, and 600 ul of anti-solvent n-heptane was added to allow precipitation of solids. The mixture was stirred magnetically in a metal bath at room temperature overnight, and centrifuged rapidly to remove the supernatant. The solid was vacuum dried at 40°C to a constant weight, to afford crystal form A of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 1, and a DSC pattern substantially as shown in FIG. 2.

### II. Preparation of crystal form B of hydrochloride salt

11 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 0.2 mL of dichloromethane was added. The mixture was dissolved clear and filtered, and a hydrogen chloride solution at a molar ratio of 1: 1.2 was added. The mixture was slowly evaporated to dryness at room temperature, and 1.4 mL of ethanol was added to allow precipitation of solids. The resulting mixture was centrifuged to remove the supernatant, and the solid was vacuum dried at 40°C to a constant weight, to afford crystal form B of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 3, and a DSC pattern substantially as shown in FIG. 4.

### III. Preparation of crystal form A of ethylsulfonic acid salt

11 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 0.2 mL of ethanol was added. The mixture was dissolved clear by ultrasonication and filtered, and an ethylsulfonic acid solution at a molar ratio of 1: 1.2 was added. The mixture was slowly evaporated to dryness at room temperature to obtain only a glassy solid, and 100 µL of ethyl acetate was added to continue slurrying and the mixture turned into a solid. The resulting mixture was centrifuged rapidly to remove the supernatant, and the solid was vacuum dried at 40°C to a constant weight, to afford crystal form A of the ethylsulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 5.

### IV. Preparation of crystal form B of ethylsulfonic acid salt

20 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 100 µL of ethyl acetate was added. The mixture was stirred at room temperature, and an ethylsulfonic acid solution at a molar ratio of 1 : 1.2 was added. No solid was precipitated, and further addition of isopropyl ether resulted in the mixture gradually adhering to the wall to form an oil and then turning into a solid. The resulting mixture was centrifuged rapidly to remove the supernatant, and the solid was vacuum dried at 40°C to a constant weight, to afford crystal form B of the ethylsulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 6.

### V. Preparation of crystal form A of p-toluenesulfonic acid salt

11 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 0.2 mL of ethanol was added. The mixture was dissolved clear by ultrasonication and filtered, and a p-toluenesulfonic acid solution at a molar ratio of 1: 1.2 was added. The mixture was slowly evaporated to dryness at room temperature to obtain only a glassy solid, and 100 µL of ethyl acetate was added to continue slurrying and the mixture turned into a solid. The resulting mixture was centrifuged rapidly to remove the supernatant, and the solid was vacuum dried at 40°C to a constant weight, to afford crystal form A of the p-toluenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 7.

### VI. Preparation of crystal form A of methanesulfonic acid salt

11 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 0.2 mL of ethanol was added. The mixture was dissolved clear by ultrasonication and filtered, and a methanesulfonic acid solution at a molar ratio of 1: 1.2 was added. The mixture was slowly evaporated to dryness at room temperature to obtain only a glassy solid, and 100 µL of ethyl acetate was added to continue slurrying and the mixture turned into a solid. The resulting mixture was centrifuged rapidly to remove the supernatant, and the solid was vacuum dried at 40°C to a constant weight, to afford crystal form A of the methanesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 8, and a DSC pattern substantially as shown in FIG. 9.

### VII. Preparation of crystal form A of sulphuric acid salt

11 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 0.2 mL of ethanol was added. The mixture was dissolved clear by ultrasonication and filtered, and a sulphuric acid aqueous solution at a molar ratio of 1: 1.2 was added. The mixture was slowly evaporated to dryness at room temperature to obtain only a glassy solid, and 100 µL of ethyl acetate was added to continue slurrying and the mixture turned into a solid. The resulting mixture was centrifuged rapidly to remove the supernatant, and the solid was vacuum dried at 40°C to a constant weight, to afford crystal form A of the sulphuric acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 10, and a DSC pattern substantially as shown in FIG. 11.

### VIII. Preparation of crystal form A of benzenesulfonic acid salt

11 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 0.2 mL of ethanol was added. The mixture was dissolved clear by ultrasonication and filtered, and a benzenesulfonic acid solution at a molar ratio of 1: 1.2 was added. The mixture was slowly evaporated to dryness at room temperature to obtain only a glassy solid, and 100 µL of ethyl acetate was added to continue slurrying and the mixture turned into a solid. The resulting mixture was centrifuged rapidly to remove the supernatant, and the solid was vacuum dried at 40°C to a constant weight, to afford crystal form A of the benzenesulfonic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 12.

### IX. Preparation of crystal form A of isethionic acid salt

20 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 100 µL of ethyl acetate was added. The mixture was stirred at room temperature, and an isethionic acid solution at a molar ratio of 1: 1.2 was added. No solid was precipitated, and further addition of isopropyl ether resulted in the mixture gradually adhering to the wall to form an oil and then turning into a solid. The resulting mixture was centrifuged rapidly to remove the supernatant, and the solid was vacuum dried at 40°C to a constant weight, to afford crystal form A of the isethionic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 13, and a DSC pattern substantially as shown in FIG. 14.

### X. Preparation of crystal form A of hydrobromic acid salt

11 mg of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, and 0.2 mL of ethanol was added. The mixture was dissolved clear by ultrasonication and filtered, and a hydrobromic acid solution at a molar ratio of 1: 1.2 was added. The mixture was slowly evaporated to dryness at room temperature to obtain only a glassy solid, and 100 µL of ethyl acetate was added to continue slurrying and the mixture turned into a solid. The resulting mixture was centrifuged rapidly to remove the supernatant, and the solid was vacuum dried at 40°C to a constant weight, to afford crystal form A of the hydrobromic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 15.

### XI. Preparation of crystal form C of hydrochloride salt

1 g of compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide free base was weighed, ethanol (2 mL) and n-heptane (4 mL) were added, and the mixture was stirred at room temperature for 2 h and filtered. The solid was rinsed with n-heptane to obtain 600 mg of white solid, which is crystal form C of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide.

After detection and analysis, the crystal form had an XRPD pattern substantially as shown in FIG. 16.

### 4. Solid stability experiment

4.1 Solid stability experiment of crystal forms of different salts of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide

### 4.1.1 Experimental objective:

To investigate the physical and chemical stability of crystal forms of the compound under high temperature, high humidity, high temperature and high humidity, and light conditions, thereby providing a basis for crystal form screening and storage.

### 4.1.2 Instrument and liquid phase analysis conditions

| | | | |
|---|---|---|---|
| Mobile phase | A: 0.05% trifluoroacetic acid in water; B: 0.05% trifluoroacetic acid in acetonitrile | | |
| Chromatographic column | Bonus RP (4.6 mm*75 mm, 3.5 um) | | |
| Chromatographic column temperature | 35°C | | |
| Sample pan temperature | 37°C | | |
| Flow rate | 1.0 mL/min | | |
| Wavelength | 254 nm | | |
| Injection volume | 20 µL | | |

| | Time (min) | A% | B% |
|---|---|---|---|
| Gradient | 0.00 | 80.0 | 20.0 |
| | 6.00 | 40.0 | 60.0 |
| | 10.00 | 10.0 | 90.0 |
| | 10.10 | 80.0 | 20.0 |
| | 15.00 | 80.0 | 20.0 |

### 4.1.3 Experimental scheme

4.1.3.1 Appropriate amounts of crystal form B of the hydrochloride salt, crystal form A of the methanesulfonic acid salt, crystal form A of the sulphuric acid salt, and crystal form A of the hydrobromic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide were weighed and treated under light (5000±500lux), high humidity (25°C, 92.5%), high temperature (60°C), and 50°C±2°C/RH75%±5% for a certain period of time, and then their XRPD, purity and impurity content data were measured.

### 4.1.4.1 Experimental results:

| Study parameters | Crystal form B of hydrochloride salt | Crystal form A of methanesulfonic acid salt | Crystal form A of sulphuric acid salt | Crystal form A of hydrobromic acid salt |
|---|---|---|---|---|
| Solid stability | Maximum individual impurity increase | | | |
| 5000±500lux | 0.04% | 0.09% | 0.06% | / |
| 60°C | 0.02% | 0.05% | 0.11% | 0.05% |
| 92.5% RH | <0.01% | <0.01% | 0.05% | 0.02% |
| 50°C&75%RH | <0.01% | 0.08% | 0.14% | 0.02% |
| XRPD | Crystal form unchanged | Crystal form unchanged | Crystal form unchanged | Crystal form unchanged |

The maximum individual impurity increase for crystal form B of hydrochloride salt, crystal form A of methanesulfonic acid salt and crystal form A of sulphuric acid salt was determined by comparing Day 20 with Day 0, whereas for crystal form A of hydrobromic acid salt, it was determined by comparing Day 10 with Day 0. The results show that crystal form B of the hydrochloride salt, crystal form A of the methanesulfonic acid salt, crystal form A of the sulphuric acid salt and crystal form A of the hydrobromic acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide were relatively stable.

### 5 Hygroscopicity experiment

### 5.1 Experimental objective

To investigate the hygroscopicity of crystal form B of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide under different relative humidity conditions, thereby providing a basis for compound crystal form screening and storage.

### 5.2 Experimental scheme:

Crystal form B of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide was placed in saturated water vapor with different relative humidity to allow the compound and the water vapor to reach dynamic equilibrium, and the percentage of hygroscopic weight gain of the compound after equilibrium was calculated.

Parameters of dynamic vapor sorption (DVS) instrument

| | |
|---|---|
| Instrument model | SMS Intrinsic |
| Experimental temperature | 25°C |
| Drying time | 0%RH 120 min |
| Equilibrium dm/dt | 0.02%/min (minimum 10 min, maximum 180 min) |
| RH (%) measurement step | 10% |
| Measurement gradient | 0-95-0% |
| Number of cycles | 2 |

### 5.3 Experimental results:

### 5.3.1 Hygroscopicity of crystal form B of hydrochloride salt of the compound

Crystal form B of the hydrochloride salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide exhibited a hygroscopic weight gain of 0.378% under RH80%, indicating slight hygroscopicity. In addition, after 2 cycles of moisture absorption and desorption under 0-95% relative humidity condition, there was no change in the XRPD pattern of crystal form B of the hydrochloride salt, that is, no crystal form transformation occurred.

## Claims

1. An acid salt of a compound as represented by general formula (I): 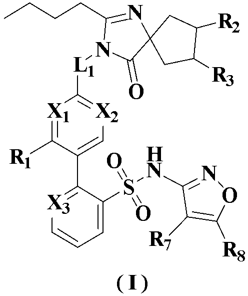 **characterized in that**
L₁ is -CH₂ or -CD₂-;
X₁ is N or CR¹;
X₂ is N or CR²;
X₃ is N or CR³;
R¹, R² and R³ are each independently selected from hydrogen, deuterium, halogen, amino, hydroxyl, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms, and the C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms is optionally further substituted with one or more substituents selected from deuterium, halogen, amino, hydroxyl, cyano, oxo, thio, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms;
R₁ is selected from hydrogen, C₁₋₃ alkyl or -(CH₂)ₙ₁O(CH₂)ₙ₂R_{A2}, and the C₁₋₃ alkyl is optionally further substituted with one or more substituents selected from deuterium, halogen, amino, hydroxyl, cyano, oxo, thio, C₁₋₃ alkyl, C₂₋₄ alkenyl, C₂₋₄ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy or C₃₋₆ cycloalkyl;
R_{A2} is selected from C₁₋₃ alkyl, C₁₋₃ deuteroalkyl, C₁₋₃ haloalkyl, C₃₋₆ cycloalkyl, 4- to 7-membered heterocyclyl, C₃₋₆ cycloalkyl C₁₋₃ alkyl or 4- to 7-membered heterocyclyl C₁₋₃ alkyl;
R₂ and R₃ are each independently selected from hydrogen, deuterium, halogen, amino, hydroxyl, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms, and the C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms is optionally further substituted with one or more substituents selected from deuterium, halogen, amino, hydroxyl, cyano, oxo, thio, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl containing 1 to 3 N, O or S atoms, C₆₋₁₀ aryl or 5- to 10-membered heteroaryl containing 1 to 3 N, O or S atoms;
alternatively, R₂ and R₃, together with the atom to which they are attached, form C₃₋₈ cycloalkyl or 3- to 8-membered heterocyclyl;
R₇ or R₈ is each independently selected from hydrogen, deuterium, halogen, amino, hydroxyl, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl or 5- to 10-membered heteroaryl;
n1 and n2 are each independently 0, 1, 2 or 3;
when L₁ is -CH₂-, and R₂ and R₃ are both hydrogen, R₇ and R₈ are not both - CH₃;
when L₁ is -CH₂-, and R₂ and R₃ are both hydrogen, R₁ is not hydrogen;
the acid in the acid salt is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, capric acid, caproic acid, caprylic acid, cinnamic acid, citric acid, cyclohexanesulfamic acid, camphorsulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfate, dibenzoyltartaric acid, ethane-1,2-disulfonic acid, ethylsulfonic acid, formic acid, fumaric acid, galactosonic acid, gentisic acid, glutaric acid, 2-ketoglutaric acid, glycolic acid, hippuric acid, isethionic acid, lactobionic acid, ascorbic acid, aspartic acid, lauric acid, camphoric acid, maleic acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, butanedioic acid, thiocyanic acid, pamoic acid, formic acid, undecylenic acid, trifluoroacetic acid, benzenesulfonic acid, p-toluenesulfonic acid or L-malic acid; preferably methanesulfonic acid, benzenesulfonic acid, isethionic acid, ethylsulfonic acid, hydrochloric acid, sulphuric acid, p-toluenesulfonic acid, phosphoric acid or hydrobromic acid.

2. The acid salt of the compound as represented by formula (I) according to claim 1, **characterized in that**
R¹, R² and R³ are each independently selected from hydrogen, deuterium, halogen, amino, hydroxyl, methyl, ethyl or propyl;
R₁ is selected from hydrogen, -CH₃, -CH₂CH₃, 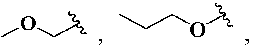 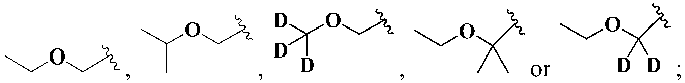
R₂ or R₃ is each independently selected from hydrogen, deuterium, fluorine, chlorine, bromine, methyl, ethyl or propyl;
alternatively, R₂ and R₃, together with the atom to which they are attached, form cyclopropyl, cyclobutyl, cyclopentyl or cyclohexyl;
R₇ is selected from deuterium, fluorine, chlorine, bromine, methyl, ethyl, propyl, deuterated methyl, dideuterated methyl or trideuterated methyl;
R₈ is selected from methyl, ethyl or propyl.

3. The acid salt of the compound as represented by formula (I) according to claim 1, **characterized in that** the compound is selected from:

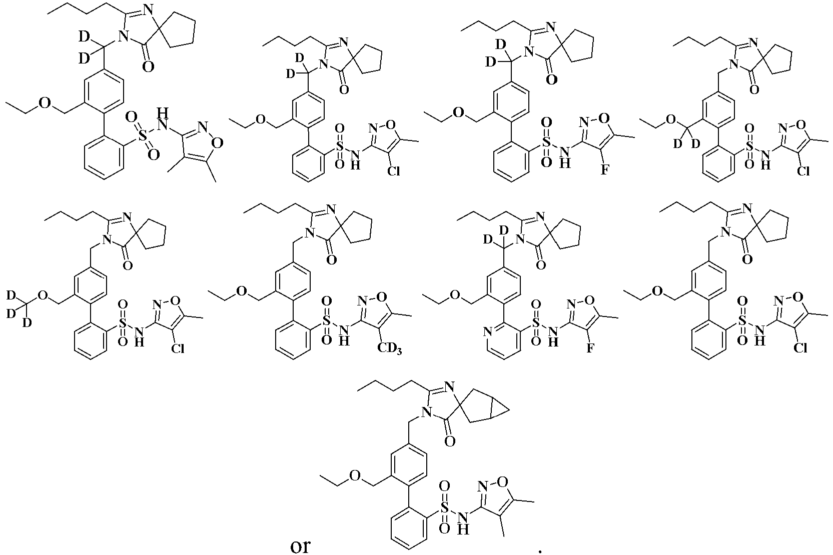

4. The acid salt of the compound as represented by formula (I) according to claim 1, **characterized in that** the compound is
4'-((2-butyl-4-oxo-1,3-diazospiro[4.4]non-1-en-3-yl)methyl-d2)-N-(4,5-dimethylisoxazol-3-yl)- 2'-(ethoxymethyl)-[1,1'-biphenyl] -2-sulfonamide;
4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide;
4'-((2-butyl-4-oxo-1,3-diazaspiro[4.4]non-1-en-3-yl)methyl-d2)-N-(4-fluoro-5-methylisoxazol-3-yl)-2'-(ethoxymethyl)-[1,1'-biphenyl]-2-sulfonamide;
4'-((2-butyl-4-oxo-1,3-diazospiro[4.4]non-1-en-3-yl)methyl)-N-(4-chloro-5-methylisoxazol-3-yl)-2'-(ethoxydeuteromethyl)-[1,1'-biphenyl]-2-sulfonamide;
2-[4-[(2-butyl-4-oxo-1,3-diazaspiro[4.4]non-1-en-3-yl)methyl]-2-(ethoxymethyl)phenyl]-N-(4-chloro-5-methyl-isoxazol-3-yl)benzenesulfonamide; or
4'-((2'-butyl-5'-oxaspiro[bicyclo[3.1.0]hexane-3,4'-imidazole] -1'(5'H)-yl)methyl)-N-(4,5 -dimethylisoxazol-3-yl)-2'-(ethoxymethyl)-[1,1'-biphenyl] -2-sulfonamide;
the acid in the acid salt is selected from methanesulfonic acid, ethylsulfonic acid, hydrochloric acid, sulphuric acid, p-toluenesulfonic acid, benzenesulfonic acid, isethionic acid or hydrobromic acid.

5. The acid salt of the compound as represented by formula (I) according to claim 1, **characterized in that** the number of acid molecules is 0.2 to 3; preferably 0.2, 0.5, 1, 1.5, 2, 2.5 or 3; more preferably 0.5, 1, 2 or 3, further preferably 1;
preferably, the acid salt is a hydrate or an anhydrate; when the acid salt is a hydrate, the number of water molecules is 0.2 to 3; preferably 0.2, 0.5, 1, 1.5, 2, 2.5 or 3; more preferably 0.5, 1, 2 or 3.

6. The acid salt of the compound as represented by formula (I) according to claim 1, **characterized in that** the acid salt is in a crystal form;
preferably the crystal form of the acid salt of the compound 4'-((2-butyl-4-oxo-1,3-diazospiro[4.4]non-1-en-3-yl)methyl-d2)-N-(4,5-dimethylisoxazol-3-yl)- 2'-(ethoxymethyl)-[1,1'-biphenyl] -2-sulfonamide;
the crystal form of the acid salt of 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide;
the crystal form of the acid salt of 4'-((2-butyl-4-oxo-1,3-diazaspiro[4.4]non-1-en-3-yl)methyl-d2)-N-(4-fluoro-5-methylisoxazol-3-yl)-2'-(ethoxymethyl)-[1,1'-biphenyl]-2-sulfonamide;
the crystal form of the acid salt of 2-[4-[(2-butyl-4-oxo-1,3-diazaspiro[4.4]non-1-en-3-yl)methyl]-2-(ethoxymethyl)phenyl]-N-(4-chloro-5-methyl-isoxazol-3-yl)benzenesulfonamide;
the crystal form of the acid salt of 4'-((2'-butyl-5'-oxaspiro[bicyclo[3.1.0]hexane-3,4'-imidazole] -1'(5'H)-yl)methyl)-N-(4,5 - dimethylisoxazol-3-yl)-2'-(ethoxymethyl)-[1,1'-biphenyl] -2-sulfonamide;
more preferably the crystal form of methanesulfonic acid salt, the crystal form of ethylsulfonic acid salt, the crystal form of hydrochloride salt, the crystal form of sulphuric acid salt, the crystal form of p-toluenesulfonic acid salt, the crystal form of benzenesulfonic acid salt, the crystal form of isethionic acid salt or the crystal form of hydrobromic acid salt.

7. The acid salt of the compound as represented by formula (I) according to claim 6, **characterized in that** the crystal form of the acid salt of the compound 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide is:
crystal form A of the hydrochloride salt, **characterized by** an X-ray powder diffraction pattern having a diffraction peak at 2θ = 9.2±0.2°; or a diffraction peak at 2θ = 9.0±0.2°; or a diffraction peak at 2θ = 8.2±0.2°; or a diffraction peak at 2θ = 10.8±0.2°; or a diffraction peak at 2θ = 16.8±0.2°; or a diffraction peak at 2θ = 18.8±0.2°; or a diffraction peak at 2θ = 20.2±0.2°; or a diffraction peak at 2θ = 20.6±0.2°; or a diffraction peak at 2θ = 20.9±0.2°; or a diffraction peak at 2θ = 23.2±0.2°; or a diffraction peak at 2θ = 25.3±0.2°; or a diffraction peak at 2θ = 24.0±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
preferably, the crystal form A of the hydrochloride salt is **characterized by** an X-ray powder diffraction pattern comprising at least one or more diffraction peaks at 2θ = 9.2±0.2°, 9.0±0.2° or 8.2±0.2°, preferably comprising two, more preferably comprising three; optionally further comprising at least one diffraction peak at 2θ = 10.8±0.2°, 16.8±0.2°, 18.8±0.2°, 20.2±0.2°, 20.6±0.2°, 20.9±0.2° or 23.2±0.2°, preferably comprising 2, 3, 4 or 5; for example, at 2θ =
9.2+0.2° or 9.0+0.2°;
9.2±0.2° or 8.2+0.2°;
9.0+0.2° or 8.2+0.2°;
9.2+0.2°, 9.0+0.2° or 8.2+0.2°;
9.2±0.2°, 8.2±0.2° or 10.8±0.2°;
9.0±0.2°, 8.2±0.2° or 10.8±0.2°;
9.2±0.2°, 9.0±0.2°, 8.2±0.2° or 10.8±0.2°;
9.2±0.2°, 8.2±0.2°, 10.8±0.2° or 16.8±0.2°;
9.0±0.2°, 8.2±0.2°, 10.8±0.2° or 16.8±0.2°;
9.2±0.2°, 9.0±0.2°, 8.2±0.2°, 10.8±0.2° or 16.8±0.2°;
9.2±0.2°, 8.2±0.2°, 10.8±0.2°, 16.8±0.2° or 18.8±0.2°;
9.0±0.2°, 8.2±0.2°, 10.8±0.2°, 16.8±0.2° or 18.8±0.2°;
9.2±0.2°, 9.0±0.2°, 8.2±0.2°, 10.8±0.2°, 16.8±0.2° or 18.8±0.2°;
9.2±0.2°, 8.2±0.2°, 10.8±0.2°, 16.8±0.2°, 18.8±0.2° or 20.2±0.2°;
9.0±0.2°, 8.2±0.2°, 10.8±0.2°, 16.8±0.2°, 18.8±0.2° or 20.2±0.2°;
9.2±0.2°, 9.0±0.2°, 8.2±0.2°, 10.8±0.2°, 16.8±0.2°, 18.8±0.2°, 20.2±0.2° or 20.6±0.2°;
9.2±0.2°, 8.2±0.2°, 10.8±0.2°, 16.8±0.2°, 18.8±0.2°, 20.2±0.2°, 20.6±0.2° or 20.9±0.2°;
9.0±0.2°, 8.2±0.2°, 10.8±0.2°, 16.8±0.2°, 18.8±0.2°, 20.2±0.2°, 20.6±0.2° or 20.9±0.2°;
more preferably, the crystal form A of the hydrochloride salt is **characterized by** an X-ray powder diffraction pattern comprising one or more diffraction peaks at 2θ = 9.2±0.2°, 9.0±0.2°, 8.2±0.2°, 10.8±0.2°, 16.8±0.2°, 18.8±0.2°, 20.2±0.2°, 20.6±0.2°, 20.9±0.2°, 23.2±0.2°, 25.3±0.2° or 24.0±0.2°, preferably comprising any 4, 5, 6, 8 or 10 of the aforementioned diffraction peaks; for example, at 2θ =
9.2±0.2°, 9.0±0.2°, 16.8±0.2° or 18.8±0.2°;
9.2±0.2°, 8.2±0.2°, 16.8±0.2° or 18.8±0.2°;
9.2±0.2°, 9.0±0.2°, 16.8±0.2°, 18.8±0.2°, 20.2±0.2° or 20.6±0.2°;
9.2±0.2°, 8.2±0.2°, 16.8±0.2°, 18.8±0.2°, 20.2±0.2° or 20.6±0.2°;
9.2±0.2°, 9.0±0.2°, 16.8±0.2°, 18.8±0.2°, 20.2±0.2°, 20.6±0.2°, 20.9±0.2° or 23.2±0.2°;
9.2+0.2°, 8.2+0.2°, 16.8+0.2°, 18.8±0.2°, 20.2+0.2°, 20.6+0.2°, 20.9+0.2° or 23.2±0.2°;
9.2+0.2°, 9.0+0.2°, 16.8+0.2°, 18.8+0.2°, 20.2+0.2°, 20.6+0.2°, 20.9+0.2°, 23.2+0.2°, 25.3+0.2° or 24.0+0.2°;
9.2+0.2°, 8.2+0.2°, 16.8+0.2°, 18.8+0.2°, 20.2+0.2°, 20.6+0.2°, 20.9+0.2°, 23.2±0.2°, 25.3±0.2° or 24.0±0.2°;
further preferably, the crystal form A of the hydrochloride salt has an X-ray powder diffraction pattern substantially as shown in FIG. 1;
even further preferably, the crystal form A of the hydrochloride salt has a DSC pattern as shown in FIG. 2;
crystal form B of the hydrochloride salt, **characterized by** an X-ray powder diffraction pattern having a diffraction peak at 20 = 10.4±0.2°; or a diffraction peak at 2θ = 7.3±0.2°; or a diffraction peak at 2θ = 14.1±0.2°; or a diffraction peak at 2θ = 14.6±0.2°; or a diffraction peak at 2θ = 17.0±0.2°; or a diffraction peak at 2θ = 19.3±0.2°; or a diffraction peak at 2θ = 21.9±0.2°; or a diffraction peak at 2θ = 24.8±0.2°; or a diffraction peak at 2θ = 11.0±0.2°; or a diffraction peak at 2θ = 13.1±0.2°; or a diffraction peak at 2θ = 20.3±0.2°; or a diffraction peak at 2θ = 22.1±0.2°; or a diffraction peak at 2θ = 24.1±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
preferably, the crystal form B of the hydrochloride salt is **characterized by** an X-ray powder diffraction pattern comprising at least one or more diffraction peaks at 2θ = 10.4±0.2°, 7.3±0.2° or 14.1±0.2°, preferably comprising two, more preferably comprising three; optionally further comprising at least one diffraction peak at 2θ = 14.6±0.2°, 17.0±0.2°, 19.3±0.2°, 21.9±0.2°, 24.8±0.2°, 11.0±0.2° or 13.1±0.2°, preferably comprising 2, 3, 4 or 5; for example, at 2θ =
10.4±0.2° or 7.3±0.2°;
10.4±0.2° or 14.1±0.2°;
7.3±0.2° or 14.1±0.2°;
10.4±0.2°, 7.3±0.2° or 14.1±0.2°;
10.4±0.2°, 14.1±0.2° or 14.6±0.2°;
7.3±0.2°, 14.1±0.2° or 14.6±0.2°;
10.4±0.2°, 7.3±0.2°, 14.1±0.2° or 14.6±0.2°;
10.4+0.2°, 14.1+0.2°, 14.6+0.2° or 17.0+0.2°;
7.3+0.2°, 14.1+0.2°, 14.6+0.2° or 17.0+0.2°;
10.4+0.2°, 7.3+0.2°, 14.1+0.2°, 14.6+0.2° or 17.0+0.2°;
10.4+0.2°, 14.1±0.2°, 14.6+0.2°, 17.0+0.2° or 19.3±0.2°;
7.3+0.2°, 14.1+0.2°, 14.6+0.2°, 17.0+0.2° or 19.3+0.2°;
10.4±0.2°, 7.3±0.2°, 14.1±0.2°, 14.6±0.2°, 17.0±0.2° or 19.3±0.2°;
10.4±0.2°, 14.1±0.2°, 14.6±0.2°, 17.0±0.2°, 19.3±0.2° or 21.9±0.2°;
7.3±0.2°, 14.1±0.2°, 14.6±0.2°, 17.0±0.2°, 19.3±0.2° or 21.9±0.2°;
10.4±0.2°, 7.3±0.2°, 14.1±0.2°, 14.6±0.2°, 17.0±0.2°, 19.3±0.2°, 21.9±0.2° or 24.8±0.2°;
10.4±0.2°, 14.1±0.2°, 14.6±0.2°, 17.0±0.2°, 19.3±0.2°, 21.9±0.2°, 24.8±0.2° or 11.0±0.2°;
7.3±0.2°, 14.1±0.2°, 14.6±0.2°, 17.0±0.2°, 19.3±0.2°, 21.9±0.2°, 24.8±0.2° or 11.0±0.2°;
10.4±0.2°, 7.3±0.2°, 14.1±0.2°, 14.6±0.2°, 17.0±0.2°, 19.3±0.2°, 24.8±0.2° or 11.0±0.2°;
10.4±0.2°, 14.1±0.2°, 14.6±0.2°, 17.0±0.2°, 19.3±0.2°, 21.9±0.2°, 11.0±0.2° or 13.1±0.2°;
7.3±0.2°, 14.1±0.2°, 14.6±0.2°, 17.0±0.2°, 19.3±0.2°, 21.9±0.2°, 11.0±0.2° or 13.1±0.2°;
more preferably, the crystal form B of the hydrochloride salt is **characterized by** an X-ray powder diffraction pattern comprising one or more diffraction peaks at 2θ = 10.4±0.2°, 7.3±0.2°, 14.1±0.2°, 14.6±0.2°, 17.0±0.2°, 19.3±0.2°, 21.9±0.2°, 24.8±0.2°, 11.0±0.2°, 13.1±0.2°, 20.3±0.2°, 22.1±0.2° or 24.1±0.2°, preferably comprising any 4, 5, 6, 8 or 10 of the aforementioned diffraction peaks; for example, at 2θ =
10.4±0.2°, 7.3±0.2°, 17.0±0.2° or 19.3±0.2°;
10.4±0.2°, 14.1±0.2°, 17.0±0.2° or 19.3±0.2°;
10.4±0.2°, 7.3±0.2°, 17.0±0.2°, 19.3±0.2°, 21.9±0.2° or 24.8±0.2°;
10.4±0.2°, 14.1±0.2°, 17.0±0.2°, 19.3±0.2°, 21.9±0.2° or 24.8±0.2°;
10.4±0.2°, 7.3±0.2°, 17.0±0.2°, 19.3±0.2°, 21.9±0.2°, 24.8±0.2°, 11.0±0.2° or 13.1±0.2°;
10.4±0.2°, 14.1±0.2°, 17.0±0.2°, 19.3±0.2°, 21.9±0.2°, 24.8±0.2°, 11.0±0.2° or 13.1+0.2°;
10.4+0.2°, 7.3+0.2°, 17.0±0.2°, 19.3+0.2°, 21.9+0.2°, 24.8+0.2°, 11.0+0.2°, 13.1±0.2°, 20.3±0.2° or 22.1±0.2°;
10.4+0.2°, 14.1±0.2°, 17.0±0.2°, 19.3+0.2°, 21.9+0.2°, 24.8+0.2°, 11.0±0.2°, 13.1±0.2°, 20.3±0.2° or 22.1±0.2°;
10.4±0.2°, 7.3±0.2°, 14.1±0.2°, 14.6±0.2°, 17.0±0.2°, 19.3±0.2°, 21.9±0.2°, 24.8±0.2°, 11.0±0.2° or 13.1±0.2°;
10.4±0.2°, 14.1±0.2°, 14.6±0.2°, 17.0±0.2°, 19.3±0.2°, 21.9±0.2°, 24.8±0.2°, 11.0±0.2°, 13.1±0.2° or 20.3±0.2°;
7.3±0.2°, 14.1±0.2°, 14.6±0.2°, 17.0±0.2°, 19.3±0.2°, 21.9±0.2°, 24.8±0.2°, 11.0±0.2°, 13.1±0.2° or 20.3±0.2°;
10.4±0.2°, 7.3±0.2°, 14.1±0.2°, 14.6±0.2°, 17.0±0.2°, 19.3±0.2°, 24.8±0.2°, 11.0±0.2°, 13.1±0.2° or 20.3±0.2°;
10.4±0.2°, 14.1±0.2°, 14.6±0.2°, 17.0±0.2°, 19.3±0.2°, 21.9±0.2°, 11.0±0.2°, 13.1±0.2°, 20.3±0.2° or 22.1±0.2°;
7.3±0.2°, 14.1±0.2°, 14.6±0.2°, 17.0±0.2°, 19.3±0.2°, 21.9±0.2°, 11.0±0.2°, 13.1±0.2°, 20.3±0.2° or 22.1±0.2°;
further preferably, the crystal form B of the hydrochloride salt has an X-ray powder diffraction pattern substantially as shown in FIG. 3;
even further preferably, the crystal form B of the hydrochloride salt has a DSC pattern as shown in FIG. 4;
crystal form C of the hydrochloride salt, **characterized by** an X-ray powder diffraction pattern having a diffraction peak at 2θ = 8.6±0.2°; or a diffraction peak at 2θ = 7.9±0.2°; or a diffraction peak at 20 = 14.7±0.2°; or a diffraction peak at 2θ = 14.9±0.2°; or a diffraction peak at 2θ = 6.3±0.2°; or a diffraction peak at 20 = 17.4±0.2°; or a diffraction peak at 2θ = 10.3±0.2°; or a diffraction peak at 2θ = 12.4±0.2°; or a diffraction peak at 2θ = 23.8±0.2°; or a diffraction peak at 2θ = 24.8±0.2°; or a diffraction peak at 2θ = 16.0±0.2°; or a diffraction peak at 2θ = 18.2±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
preferably, the crystal form C of the hydrochloride salt is **characterized by** an X-ray powder diffraction pattern comprising at least one or more diffraction peaks at 2θ = 8.6±0.2°, 7.9±0.2° or 14.7±0.2°, preferably comprising two, more preferably comprising three; optionally further comprising at least one diffraction peak at 2θ = 14.9+0.2°, 6.3±0.2°, 17.4±0.2°, 10.3+0.2°, 12.4+0.2°, 23.8+0.2° or 24.8+0.2°, preferably comprising 2, 3, 4 or 5; for example, at 2θ =
8.6+0.2° or 7.9+0.2°;
8.6+0.2° or 14.7+0.2°;
7.9±0.2° or 14.7±0.2°;
8.6±0.2°, 7.9±0.2° or 14.7±0.2°;
8.6±0.2°, 14.7±0.2° or 14.9±0.2°;
7.9±0.2°, 14.7±0.2° or 14.9±0.2°;
8.6±0.2°, 7.9±0.2°, 14.7±0.2° or 14.9±0.2°;
8.6±0.2°, 14.7±0.2°, 14.9±0.2° or 6.3±0.2°;
7.9±0.2°, 14.7±0.2°, 14.9±0.2° or 6.3±0.2°;
8.6±0.2°, 7.9±0.2°, 14.7±0.2°, 14.9±0.2° or 6.3±0.2°;
8.6±0.2°, 14.7±0.2°, 14.9±0.2°, 6.3±0.2° or 17.4±0.2°;
7.9±0.2°, 14.7±0.2°, 14.9±0.2°, 6.3±0.2° or 17.4±0.2°;
8.6±0.2°, 7.9±0.2°, 14.7±0.2°, 14.9±0.2°, 6.3±0.2° or 17.4±0.2°;
8.6±0.2°, 14.7±0.2°, 14.9±0.2°, 6.3±0.2°, 17.4±0.2° or 10.3±0.2°;
7.9±0.2°, 14.7±0.2°, 14.9±0.2°, 6.3±0.2°, 17.4±0.2° or 10.3±0.2°;
8.6±0.2°, 7.9±0.2°, 14.7±0.2°, 14.9±0.2°, 6.3±0.2°, 17.4±0.2°, 10.3±0.2° or 12.4±0.2°;
8.6±0.2°, 14.7±0.2°, 14.9±0.2°, 6.3±0.2°, 17.4±0.2°, 10.3±0.2°, 12.4±0.2° or 23.8±0.2°;
7.9±0.2°, 14.7±0.2°, 14.9±0.2°, 6.3±0.2°, 17.4±0.2°, 10.3±0.2°, 12.4±0.2° or 23.8±0.2°;
more preferably, the crystal form C of the hydrochloride salt is **characterized by** an X-ray powder diffraction pattern comprising one or more diffraction peaks at 2θ = 8.6±0.2°, 7.9±0.2°, 14.7±0.2°, 14.9±0.2°, 6.3±0.2°, 17.4±0.2°, 10.3±0.2°, 12.4±0.2°, 23.8±0.2°, 24.8±0.2°, 16.0±0.2° or 18.2±0.2°, preferably comprising any 4, 5, 6, 8 or 10 of the aforementioned diffraction peaks; for example, at 2θ =
8.6±0.2°, 7.9±0.2°, 14.7±0.2° or 6.3±0.2°;
8.6±0.2°, 7.9±0.2°, 17.4±0.2° or 10.3±0.2°;
8.6±0.2°, 7.9±0.2°, 14.7±0.2°, 6.3±0.2°, 17.4±0.2° or 10.3±0.2°;
8.6±0.2°, 7.9±0.2°, 17.4±0.2°, 10.3±0.2°, 12.4±0.2° or 23.8±0.2°;
8.6+0.2°, 7.9+0.2°, 14.7+0.2°, 6.3+0.2°, 17.4+0.2°, 10.3+0.2°, 12.4+0.2° or 23.8±0.2°;
8.6+0.2°, 7.9+0.2°, 17.4±0.2°, 10.3+0.2°, 12.4+0.2°, 23.8+0.2°, 24.8+0.2° or 16.0+0.2°;
8.6+0.2°, 7.9+0.2°, 14.7+0.2°, 6.3+0.2°, 17.4+0.2°, 10.3+0.2°, 12.4+0.2°, 23.8±0.2°, 24.8±0.2° or 16.0±0.2°;
8.6±0.2°, 7.9±0.2°, 17.4±0.2°, 10.3±0.2°, 12.4±0.2°, 23.8±0.2°, 24.8±0.2°, 16.0±0.2° or 18.2±0.2°;
further preferably, the crystal form C of the hydrochloride salt has an X-ray powder diffraction pattern substantially as shown in FIG. 16;
crystal form A of the ethylsulfonic acid salt, **characterized by** an X-ray powder diffraction pattern having a diffraction peak at 2θ = 8.7±0.2°; or a diffraction peak at 20 = 7.4±0.2°; or a diffraction peak at 2θ = 17.0±0.2°; or a diffraction peak at 2θ = 17.5±0.2°; or a diffraction peak at 2θ = 19.4±0.2°; or a diffraction peak at 2θ = 22.7±0.2°; or a diffraction peak at 2θ = 26.4±0.2°; or a diffraction peak at 2θ = 28.8±0.2°; or a diffraction peak at 2θ = 20.1±0.2°; or a diffraction peak at 2θ = 35.1±0.2°; or a diffraction peak at 2θ = 35.4±0.2°; or a diffraction peak at 2θ = 15.5±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
preferably, the crystal form A of the ethylsulfonic acid salt is **characterized by** an X-ray powder diffraction pattern comprising at least one or more diffraction peaks at 2θ = 8.7±0.2°, 7.4±0.2° or 17.0±0.2°, preferably comprising two, more preferably comprising three; optionally further comprising at least one diffraction peak at 2θ = 17.5±0.2°, 19.4±0.2°, 22.7±0.2°, 26.4±0.2°, 28.8±0.2°, 20.1±0.2° or 35.1±0.2°, preferably comprising 2, 3, 4 or 5; for example, at 2θ =
8.7±0.2° or 7.4±0.2°;
8.7±0.2° or 17.0±0.2°;
7.4±0.2° or 17.0±0.2°;
8.7±0.2°, 7.4±0.2° or 17.0±0.2°;
8.7±0.2°, 17.0±0.2° or 17.5±0.2°;
7.4±0.2°, 17.0±0.2° or 17.5±0.2°;
8.7±0.2°, 7.4±0.2°, 17.0±0.2° or 17.5±0.2°;
8.7±0.2°, 17.0+0.2°, 17.5+0.2° or 19.4+0.2°;
7.4+0.2°, 17.0+0.2°, 17.5+0.2° or 19.4+0.2°;
8.7+0.2°, 7.4+0.2°, 17.0±0.2°, 17.5+0.2° or 19.4+0.2°;
8.7+0.2°, 17.0+0.2°, 17.5+0.2°, 19.4+0.2° or 22.7+0.2°;
7.4+0.2°, 17.0+0.2°, 17.5+0.2°, 19.4+0.2° or 22.7+0.2°;
8.7±0.2°, 7.4±0.2°, 17.0±0.2°, 17.5±0.2°, 19.4±0.2° or 22.7±0.2°;
8.7±0.2°, 17.0±0.2°, 17.5±0.2°, 19.4±0.2°, 22.7±0.2° or 26.4±0.2°;
7.4±0.2°, 17.0±0.2°, 17.5±0.2°, 19.4±0.2°, 22.7±0.2° or 26.4±0.2°;
8.7±0.2°, 7.4±0.2°, 17.0±0.2°, 17.5±0.2°, 19.4±0.2°, 22.7±0.2°, 26.4±0.2° or 28.8±0.2°;
8.7±0.2°, 17.0±0.2°, 17.5±0.2°, 19.4±0.2°, 22.7±0.2°, 26.4±0.2°, 28.8±0.2° or 20.1±0.2°;
7.4±0.2°, 17.0±0.2°, 17.5±0.2°, 19.4±0.2°, 22.7±0.2°, 26.4±0.2°, 28.8±0.2° or 20.1±0.2°;
more preferably, the crystal form A of the ethylsulfonic acid salt is **characterized by** an X-ray powder diffraction pattern comprising one or more diffraction peaks at 2θ = 8.7±0.2°, 7.4±0.2°, 17.0±0.2°, 17.5±0.2°, 19.4±0.2°, 22.7±0.2°, 26.4±0.2°, 28.8±0.2°, 20.1±0.2°, 35.1±0.2°, 35.4±0.2° or 15.5±0.2°, preferably comprising any 4, 5, 6, 8 or 10 of the aforementioned diffraction peaks; for example, at 20 =
8.7±0.2°, 7.4±0.2°, 19.4±0.2° or 22.7±0.2°;
8.7±0.2°, 17.0±0.2°, 19.4±0.2° or 22.7±0.2°;
8.7±0.2°, 7.4±0.2°, 19.4±0.2°, 22.7±0.2°, 26.4±0.2° or 28.8±0.2°;
8.7±0.2°, 17.0±0.2°, 19.4±0.2°, 22.7±0.2°, 26.4±0.2° or 28.8±0.2°;
8.7±0.2°, 7.4±0.2°, 19.4±0.2°, 22.7±0.2°, 26.4±0.2°, 28.8±0.2°, 20.1±0.2° or 35.1±0.2°;
8.7±0.2°, 17.0±0.2°, 19.4±0.2°, 22.7±0.2°, 26.4±0.2°, 28.8±0.2°, 20.1±0.2° or 35.1±0.2°;
8.7±0.2°, 7.4±0.2°, 19.4±0.2°, 22.7±0.2°, 26.4±0.2°, 28.8±0.2°, 20.1±0.2°, 35.1±0.2°, 35.4±0.2° or 15.5±0.2°;
8.7±0.2°, 17.0±0.2°, 19.4±0.2°, 22.7±0.2°, 26.4±0.2°, 28.8±0.2°, 20.1±0.2°, 35.1±0.2°, 35.4±0.2° or 15.5±0.2°;
further preferably, the crystal form A of the ethylsulfonic acid salt has an X-ray powder diffraction pattern substantially as shown in FIG. 5;
crystal form B of the ethylsulfonic acid salt, **characterized by** an X-ray powder diffraction pattern having a diffraction peak at 2θ = 8.8±0.2°; or a diffraction peak at 20 = 8.3±0.2°; or a diffraction peak at 2θ = 7.9±0.2°; or a diffraction peak at 2θ = 17.0±0.2°; or a diffraction peak at 2θ = 16.2±0.2°; or a diffraction peak at 2θ = 18.4±0.2°; or a diffraction peak at 2θ = 20.6±0.2°; or a diffraction peak at 2θ = 24.1±0.2°; or a diffraction peak at 2θ = 26.4±0.2°; or a diffraction peak at 2θ = 10.8±0.2°; or a diffraction peak at 2θ = 12.6±0.2°; or a diffraction peak at 2θ = 19.9±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
preferably, the crystal form B of the ethylsulfonic acid salt is **characterized by** an X-ray powder diffraction pattern comprising at least one or more diffraction peaks at 2θ = 8.8±0.2°, 8.3±0.2° or 7.9±0.2°, preferably comprising two, more preferably comprising three; optionally further comprising at least one diffraction peak at 2θ = 17.0±0.2°, 16.2±0.2°, 18.4±0.2°, 20.6±0.2°, 24.1±0.2°, 26.4±0.2° or 10.8±0.2°, preferably comprising 2, 3, 4 or 5; for example, at 2θ =
8.8±0.2° or 8.3±0.2°;
8.8±0.2° or 7.9±0.2°;
8.3±0.2° or 7.9±0.2°;
8.8±0.2°, 8.3±0.2° or 7.9±0.2°
8.8±0.2°, 7.9±0.2° or 17.0±0.2°;
8.3±0.2°, 7.9±0.2° or 17.0±0.2°;
8.8±0.2°, 8.3±0.2°, 7.9±0.2° or 17.0±0.2°;
8.8±0.2°, 7.9±0.2°, 17.0±0.2° or 16.2±0.2°;
8.3±0.2°, 7.9±0.2°, 17.0±0.2° or 16.2±0.2°;
8.8±0.2°, 8.3±0.2°, 7.9±0.2°, 17.0±0.2° or 16.2±0.2°;
8.8±0.2°, 7.9±0.2°, 17.0±0.2°, 16.2±0.2° or 18.4±0.2°;
8.3±0.2°, 7.9±0.2°, 17.0±0.2°, 16.2±0.2° or 18.4±0.2°;
8.8±0.2°, 8.3±0.2°, 7.9±0.2°, 17.0±0.2°, 16.2±0.2° or 18.4±0.2°;
8.8±0.2°, 7.9±0.2°, 17.0±0.2°, 16.2±0.2°, 18.4±0.2° or 20.6±0.2°;
8.3±0.2°, 7.9±0.2°, 17.0±0.2°, 16.2±0.2°, 18.4±0.2° or 20.6±0.2°;
8.8±0.2°, 8.3±0.2°, 7.9±0.2°, 17.0±0.2°, 16.2±0.2°, 18.4±0.2°, 20.6±0.2° or 24.1±0.2°;
8.8+0.2°, 7.9+0.2°, 17.0±0.2°, 16.2+0.2°, 18.4+0.2°, 20.6+0.2°, 24.1±0.2° or 26.4+0.2°;
8.3+0.2°, 7.9+0.2°, 17.0±0.2°, 16.2+0.2°, 18.4+0.2°, 20.6+0.2°, 24.1±0.2° or 26.4+0.2°;
more preferably, the crystal form B of the ethylsulfonic acid salt is **characterized by** an X-ray powder diffraction pattern comprising one or more diffraction peaks at 2θ = 8.8±0.2°, 8.3±0.2°, 7.9±0.2°, 17.0±0.2°, 16.2±0.2°, 18.4±0.2°, 20.6±0.2°, 24.1±0.2°, 26.4±0.2°, 10.8±0.2°, 12.6±0.2° or 19.9±0.2°, preferably comprising any 4, 5, 6, 8 or 10 of the aforementioned diffraction peaks; for example, at 20 =
8.8±0.2°, 8.3±0.2°, 16.2±0.2° or 18.4±0.2°;
8.8±0.2°, 7.9±0.2°, 16.2±0.2° or 18.4±0.2°;
8.8±0.2°, 8.3±0.2°, 16.2±0.2°, 18.4±0.2°, 20.6±0.2° or 24.1±0.2°;
8.8±0.2°, 7.9±0.2°, 16.2±0.2°, 18.4±0.2°, 20.6±0.2° or 24.1±0.2°;
8.8±0.2°, 8.3±0.2°, 16.2±0.2°, 18.4±0.2°, 20.6±0.2°, 24.1±0.2°, 26.4±0.2° or 10.8±0.2°;
8.8±0.2°, 7.9±0.2°, 16.2±0.2°, 18.4±0.2°, 20.6±0.2°, 24.1±0.2°, 26.4±0.2° or 10.8±0.2°;
8.8±0.2°, 8.3±0.2°, 16.2±0.2°, 18.4±0.2°, 20.6±0.2°, 24.1±0.2°, 26.4±0.2°, 10.8±0.2°, 12.6±0.2° or 19.9±0.2°;
8.8±0.2°, 7.9±0.2°, 16.2±0.2°, 18.4±0.2°, 20.6±0.2°, 24.1±0.2°, 26.4±0.2°, 10.8±0.2°, 12.6±0.2° or 19.9±0.2°;
further preferably, the crystal form B of the ethylsulfonic acid salt has an X-ray powder diffraction pattern substantially as shown in FIG. 6;
crystal form A of the p-toluenesulfonic acid salt, **characterized by** an X-ray powder diffraction pattern having a diffraction peak at 2θ = 5.8±0.2°; or a diffraction peak at 2θ = 6.2±0.2°; or a diffraction peak at 2θ = 18.6±0.2°; or a diffraction peak at 2θ = 23.2±0.2°; or a diffraction peak at 2θ = 29.1±0.2°; preferably comprising any 1, or 1-2, or 2-3 of the aforementioned diffraction peaks, more preferably comprising any 1, 2, 3, 4 or 5;
preferably, the crystal form A of the p-toluenesulfonic acid salt is **characterized by** an X-ray powder diffraction pattern comprising at least a diffraction peak at 2θ = 5.8±0.2°, optionally further comprising at least one diffraction peak at 2θ = 6.2±0.2°, 18.6±0.2°, 23.2±0.2° or 29.1±0.2°, preferably comprising 1, 2, 3 or 4; for example, at 2θ =
5.8±0.2°;
5.8±0.2° or 6.2±0.2°;
5.8±0.2° or 18.6±0.2°;
5.8±0.2° or 23.2±0.2°;
5.8±0.2° or 29.1±0.2°;
5.8±0.2°, 6.2±0.2° or 18.6±0.2°;
5.8±0.2°, 18.6±0.2° or 23.2±0.2°;
5.8±0.2°, 23.2±0.2° or 29.1±0.2°;
5.8±0.2°, 6.2±0.2°, 18.6±0.2° or 23.2±0.2°;
5.8±0.2°, 18.6±0.2°, 23.2±0.2° or 29.1±0.2°;
5.8±0.2°, 6.2±0.2°, 18.6±0.2°, 23.2±0.2° or 29.1±0.2°;
more preferably, the crystal form A of the p-toluenesulfonic acid salt is **characterized by** an X-ray powder diffraction pattern comprising one or more diffraction peaks at 2θ = 5.8±0.2°, 6.2±0.2°, 18.6±0.2°, 23.2±0.2° or 29.1±0.2°, preferably comprising any 4, 5 or 6 of the aforementioned diffraction peaks; for example, at 2θ =
5.8±0.2°, 6.2±0.2°, 23.2±0.2° or 29.1±0.2°;
5.8±0.2°, 6.2±0.2°, 18.6±0.2° or 29.1±0.2°;
further preferably, the crystal form A of the p-toluenesulfonic acid salt has an X-ray powder diffraction pattern substantially as shown in FIG. 7;
crystal form A of the methanesulfonic acid salt, **characterized by** an X-ray powder diffraction pattern having a diffraction peak at 2θ = 8.1±0.2°; or a diffraction peak at 2θ = 8.4±0.2°; or a diffraction peak at 2θ = 4.1±0.2°; or a diffraction peak at 2θ = 20.8±0.2°; or a diffraction peak at 2θ = 22.3±0.2°; or a diffraction peak at 2θ = 18.9±0.2°; or a diffraction peak at 2θ = 24.5±0.2°; or a diffraction peak at 2θ = 20.0±0.2°; or a diffraction peak at 2θ = 24.3±0.2°; or a diffraction peak at 2θ = 11.1±0.2°; or a diffraction peak at 2θ = 12.6±0.2°; or a diffraction peak at 2θ = 16.5±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
preferably, the crystal form A of the methanesulfonic acid salt is **characterized by** an X-ray powder diffraction pattern comprising at least one or more diffraction peaks at 2θ = 8.1±0.2°, 8.4+0.2° or 4.1+0.2°, preferably comprising two, more preferably comprising three; optionally further comprising at least one diffraction peak at 2θ = 20.8±0.2°, 22.3+0.2°, 18.9+0.2°, 24.5+0.2°, 20.0+0.2°, 24.3+0.2° or 11.1±0.2°, preferably comprising 2, 3, 4 or 5; for example, at 2θ =
8.1±0.2° or 8.4+0.2°;
8.1±0.2° or 4.1±0.2°;
8.4±0.2° or 4.1±0.2°;
8.1±0.2°, 8.4±0.2° or 4.1±0.2°;
8.1±0.2°, 4.1±0.2° or 20.8±0.2°;
8.4±0.2°, 4.1±0.2° or 20.8±0.2°;
8.1±0.2°, 8.4±0.2°, 4.1±0.2° or 20.8±0.2°;
8.1±0.2°, 4.1±0.2°, 20.8±0.2° or 22.3±0.2°;
8.4±0.2°, 4.1±0.2°, 20.8±0.2° or 22.3±0.2°;
8.1±0.2°, 8.4±0.2°, 4.1±0.2°, 20.8±0.2° or 22.3±0.2°;
8.1±0.2°, 4.1±0.2°, 20.8±0.2°, 22.3±0.2° or 18.9±0.2°;
8.4±0.2°, 4.1±0.2°, 20.8±0.2°, 22.3±0.2° or 18.9±0.2°;
8.1±0.2°, 8.4±0.2°, 4.1±0.2°, 20.8±0.2°, 22.3±0.2° or 18.9±0.2°;
8.1±0.2°, 4.1±0.2°, 20.8±0.2°, 22.3±0.2°, 18.9±0.2° or 24.5±0.2°;
8.4±0.2°, 4.1±0.2°, 20.8±0.2°, 22.3±0.2°, 18.9±0.2° or 24.5±0.2°;
8.1±0.2°, 8.4±0.2°, 4.1±0.2°, 20.8±0.2°, 22.3±0.2°, 18.9±0.2°, 24.5±0.2° or 20.0±0.2°;
8.1±0.2°, 4.1±0.2°, 20.8±0.2°, 22.3±0.2°, 18.9±0.2°, 24.5±0.2°, 20.0±0.2° or 24.3±0.2°;
8.4±0.2°, 4.1±0.2°, 20.8±0.2°, 22.3±0.2°, 18.9±0.2°, 24.5±0.2°, 20.0±0.2° or 24.3±0.2°;
more preferably, the crystal form A of the methanesulfonic acid salt is **characterized by** an X-ray powder diffraction pattern comprising one or more diffraction peaks at 2θ = 8.1±0.2°, 8.4±0.2°, 4.1±0.2°, 20.8±0.2°, 22.3±0.2°, 18.9±0.2°, 24.5±0.2°, 20.0±0.2°, 24.3±0.2°, 11.1±0.2°, 12.6±0.2° or 16.5±0.2°, preferably comprising any 4, 5, 6, 8 or 10 of the aforementioned diffraction peaks; for example, at 20 =
8.1±0.2°, 8.4±0.2°, 22.3±0.2° or 18.9±0.2°;
8.1±0.2°, 4.1±0.2°, 22.3±0.2° or 18.9±0.2°;
8.1±0.2°, 8.4±0.2°, 22.3±0.2°, 18.9±0.2°, 24.5±0.2° or 20.0±0.2°;
8.1±0.2°, 4.1+0.2°, 22.3+0.2°, 18.9+0.2°, 24.5+0.2° or 20.0+0.2°;
8.1±0.2°, 8.4+0.2°, 22.3±0.2°, 18.9+0.2°, 24.5+0.2°, 20.0+0.2°, 24.3+0.2° or 11.1±0.2°;
8.1±0.2°, 4.1±0.2°, 22.3±0.2°, 18.9±0.2°, 24.5+0.2°, 20.0+0.2°, 24.3+0.2° or 11.1±0.2°;
8.1±0.2°, 8.4±0.2°, 22.3±0.2°, 18.9±0.2°, 24.5±0.2°, 20.0±0.2°, 24.3±0.2°, 11.1±0.2°, 12.6±0.2° or 16.5±0.2°;
8.1±0.2°, 4.1±0.2°, 22.3±0.2°, 18.9±0.2°, 24.5±0.2°, 20.0±0.2°, 24.3±0.2°, 11.1±0.2°, 12.6±0.2° or 16.5±0.2°;
further preferably, the crystal form A of the methanesulfonic acid salt has an X-ray powder diffraction pattern substantially as shown in FIG. 8;
even further preferably, the crystal form A of the methanesulfonic acid salt has a DSC pattern as shown in FIG. 9;
crystal form A of the sulphuric acid salt, **characterized by** an X-ray powder diffraction pattern having a diffraction peak at 2θ = 8.3±0.2°; or a diffraction peak at 2θ = 20.9±0.2°; or a diffraction peak at 2θ = 18.5±0.2°; or a diffraction peak at 2θ = 24.4±0.2°; or a diffraction peak at 2θ = 10.0±0.2°; or a diffraction peak at 2θ = 11.0±0.2°; or a diffraction peak at 2θ = 12.6±0.2°; or a diffraction peak at 2θ = 16.3±0.2°; or a diffraction peak at 2θ = 19.9±0.2°; or a diffraction peak at 2θ = 20.4±0.2°; or a diffraction peak at 2θ = 21.9±0.2°; or a diffraction peak at 2θ = 26.7±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
preferably, the crystal form A of the sulphuric acid salt is **characterized by** an X-ray powder diffraction pattern comprising at least one or more diffraction peaks at 2θ = 8.3±0.2°, 20.9±0.2° or 18.5±0.2°, preferably comprising two, more preferably comprising three; optionally further comprising at least one diffraction peak at 2θ = 24.4±0.2°, 10.0±0.2°, 11.0±0.2°, 12.6±0.2°, 16.3±0.2°, 19.9±0.2° or 20.4±0.2°, preferably comprising 2, 3, 4 or 5; for example, at 2θ =
8.3±0.2° or 20.9±0.2°;
8.3±0.2° or 18.5±0.2°;
20.9±0.2° or 18.5±0.2°;
8.3±0.2°, 20.9±0.2° or 18.5±0.2°;
8.3±0.2°, 18.5±0.2° or 24.4±0.2°;
20.9+0.2°, 18.5+0.2° or 24.4+0.2°;
8.3+0.2°, 20.9+0.2°, 18.5+0.2° or 24.4+0.2°;
8.3+0.2°, 18.5±0.2°, 24.4+0.2° or 10.0+0.2°;
20.9+0.2°, 18.5±0.2°, 24.4+0.2° or 10.0±0.2°;
8.3+0.2°, 20.9±0.2°, 18.5+0.2°, 24.4+0.2° or 10.0±0.2°;
8.3±0.2°, 18.5±0.2°, 24.4±0.2°, 10.0±0.2° or 11.0±0.2°;
20.9±0.2°, 18.5±0.2°, 24.4±0.2°, 10.0±0.2° or 11.0±0.2°;
8.3±0.2°, 20.9±0.2°, 18.5±0.2°, 24.4±0.2°, 10.0±0.2° or 11.0±0.2°;
8.3±0.2°, 18.5±0.2°, 24.4±0.2°, 10.0±0.2°, 11.0±0.2° or 12.6±0.2°;
20.9±0.2°, 18.5±0.2°, 24.4±0.2°, 10.0±0.2°, 11.0±0.2° or 12.6±0.2°;
8.3±0.2°, 20.9±0.2°, 18.5±0.2°, 24.4±0.2°, 10.0±0.2°, 11.0±0.2°, 12.6±0.2° or 16.3±0.2°;
8.3±0.2°, 18.5±0.2°, 24.4±0.2°, 10.0±0.2°, 11.0±0.2°, 12.6±0.2°, 16.3±0.2° or 19.9±0.2°;
20.9±0.2°, 18.5±0.2°, 24.4±0.2°, 10.0±0.2°, 11.0±0.2°, 12.6±0.2°, 16.3±0.2° or 19.9±0.2°;
more preferably, the crystal form A of the sulphuric acid salt is **characterized by** an X-ray powder diffraction pattern comprising one or more diffraction peaks at 2θ = 8.3±0.2°, 20.9±0.2°, 18.5±0.2°, 24.4±0.2°, 10.0±0.2°, 11.0±0.2°, 12.6±0.2°, 16.3±0.2°, 19.9±0.2°, 20.4±0.2°, 21.9±0.2° or 26.7±0.2°, preferably comprising any 4, 5, 6, 8 or 10 of the aforementioned diffraction peaks; for example, at 2θ =
8.3±0.2°, 20.9±0.2°, 10.0±0.2° or 11.0±0.2°;
8.3±0.2°, 18.5±0.2°, 10.0±0.2° or 11.0±0.2°;
8.3±0.2°, 20.9±0.2°, 10.0±0.2°, 11.0±0.2°, 12.6±0.2° or 16.3±0.2°;
8.3±0.2°, 18.5±0.2°, 10.0±0.2°, 11.0±0.2°, 12.6±0.2° or 16.3±0.2°;
8.3±0.2°, 20.9±0.2°, 10.0±0.2°, 11.0±0.2°, 12.6±0.2°, 16.3±0.2°, 19.9±0.2° or 20.4±0.2°;
8.3±0.2°, 18.5±0.2°, 10.0±0.2°, 11.0±0.2°, 12.6±0.2°, 16.3±0.2°, 19.9±0.2° or 20.4±0.2°;
8.3±0.2°, 20.9±0.2°, 10.0±0.2°, 11.0±0.2°, 12.6±0.2°, 16.3±0.2°, 19.9±0.2°, 20.4±0.2°, 21.9±0.2° or 26.7±0.2°;
8.3±0.2°, 18.5±0.2°, 10.0±0.2°, 11.0±0.2°, 12.6±0.2°, 16.3±0.2°, 19.9±0.2°, 20.4±0.2°, 21.9±0.2° or 26.7±0.2°;
further preferably, the crystal form A of the sulphuric acid salt has an X-ray powder diffraction pattern substantially as shown in FIG. 10;
even further preferably, the crystal form A of the sulphuric acid salt has a DSC pattern as shown in FIG. 11;
crystal form A of the benzenesulfonic acid salt, **characterized by** an X-ray powder diffraction pattern having a diffraction peak at 2θ = 5.9±0.2°; or a diffraction peak at 2θ = 23.8±0.2°; or a diffraction peak at 2θ = 17.8±0.2°; or a diffraction peak at 2θ = 29.9±0.2°; preferably comprising any 1-3 or 2-4 of the aforementioned diffraction peaks, more preferably comprising any 1, 2, 3 or 4;
preferably, the crystal form A of the benzenesulfonic acid salt is **characterized by** an X-ray powder diffraction pattern comprising at least one or more diffraction peaks at 2θ = 5.9±0.2°, 23.8±0.2°, 17.8±0.2° or 29.9±0.2°, preferably comprising two, more preferably comprising three, further preferably comprising four; for example, at 2θ =
5.9±0.2°;
5.9±0.2° or 23.8±0.2°;
5.9±0.2° or 17.8±0.2°;
5.9±0.2° or 29.9±0.2°;
5.9±0.2°, 23.8±0.2° or 17.8±0.2°;
5.9±0.2°, 17.8±0.2° or 29.9±0.2°;
5.9±0.2°, 23.8±0.2°, 17.8±0.2° or 29.9±0.2°;
further preferably, the crystal form A of the benzenesulfonic acid salt has an X-ray powder diffraction pattern substantially as shown in FIG. 12;
crystal form A of the isethionic acid salt, **characterized by** an X-ray powder diffraction pattern having a diffraction peak at 2θ = 9.9±0.2°; or a diffraction peak at 2θ = 19.9±0.2°; or a diffraction peak at 2θ = 8.3±0.2°; or a diffraction peak at 2θ = 20.5±0.2°; or a diffraction peak at 2θ = 18.2±0.2°; or a diffraction peak at 2θ = 23.9±0.2°; or a diffraction peak at 2θ = 10.6±0.2°; preferably comprising any 2-5, or 3-5, or 3-6, or 3-7 of the aforementioned diffraction peaks, more preferably comprising any 2, 3, 4 or 5;
preferably, the crystal form A of the isethionic acid salt is **characterized by** an X-ray powder diffraction pattern comprising at least one or more diffraction peaks at 2θ = 9.9±0.2°, 19.9±0.2° or 8.3±0.2°, preferably comprising two, more preferably comprising three; optionally further comprising at least one diffraction peak at 2θ = 20.4±0.2°, 18.3±0.2°, 23.9±0.2° or 10.6±0.2°, preferably comprising 2, 3 or 4; for example, at 2θ =
9.9+0.2° or 19.9±0.2°;
9.9+0.2° or 8.3+0.2°;
19.9+0.2° or 8.3±0.2°;
9.9+0.2°, 19.9±0.2° or 8.3±0.2°;
9.9±0.2°, 8.3±0.2° or 20.4±0.2°;
19.9±0.2°, 8.3±0.2° or 20.4±0.2°;
9.9±0.2°, 19.9±0.2°, 8.3±0.2° or 20.4±0.2°;
9.9±0.2°, 8.3±0.2°, 20.4±0.2° or 18.2±0.2°;
19.9±0.2°, 8.3±0.2°, 20.4±0.2° or 18.2±0.2°;
9.9±0.2°, 19.9±0.2°, 8.3±0.2°, 20.4±0.2° or 18.2±0.2°;
9.9±0.2°, 8.3±0.2°, 20.4±0.2°, 18.2±0.2° or 23.9±0.2°;
19.9±0.2°, 8.3±0.2°, 20.4±0.2°, 18.2±0.2° or 23.9±0.2°;
9.9±0.2°, 19.9±0.2°, 8.3±0.2°, 20.4±0.2°, 18.2±0.2° or 23.9±0.2°;
9.9±0.2°, 8.3±0.2°, 20.4±0.2°, 18.2±0.2°, 23.9±0.2° or 10.6±0.2°;
19.9±0.2°, 8.3±0.2°, 20.4±0.2°, 18.2±0.2°, 23.9±0.2° or 10.6±0.2°;
9.9±0.2°, 19.9±0.2°, 8.3±0.2°, 20.4±0.2°, 18.2±0.2°, 23.9±0.2° or 10.6±0.2°;
more preferably, the crystal form A of the isethionic acid salt is **characterized by** an X-ray powder diffraction pattern comprising one or more diffraction peaks at 2θ = 9.9±0.2°, 19.9±0.2°, 8.3±0.2°, 20.5±0.2°, 18.2±0.2°, 23.9±0.2° or 10.6±0.2°, preferably comprising any 4, 5 or 6 of the aforementioned diffraction peaks; for example, at 2θ =
9.9±0.2°, 19.9±0.2°, 20.4±0.2° or 18.2±0.2°;
9.9±0.2°, 8.3±0.2°, 20.5±0.2° or 18.2±0.2°;
9.9±0.2°, 19.9±0.2°, 20.5±0.2°, 18.2±0.2°, 23.9±0.2° or 10.6±0.2°;
9.9±0.2°, 8.3±0.2°, 20.5±0.2°, 18.2±0.2°, 23.9±0.2° or 10.6±0.2°;
further preferably, the crystal form A of the isethionic acid salt has an X-ray powder diffraction pattern substantially as shown in FIG. 13;
even further preferably, the crystal form A of the isethionic acid salt has a DSC pattern as shown in FIG. 14;
crystal form A of the hydrobromic acid salt, **characterized by** an X-ray powder diffraction pattern having a diffraction peak at 2θ = 30.0±0.2°; or a diffraction peak at 2θ = 22.3±0.2°; or a diffraction peak at 2θ = 25.9±0.2°; or a diffraction peak at 2θ = 29.7±0.2°; or a diffraction peak at 2θ = 17.0±0.2°; or a diffraction peak at 2θ = 35.7±0.2°; or a diffraction peak at 2θ = 33.3±0.2°; or a diffraction peak at 2θ = 23.4±0.2°; or a diffraction peak at 2θ = 28.6±0.2°; or a diffraction peak at 2θ = 28.8±0.2°; or a diffraction peak at 2θ = 18.1±0.2°; or a diffraction peak at 2θ = 26.6±0.2°; preferably comprising any 2 to 5, or 3 to 5, or 3 to 6, or 3 to 8, or 5 to 8, or 6 to 8 of the aforementioned diffraction peaks, more preferably comprising any 6, 7 or 8;
preferably, the crystal form A of the hydrobromic acid salt is **characterized by** an X-ray powder diffraction pattern comprising at least one or more diffraction peaks at 2θ = 30.0±0.2°, 22.3+0.2° or 25.9±0.2°, preferably comprising two, more preferably comprising three; optionally further comprising at least one diffraction peak at 2θ = 29.7+0.2°, 17.0±0.2°, 35.7±0.2°, 33.3±0.2°, 23.4±0.2°, 28.6±0.2° or 28.8±0.2°, preferably comprising 2, 3, 4 or 5; for example, at 2θ =
30.0±0.2° or 22.3±0.2°;
30.0±0.2° or 25.9±0.2°;
22.3±0.2° or 25.9±0.2°;
30.0±0.2°, 22.3±0.2° or 25.9±0.2°;
30.0±0.2°, 25.9±0.2° or 29.7±0.2°;
22.3±0.2°, 25.9±0.2° or 29.7±0.2°;
30.0±0.2°, 22.3±0.2°, 25.9±0.2° or 29.7±0.2°;
30.0±0.2°, 25.9±0.2°, 29.7±0.2° or 17.0±0.2°;
22.3±0.2°, 25.9±0.2°, 29.7±0.2° or 17.0±0.2°;
30.0±0.2°, 22.3±0.2°, 25.9±0.2°, 29.7±0.2° or 17.0±0.2°;
30.0±0.2°, 25.9±0.2°, 29.7±0.2°, 17.0±0.2° or 35.7±0.2°;
22.3±0.2°, 25.9±0.2°, 29.7±0.2°, 17.0±0.2° or 35.7±0.2°;
30.0±0.2°, 22.3±0.2°, 25.9±0.2°, 29.7±0.2°, 17.0±0.2° or 35.7±0.2°;
30.0±0.2°, 25.9±0.2°, 29.7±0.2°, 17.0±0.2°, 35.7±0.2° or 33.3±0.2°;
22.3±0.2°, 25.9±0.2°, 29.7±0.2°, 17.0±0.2°, 35.7±0.2° or 33.3±0.2°;
30.0±0.2°, 22.3±0.2°, 25.9±0.2°, 29.7±0.2°, 17.0±0.2°, 35.7±0.2°, 33.3±0.2° or 23.4±0.2°;
30.0±0.2°, 25.9±0.2°, 29.7±0.2°, 17.0±0.2°, 35.7±0.2°, 33.3±0.2°, 23.4±0.2° or 28.6±0.2°;
22.3±0.2°, 25.9±0.2°, 29.7±0.2°, 17.0±0.2°, 35.7±0.2°, 33.3±0.2°, 23.4±0.2° or 28.6±0.2°;
more preferably, the crystal form A of the hydrobromic acid salt is **characterized by** an X-ray powder diffraction pattern comprising one or more diffraction peaks at 2θ = 30.0±0.2°, 22.3+0.2°, 25.9+0.2°, 29.7+0.2°, 17.0±0.2°, 35.7+0.2°, 33.3+0.2°, 23.4±0.2°, 28.6+0.2°, 28.8±0.2°, 18.1±0.2° or 26.6+0.2°, preferably comprising any 4, 5, 6, 8 or 10 of the aforementioned diffraction peaks; for example, at 2θ =
30.0±0.2°, 22.3±0.2°, 17.0±0.2° or 35.7±0.2°;
30.0±0.2°, 25.9±0.2°, 17.0±0.2° or 35.7±0.2°;
30.0±0.2°, 22.3±0.2°, 17.0±0.2°, 35.7±0.2°, 33.3±0.2° or 23.4±0.2°;
30.0±0.2°, 25.9±0.2°, 17.0±0.2°, 35.7±0.2°, 33.3±0.2° or 23.4±0.2°;
30.0±0.2°, 22.3±0.2°, 17.0±0.2°, 35.7±0.2°, 33.3±0.2°, 23.4±0.2°, 28.6±0.2° or 28.8±0.2°;
30.0±0.2°, 25.9±0.2°, 17.0±0.2°, 35.7±0.2°, 33.3±0.2°, 23.4±0.2°, 28.6±0.2° or 28.8±0.2°;
30.0±0.2°, 22.3±0.2°, 17.0±0.2°, 35.7±0.2°, 33.3±0.2°, 23.4±0.2°, 28.6±0.2°, 28.8±0.2°, 18.1±0.2° or 26.6±0.2°;
30.0±0.2°, 25.9±0.2°, 17.0±0.2°, 35.7±0.2°, 33.3±0.2°, 23.4±0.2°, 28.6±0.2°, 28.8±0.2°, 18.1±0.2° or 26.6±0.2°;
further preferably, the crystal form A of the hydrobromic acid salt has an X-ray powder diffraction pattern substantially as shown in FIG. 15.

8. The acid salt of the compound as represented by formula (I) according to claim 7, **characterized in that**
the crystal form A of the hydrochloride salt exhibits an X-ray powder diffraction pattern wherein the 2θ error between the diffraction peaks with the top ten relative peak intensities and the diffraction peaks at the corresponding positions in FIG. 1 is ±0.2° to ±0.5°, preferably ±0.2° to ±0.3°, most preferably ±0.2°;
the crystal form B of the hydrochloride salt exhibits an X-ray powder diffraction pattern wherein the 2θ error between the diffraction peaks with the top ten relative peak intensities and the diffraction peaks at the corresponding positions in FIG. 3 is ±0.2° to ±0.5°, preferably ±0.2° to ±0.3°, most preferably ±0.2°;
the crystal form A of the ethylsulfonic acid salt exhibits an X-ray powder diffraction pattern wherein the 2θ error between the diffraction peaks with the top ten relative peak intensities and the diffraction peaks at the corresponding positions in FIG. 5 is ±0.2° to ±0.5°, preferably ±0.2° to ±0.3°, most preferably ±0.2°;
the crystal form B of the ethylsulfonic acid salt exhibits an X-ray powder diffraction pattern wherein the 2θ error between the diffraction peaks with the top ten relative peak intensities and the diffraction peaks at the corresponding positions in FIG. 6 is ±0.2° to ±0.5°, preferably ±0.2° to ±0.3°, most preferably ±0.2°;
the crystal form A of the p-toluenesulfonic acid salt exhibits an X-ray powder diffraction pattern wherein the 2θ error between the diffraction peaks with the top six relative peak intensities and the diffraction peaks at the corresponding positions in FIG. 7 is ±0.2° to ±0.5°, preferably ±0.2° to ±0.3°, most preferably ±0.2°;
the crystal form A of the methanesulfonic acid salt exhibits an X-ray powder diffraction pattern wherein the 2θ error between the diffraction peaks with the top four relative peak intensities and the diffraction peaks at the corresponding positions in FIG. 8 is ±0.2° to ±0.5°, preferably ±0.2° to ±0.3°, most preferably ±0.2°;
the crystal form A of the sulphuric acid salt exhibits an X-ray powder diffraction pattern wherein the 2θ error between the diffraction peaks with the top ten relative peak intensities and the diffraction peaks at the corresponding positions in FIG. 10 is ±0.2° to ±0.5°, preferably ±0.2° to ±0.3°, most preferably ±0.2°;
the crystal form A of the benzenesulfonic acid salt exhibits an X-ray powder diffraction pattern wherein the 2θ error between the diffraction peaks with the top ten relative peak intensities and the diffraction peaks at the corresponding positions in FIG. 12 is ±0.2° to ±0.5°, preferably ±0.2° to ±0.3°, most preferably ±0.2°;
the crystal form A of the isethionic acid salt exhibits an X-ray powder diffraction pattern wherein the 2θ error between the diffraction peaks with the top seven relative peak intensities and the diffraction peaks at the corresponding positions in FIG. 13 is ±0.2° to ±0.5°, preferably ±0.2° to ±0.3°, most preferably ±0.2°;
the crystal form A of the hydrobromic acid salt exhibits an X-ray powder diffraction pattern wherein the 2θ error between the diffraction peaks with the top five relative peak intensities and the diffraction peaks at the corresponding positions in FIG. 15 is ±0.2° to ±0.5°, preferably ±0.2° to ±0.3°, most preferably ±0.2°;
the crystal form C of the hydrochloride salt exhibits an X-ray powder diffraction pattern wherein the 2θ error between the diffraction peaks with the top five relative peak intensities and the diffraction peaks at the corresponding positions in FIG. 16 is ±0.2° to ±0.5°, preferably ±0.2° to ±0.3°, most preferably ±0.2°.

9. The acid salt of the compound as represented by formula (I) according to claim 7, **characterized in that** the crystal form of the acid salt is a hydrate or an anhydrate, and when the crystal form of the acid salt is a hydrate, the number of water molecules is 0.2 to 3, preferably 0.2, 0.5, 1, 1.5, 2, 2.5 or 3, more preferably 0.5, 1, 2 or 3; further, the water in the hydrate exists as channel water, or crystal water, or a combination thereof.

10. A method for preparing the acid salt of the compound as represented by formula (I) according to any one of claims 1 to 9, **characterized by** comprising the following steps:
1) weighing an appropriate amount of a free base and dissolving the free base in a benign solvent;
2) weighing an appropriate amount of a counter-ion acid and dissolving the counter-ion acid in an organic solvent, wherein the counter-ion acid is preferably used in an amount of 1.0 to 1.5 equivalents;
3) combining the aforementioned two solutions, and stirring the combined solution to allow precipitation, or dropwise adding a poor solvent followed by the stirring to allow precipitation; and
4) rapidly centrifuging the resultant or leaving the resultant to stand for blow drying to obtain a target product,
wherein
the benign solvent is selected from one or more of acetone, tetrahydrofuran, ethyl formate, ethyl acetate, 2-methyl-tetrahydrofuran, 2-butanone, n-butanol, 1,4-dioxane, isobutanol, N,N-dimethylformamide, N,N-dimethylacetamide, n-propanol or tert-butanol; preferably one or more of 2-methyl-tetrahydrofuran, ethyl acetate, 2-butanone, acetone or ethyl formate;
the organic solvent is selected from one or more of methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyl-tetrahydrofuran, 2-butanone, 3-pentanone, heptane, methyl tert-butyl ether, isopropyl ether, 1,4-dioxane, tert-butanol or N,N-dimethylformamide; preferably one or more of methanol, ethanol or acetonitrile;
the poor solvent is selected from one or more of heptane, water, methyl tert-butyl ether, cyclohexane, toluene, isopropyl ether or ethyl acetate; preferably one or more of water, methyl tert-butyl ether or isopropyl ether;
the counter-ion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4-acetamidobenzoic acid, 4-aminobenzoic acid, capric acid, caproic acid, caprylic acid, cinnamic acid, citric acid, cyclohexanesulfamic acid, camphorsulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfate, dibenzoyltartaric acid, ethane-1,2-disulfonic acid, ethylsulfonic acid, isethionic acid, formic acid, fumaric acid, galactosonic acid, gentisic acid, glutaric acid, 2-ketoglutaric acid, glycolic acid, hippuric acid, isethionic acid, lactobionic acid, ascorbic acid, aspartic acid, lauric acid, camphoric acid, maleic acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, butanedioic acid, thiocyanic acid, pamoic acid, formic acid, undecylenic acid, trifluoroacetic acid, p-toluenesulfonic acid or L-malic acid; preferably methanesulfonic acid, ethylsulfonic acid, hydrochloric acid, sulphuric acid, p-toluenesulfonic acid, benzenesulfonic acid, isethionic acid or hydrobromic acid;
alternatively, comprising the following steps:
1) weighing an appropriate amount of a free base and suspending the free base with a poor solvent;
2) weighing an appropriate amount of a counter-ion acid and dissolving the counter-ion acid in an organic solvent, wherein the counter-ion acid is preferably used in an amount of 1.0 to 1.5 equivalents;
3) adding the aforementioned solution to the aforementioned suspension and stirring the mixed solution for 2 hours;
4) rapidly centrifuging the resultant or leaving the resultant to stand for blow drying to obtain a salt of a compound as represented by general formula (I),
wherein
the poor solvent is selected from one or more of ethanol, ethyl acetate, ethyl formate, isopropanol, isopropyl acetate, methyl tert-butyl ether, dichloromethane, methanol, acetonitrile, chlorobenzene, benzene, toluene, n-butanol, isobutanol or 3-pentanone; preferably one or more of ethanol, ethyl acetate, isopropanol or isopropyl acetate;
the organic solvent is selected from one or more of methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyl-tetrahydrofuran, 2-butanone, 3-pentanone, heptane, methyl tert-butyl ether, isopropyl ether, 1,4-dioxane, tert-butanol or N,N-dimethylformamide; preferably one or more of methanol, ethanol or acetonitrile;
the counter-ion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulfonic acid, 4-chlorobenzenesulfonic acid, benzoic acid, 4- acetamidobenzoic acid, 4-aminobenzoic acid, capric acid, caproic acid, caprylic acid, cinnamic acid, citric acid, cyclohexanesulfamic acid, camphorsulfonic acid, aspartic acid, camphoric acid, gluconic acid, glucuronic acid, glutamic acid, isoascorbic acid, lactic acid, malic acid, mandelic acid, pyroglutamic acid, tartaric acid, dodecyl sulfate, dibenzoyltartaric acid, ethane-1,2-disulfonic acid, ethylsulfonic acid, isethionic acid, formic acid, fumaric acid, galactosonic acid, gentisic acid, glutaric acid, 2-ketoglutaric acid, glycolic acid, hippuric acid, isethionic acid, lactobionic acid, ascorbic acid, aspartic acid, lauric acid, camphoric acid, maleic acid, malonic acid, methanesulfonic acid, 1,5-naphthalene disulfonic acid, naphthalene-2-sulfonic acid, nicotinic acid, oleic acid, orotic acid, oxalic acid, palmitic acid, pamoic acid, propanoic acid, salicylic acid, 4-aminosalicylic acid, sebacic acid, stearic acid, butanedioic acid, thiocyanic acid, pamoic acid, formic acid, undecylenic acid, trifluoroacetic acid, p-toluenesulfonic acid or L-malic acid; preferably methanesulfonic acid, ethylsulfonic acid, hydrochloric acid, sulphuric acid, p-toluenesulfonic acid, benzenesulfonic acid, isethionic acid or hydrobromic acid.

11. A pharmaceutical composition, **characterized by** comprising a therapeutically effective amount of the acid salt of the compound as represented by formula (I) according to any one of claims 1 to 9, and one or more pharmaceutically acceptable carriers or excipients, wherein
preferably, the compound as represented by formula (I) has a weight percentage of 0.1% to 95%, preferably 0.5% to 85%, more preferably 1% to 60%, further preferably 10% to 50%, even further preferably 15% to 40%, yet further preferably 20% to 30%, most preferably 20% to 25%.

12. Use of the acid salt of the compound as represented by formula (I) according to any one of claims 1 to 9, and the pharmaceutical composition according to claim 11 in the preparation of a medicament for treating an angiotensin II-dependent or endothelin-dependent disease; particularly, in the preparation of a medicament for treating a disease with dual angiotensin-dependent and endothelin-dependent mechanisms;
alternatively, in the preparation of a medicament for treating pain, sexual dysfunction, hypoxia and an ischemic disease, dementia, a neurological disease, a liver disease, cancer, hypertension, diabetes or a kidney disease, **characterized in that** the kidney disease is selected from diseases or conditions related to kidney, glomerular or glomerular mesangial cell function, preferably focal segmental glomerulosclerosis or IgA nephropathy.
